(19) Europäisches Patentamt
European Patent Office
Office européen des brevets

(11) Veröffentlichungsnummer: **0 016 296**
**A1**

(12) # EUROPÄISCHE PATENTANMELDUNG

(21) Anmeldenummer: 79810157.2

(22) Anmeldetag: 19.11.79

(51) Int. Cl.³: C 07 D 501/20
A 61 K 31/545, C 07 D 277/48
//C07C157/12, C07D277/40

(30) Priorität: 23.11.78 CH 12025/78

(43) Veröffentlichungstag der Anmeldung:
01.10.80 Patentblatt 80/20

(84) Benannte Vertragsstaaten:
AT BE CH DE FR GB IT LU NL SE

(71) Anmelder: CIBA-GEIGY AG
Patentabteilung Postfach
CH-4002 Basel(CH)

(72) Erfinder: Wiederkehr, René, Dr.
Grenzweg 9
CH-4148 Pfeffingen(CH)

(72) Erfinder: Scartazzini, Riccardo, Dr.
Conrad Ferdinand Meyer-Strasse 38
CH-4059 Basel(CH)

(54) **Cephalosporinderivate, Verfahren zu ihrer Herstellung, diese enthaltende Präparate und Zwischenprodukte sowie deren Herstellung.**

(57) Die Erfindung betrifft Aminothiazolacetamido-3-cephem-4-carbonsäureverbindungen der Formel

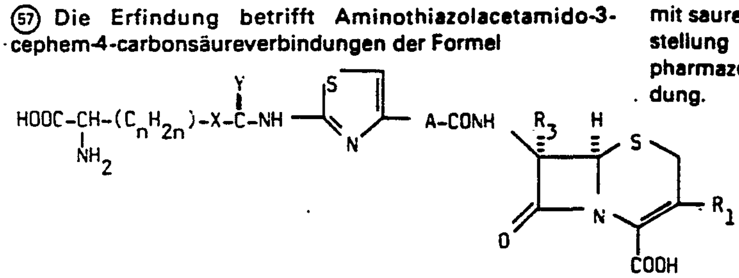

(I)

worin der Index n eine ganze Zahl von 1 bis 4, X Sauerstoff, Schwefel, die Gruppe -NH-, oder die direkte Bindung, Y Sauerstoff oder Schwefel, A Methylen oder durch Amino, Hydroxy, Carboxyl, Sulfo, Oxo, oder die Gruppe =N-O-R", worin R" Wasserstoff oder gegebenenfalls substituiertes Niederalkyl darstellt, substituiertes Methylen, $R_1$ Wasserstoff, Niederalkyl, eine veresterte oder verätherte Hydroxy- oder Mercaptogruppe, Halogen, Formyl oder eine Gruppe der Formel $-CH_2-R_2$, worin $R_2$ eine veresterte oder verätherte Hydroxy- oder Mercaptogruppe oder eine quaternäre Ammoniogruppe darstellt, und $R_3$ Wasserstoff oder Methoxy bedeuten, worin funktionelle Gruppen gegebenenfalls in geschützter Form vorliegen, Salze von solchen Verbindungen mit sauren und/oder basischen Gruppen, Verfahren zur Herstellung dieser Verbindungen, solche Stoffe enthaltende pharmazeutische Mittel und deren therapeutische Verwendung.

Die Verbindungen haben antibiotische Wirksamkeit und können zur Bekämpfung von Infektionen verwendet werden.

Die Erfindung betrifft ferner neue Zwischenprodukte zur Herstellung vorstehender Verbindungen, Verfahren zu ihrer Herstellung und ihrer Verwendung.

EP 0 016 296 A1

BEZEICHNUNG GEÄNDERT
siehe Titelseite

4-12128/=

Aminothiazole mit endständiger Aminocarbonsäuregruppierung

Die Erfindung betrifft neue Aminothiazolacetamido-3-cephem-4-carbonsäureverbindungen und ihre Salze, Verfahren zu ihrer Herstellung, diese Verbindungen enthaltende pharmazeutische Mittel mit antibiotischer Wirksamkeit und ihre therapeutische Verwendung zur Behandlung von Infektionen, sowie neue Zwischenprodukte und deren Herstellung.

Eine Reihe von Aminothiazolacetamido-3-cephem-4-carbonsäureverbindungen sind bereits als gut wirksame Antibiotika bekannt geworden.

Der vorliegenden Erfindung liegt die Aufgabe zugrunde, neue 7β-Aminothiazolacetamido-3-cephem-4-carbonsäureverbindungen herzustellen, worin die Aminogruppe durch einen Rest mit einer endständigen α-Aminocarbonsäuregruppierung substituiert ist. Die neuen Verbindungen zeichnen sich durch eine hervorragende Wirkung gegenüber normalen und resistenten Keimen aus.

Die als Ausgangsmaterialien zu benutzenden neuartigen substituierten Aminothiazolessigsäuren und deren reaktionsfähige funktionelle Derivate, worin funktionelle Gruppen gegebenenfalls geschützt sind, sowie Verfahren zu ihrer Herstellung, sind ebenfalls Gegenstand der vorliegenden Erfindung.

Die Erfindung betrifft insbesondere Aminothiazolacetamido-3-cephem-4-carbonsäureverbindungen der Formel

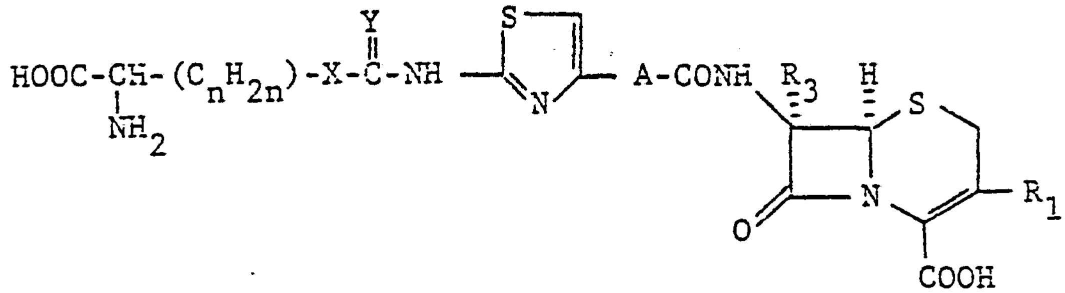

(I),

worin der Index n eine ganze Zahl von 1 bis 4, X Sauerstoff, Schwefel, die Gruppe -NH-, oder die direkte Bindung, Y Sauerstoff oder Schwefel, A Methylen oder durch Amino, Hydroxy, Carboxyl, Sulfo, Oxo, oder die Gruppe $=N-O-R^{o}$, worin $R^{o}$ Wasserstoff oder gegebenenfalls substituiertes Niederalkyl darstellt, substituiertes Methylen, $R_1$ Wasserstoff, Niederalkyl, eine veresterte oder verätherte Hydroxy- oder Mercaptogruppe, Halogen, Formyl oder eine Gruppe der Formel $-CH_2-R_2$, worin $R_2$ eine veresterte oder verätherte Hydroxy- oder Mercaptogruppe oder eine quaternäre Ammoniogruppe darstellt, und $R_3$ Wasserstoff oder Methoxy bedeuten, worin funktionelle Gruppen gegebenenfalls in geschützter Form vorliegen, Salze von solchen Verbindungen mit sauren und/oder basischen Gruppen, Verfahren zur Herstellung dieser Verbindungen, solche Stoffe enthaltende pharmazeutische Mittel und deren therapeutische Verwendung.

In der vorliegenden Beschreibung der Erfindung bedeutet der Ausdruck "Nieder" in Gruppen wie Niederalkyl, Niederalkylen, Niederalkoxy, Niederalkanoyl und dergleichen, dass die entsprechenden Gruppen, sofern nicht ausdrücklich anders definiert, bis zu 7, bevorzugt bis zu 4 C-Atome enthalten.

Eine Gruppe $-(C_nH_{2n})-$ ist eine verzweigte oder unverzweigte Alkylenkette, und ist insbesondere Methylen, 1,2-Aethylen, 1,3-Propylen oder 1,4-Butylen, ferner beispielsweise 1,1-Aethylen, 1,1-Propylen, 1,2-Propylen, 1,1-Butylen, oder 1,1-Isobutylen.

In einer Gruppe $=N-O-R^o$ enthält $R^o$ als gegebenenfalls substituiertes Niederalkyl 1-4 Kohlenstoffatome. Substituenten einer solchen Niederalkylgruppe $R^o$ sind beispielsweise Niederalkoxy, wie Methoxy, Halogen, wie Fluor, Chlor oder Brom, Hydroxy oder acyliertes Hydroxy, wie Niederalkanoyloxy, z.B. Acetoxy, Sulfo, und insbesondere freies oder auch verestertes Carboxy.

Repräsentative Gruppen $R^o$ sind beispielsweise Methyl, Aethyl, Propyl, Butyl, Methoxymethyl, Methoxyäthyl, wie 2-Methoxyäthyl, 3-Methoxypropyl, 4-Methoxybutyl, 2-Halogen-, wie 2-Chlor-äthyl, 3-Halogen-, wie 3-Chlorpropyl, oder 4-Halogen-, wie 4-Chlorbutyl, 2-Hydroxyäthyl, 3-Hydroxypropyl oder 4-Hydroxybutyl, worin die Hydroxygruppe beispielsweise durch Niederalkanoyl, wie Acetyl, acyliert sein kann, 2-Sulfoäthyl, 3-Sulfopropyl, 2-Carboxyäthyl, 3-Carboxypropyl oder 4-Carboxybutyl, worin die Carboxygruppe beispielsweise durch Niederalkyl, wie Methyl oder Aethyl verestert sein kann.

Eine Niederalkylgruppe $R_1$ enthält 1-4 C-Atome und ist beispielsweise Aethyl, Propyl, Butyl oder insbesondere Methyl.

Eine veresterte Hydroxy- oder Mercaptogruppe $R_1$ ist beispielsweise durch eine niederaliphatische Carbonsäure oder durch eine gegebenenfalls N-substituierte Carbaminsäure verestert.

Mit niederaliphatischen Carbonsäuren veresterte Hydroxygruppen $R_1$ sind insbesondere Niederalkanoyloxy, insbesondere Acetyloxy, ferner Formyloxy, Propionyloxy, Valeryloxy, Hexanoyloxy, Heptanoyloxy oder Pivaloyloxy.

Mit niederaliphatischen Carbonsäuren veresterte Mercaptogruppen $R_1$ sind Niederalkanoylthio, wie Acetylthio, Formylthio, Propionylthio, Valeroylthio, Hexanoylthio, Heptanoylthio oder Pivaloylthio.

In einer durch eine gegebenenfalls N-substituierte Carbaminsäure veresterten Hydroxy- oder Mercaptogruppe $R_1$ sind N-Substituenten gegebenenfalls durch Halogen, z.B. Chlor, oder durch Niederalkanoyl, z.B. Acetyl oder Propionyl, substituiertes Niederalkyl, z.B. Methyl, Aethyl, 2-Chloräthyl, oder 2-Acetoxyäthyl. In dieser Art veresterte Hydroxygruppen $R_1$ sind z.B. Carbamoyloxy, N-Methylcarbamoyloxy, N-Aethylcarbamoyloxy, N-(2-Chloräthyl)-carbamoyloxy oder N-(2-Acetoxyäthyl)-carbamoyloxy. Entsprechende veresterte Mercaptogruppen $R_1$ sind z.B. Carbamoylthio, N-Methylcarbamoylthio, N-Aethylcarbamoylthio, N-(2-Chloräthyl)-carbamoylthio oder N-(2-Acetoxyäthyl)-carbamoylthio.

Verätherte Hydroxy- und Mercaptogruppen $R_1$ sind beispielsweise mit einem gegebenenfalls substituierten aliphatischen oder araliphatischen Kohlenwasserstoffrest veräthert, und sind insbesondere Niederalkoxy, insbesondere mit 1-4 C-Atomen, in erster Linie Methoxy, sowie Aethoxy, n-Propyloxy oder Isopropyloxy, ferner geradkettiges oder verzweigtes Butyloxy, Benzyloxy oder Diphenylmethoxy oder Niederalkylthio, vorzugsweise mit 1-4 C-Atomen, in erster Linie Methylthio, sowie Aethylthio, n-Propylthio oder Isopropylthio, ferner geradkettiges oder verzweigtes Butylthio, Benzylthio oder Diphenylmethylthio.

Verätherte Mercaptogruppen $R_1$ sind insbesondere durch einen gegebenenfalls substituierten, über ein Ringkohlenstoffatom an die Mercaptogruppe gebundenen, heterocyclischen Rest mit 1 bis 4 Ringstickstoffatomen und gegebenenfalls einem weiteren Ringheteroatom der Gruppe Sauerstoff und Schwefel veräthert.

Solche heterocyclische Reste sind in erster Linie gegebenenfalls substituierte, z.B. die unten genannten Substituenten enthaltende, monocyclische, fünfgliedrige diaza-, triaza-, tetraza-, thiaza-, thiadiaza-, thiatriaza-, oxaza- oder oxadiazacyclische Reste aromatischen Charakters.

Substituenten solcher Heterocyclylreste sind u.a. Niederalkyl, insbesondere Methyl, sowie Aethyl, n-Propyl, Isopropyl oder geradkettiges oder verzweigtes Butyl, oder durch Hydroxy, verestertes Hydroxy, wie Niederalkanoyloxy, Halogen, wie Chlor, Carboxy, verestertes Carboxy, wie Niederalkoxycarbonyl, Sulfo, amidiertes Sulfo, Amino, Mono- oder Diniederalkylamino, Acylamino, wie Niederalkanoylamino, oder durch substituiertes, wie durch Carboxy oder Halogen substituiertes Niederalkanoylamino substituiertes Niederalkyl, beispielsweise 2-Hydroxyäthyl, 2-Acetoxyäthyl, 2-Chloräthyl, Carboxymethyl, 2-Carboxyäthyl, Aethoxycarbonylmethyl, 2-Aethoxycarbonyläthyl, Sulfomethyl, 2-Sulfoäthyl, Sulfamylmethyl, 2-Sulfamyläthyl, 2-Aminoäthyl, 2-Dimethylaminoäthyl oder 2-Acetylaminoäthyl. Weitere Substituenten des heterocyclischen Restes sind Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, oder Heterocyclyl, wie Furyl, z.B. 2-Furyl, Thienyl, z.B. 2-Thienyl, oder Oxazolyl, z.B. 2- oder 5-Oxazolyl, oder

funktionelle Gruppen, wie Halogen, z.B. Fluor, Chlor oder Brom, gegebenenfalls substituiertes Amino, wie gegebenenfalls durch Niederalkyl mono- oder di-substituiertes Amino, z.B. Amino, Methylamino oder Dimethylamino, Acylamino, wie Niederalkanoylamino oder durch Halogen oder Carboxy substituiertes Niederalkanoylamino, wie Acetylamino, 3-Chlorpropionylamino oder 3-Carboxypropionylamino, Nitro, Hydroxy, Niederalkoxy, z.B. Methoxy, Aethoxy, n-Butyloxy oder 2-Aethylhexyloxy, oder gegebenenfalls funktionell abgewandeltes Carboxy, wie Carboxy, verestertes Carboxy, wie Niederalkoxycarbonyl, z.B. Methoxycarbonyl oder Aethoxycarbonyl, gegebenenfalls substituiertes, wie N-mono- oder N,N-diniederalkyliertes Carbamoyl, z.B. N-Methylcarbamoyl oder N,N-Dimethylcarbamoyl, oder Cyan, sowie Oxo oder Oxido, wobei einer oder mehrere solche Substituenten, die in erster Linie mit Ringkohlenstoffatomen aber auch, insbesondere Niederalkyl und Oxido, mit Ringstickstoffatomen verbunden sind, vorhanden sein können.

Bevorzugte heterocyclisch verätherte Mercaptogruppen $R_1$, worin der heterocyclische Rest einen entsprechenden monocyclischen, fünfgliedrigen Rest darstellt, sind u.a. Imidazolylthio, z.B. 2-Imidazolylthio, gegebenenfalls durch Niederalkyl und/oder Phenyl substituiertes Triazolylthio, z.B. 1H-1,2,3-Triazol-4-ylthio, 1-Methyl-1H-1,2,3-triazol-4-ylthio, 1H-1,2,4-Triazol-3-ylthio, 5-Methyl-1H-1,2,4-triazol-3-ylthio, 3-Methyl-1-phenyl-1H-1,2,4-triazol-5-ylthio, 4,5-Dimethyl-4H-1,2,4-triazol-3-ylthio oder 4-Phenyl-4H-1,2,4-triazol-3-ylthio, insbesondere gegebenenfalls wie angegeben substituiertes Tetrazolylthio, z.B. 1H-Tetrazol-5-ylthio, 1-Methyl-1H-tetrazol-5-ylthio, 1-Carboxymethyl-1H-tetrazol-5-ylthio,

1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio, 1-Phenyl-1H-tetrazol-5-ylthio oder 1-(4-Chlorphenyl)-1H-tetrazol-5-ylthio, gegebenenfalls durch Niederalkyl oder Thienyl substituiertes Thiazolylthio oder Isothiazolylthio, z.B. 2-Thiazolylthio, 4-(2-Thienyl)-2-thiazolylthio, 4,5-Dimethyl-2-thiazolylthio, 3-Isothiazolylthio, 4-Isothiazolylthio oder 5-Isothiazolylthio, insbesondere auch gegebenenfalls wie angegeben substituiertes Thiadiazolylthio, z.B. 1,2,3-Thiadiazol-4-ylthio, 1,2,3-Thiadiazol-5-ylthio, 1,3,4-Thiadiazol-2-ylthio, 2-Methyl-1,3,4-thiadiazol-5-ylthio, 2-(3-Carboxypropionylamino)-1,3,4-thiadiazol-5-ylthio, 1,2,4-Thiadiazol-5-ylthio oder 1,2,5-Thiadiazol-3-ylthio, Thiatriazolylthio, z.B. 1,2,3,4-Thiatriazol-5-ylthio, gegebenenfalls wie angegeben substituiertes Oxazolylthio oder Isoxazolylthio, z.B. 5-Oxazolylthio, 4-Methyl-5-oxazolylthio, 2-Oxazolylthio, 4,5-Diphenyl-2-oxazolylthio oder 3-Methyl-5-isoxazolylthio, oder gegebenenfalls wie angegeben substituiertes Oxadiazolylthio, z.B. 1,2,4-Oxadiazol-5-ylthio, 2-Methyl-1,3,4-oxadiazol-5-ylthio, 2-Phenyl-1,3,4-oxadiazol-5-ylthio, 5-(4-Nitrophenyl)-1,3,4-oxadiazol-2-ylthio oder 2-(2-Thienyl)-1,3,4-oxadiazol-5-ylthio.

Bevorzugte heterocyclisch verätherte Mercaptogruppen $R_1$, worin der heterocyclische Rest einen entsprechenden monocyclischen, sechsgliedrigen Rest oder einen entsprechenden partiell gesättigten Rest darstellt, sind u.a. gegebenenfalls durch Halogen substituiertes 1-Oxidopyridylthio, z.B. 1-Oxido-2-pyridylthio oder 4-Chlor-1-oxido-2-pyridylthio, gegebenenfalls durch Hydroxy substituiertes Pyridazinylthio, z.B. 3-Hydroxy-6-pyridazinylthio, gegebenenfalls durch Niederalkyl, Niederalkoxy oder Halogen substituiertes N-Oxido-pyridazinylthio, z.B. 2-Oxido-6-pyridazinylthio, 3-Chlor-1-oxido-6-pyridazinylthio, 3-Methyl-2-oxido-6-pyridazinylthio, 3-Methoxy-1-oxido-6-pyridazinylthio, 3-Aethoxy-1-oxido-6-pyridazinylthio, 3-n-Butyloxy-1-oxido-6-pyridazinylthio oder 3-(2-Aethylhexyloxy)-1-oxido-6-pyridazinylthio, oder gegebenenfalls durch Niederalkyl, Amino, Diniederalkylamino oder Carboxy substituiertes 2-Oxo-1,2-dihydro-pyrimidinylthio, z.B. 2-Oxo-1,2-dihydro-4-pyrimidinylthio, 6-Methyl-2-oxo-1,2-dihydro-4-pyrimidinylthio, 5-Methyl-2-oxo-1,2-dihydro-4-pyrimidinylthio, 6-Amino-2-oxo-1,2-dihydro-4-pyrimidinylthio, 6-Dimethylamino-2-oxo-1,2-dihydro-4-pyrimidinylthio, 5-Carboxy-2-oxo-1,2-dihydro-4-pyrimidinylthio oder 6-Carboxy-2-oxo-1,2-dihydro-4-pyrimidinylthio.

$R_1$ als Halogen ist Fluor, Brom, Jod oder bevorzugt Chlor.

Veresterte und verätherte Hydroxy- und Mercaptogruppen $R_2$ sind die gleichen wie die entsprechenden unter $R_1$ genannten Gruppen.

Quaternäre Ammoniogruppen $R_2$ sind von tertiären organischen Basen, vorzugsweise von entsprechenden aliphatischen Aminen oder in erster Linie von entsprechenden heterocyclischen Stickstoffbasen abgeleitete, über das Stickstoffatom mit dem Methylkohlenstoffatom verbundene, quaternäre Ammoniogruppen.

In einer quaternären Ammoniogruppe $R_2$, die von einer tertiären organischen Base abgeleitet wird, ist das Stickstoffatom an das Methylkohlenstoffatom gebunden und liegt demgemäss in quaternisierter, positiv geladener Form vor. Quaternäre Ammoniogruppen sind u.a. Trinie-

deralkylammonio, z.B. Trimethylammonio, Triäthylammonio, Tripropylammonio oder Tributylammonio, insbesondere aber gegebenenfalls substituierte, z.B. durch Niederalkyl, wie Methyl, Hydroxyniederalkyl, wie Hydroxymethyl, Amino, substituiertes Sulfonamido, wie 4-Aminophenylsulfonamido, Hydroxy, Halogen, wie Fluor, Chlor, Brom oder Jod, Halogenniederalkyl, wie Trifluormethyl, Sulfo, gegebenenfalls funktionell abgewandeltes Carboxy, Cyan, gegebenenfalls durch Niederalkyl, z.B. Methyl oder Aethyl, oder Hydroxyniederalkyl, z.B. Hydroxymethyl, N-mono- oder N,N-disubstituiertes Carbamoyl, z.B. Carbamoyl, N-Methylcarbamoyl oder N,N-Dimethyl-carbamoyl, gegebenenfalls durch Niederalkyl N-substituiertes Hydrazinocarbonyl, z.B. Hydrazinocarbonyl, Carboxyniederalkyl, wie Carboxymethyl, Niederalkanoyl, wie Acetyl, oder 1-Niederalkyl-pyrrolidinyl, wie 1-Methyl-2-pyrrolidinyl, mono- oder polysubstituierte, monocyclische oder bicyclische azacyclische Ammoniogruppen aromatischen Charakters, mit 1 oder 2 Ringstickstoff- und gegebenenfalls einem Ringschwefelatom, wie Pyrimidinio, Pyridazinio, Thiazolio, Chinolinio und in erster Linie Pyridinio.

Heterocyclische Ammoniogruppen $R_2$ sind in erster Linie gegebenenfalls Niederalkyl, Hydroxyniederalkyl, Amino, substituiertes Sulfonamido, Hydroxy, Halogen, Trifluormethyl, Sulfo, Carboxy, Niederalkoxycarbonyl, Cyan, Niederalkanoyl, 1-Niederalkyl-pyrrolidinyl oder gegebenenfalls durch Niederalkyl oder Hydroxyniederalkyl N-substituiertes Carbamoyl enthaltendes Pyridinio, z.B. Pyridinio, 2-, 3- oder 4-Methyl-pyridinio, 3,5-Dimethylpyridinio, 2,4,6-Trimethylpyridinio, 2-, 3- oder 4-Aethyl-pyridinio, 2-, 3- oder 4-Propyl-pyridinio oder insbesondere 4-Hydroxymethylpyridinio, ferner 2-Amino-oder 2-Amino-6-methyl-pyridinio, 2-(4-Aminophenylsulfonylamido)-pyridinio, 3-Hydroxy-pyridinio, 3-Fluor-, 3-Chlor-, 3-Jod- oder insbesondere 3-Brompyridinio, 4-Trifluormethyl-pyridinio, 3-Sulfopyridinio, 2-, 3- oder 4-Carboxy- oder 2,3- oder 3,4-Dicarboxy-pyridinio, 4-Methoxycarbonyl-pyridinio, 3- oder 4-Cyan-pyridinio, 3-Carboxymethyl-pyridinio, 3- oder 4-Acetyl-pyridinio, 3-(1-Methyl-2-pyrrolidinyl)-pyridinio, und insbesondere 4-Carbamoyl-, 3-Carbamoyl-, 3,4-Dicarbamoyl-, 3- oder 4-N-Methylcarbamoyl-, 4-N,N-Dimethylcarbamoyl-, 4-N-Aethylcarbamoyl-, 3-N,N-Diäthylcarbamoyl-, 4-N-Propylcarbamoyl-, 4-Isopropylcarbamoyl- und 4-Hydroxymethyl-carbamoyl-pyridinio.

Die in Verbindungen der Formel I vorhandenen funktionellen Gruppen, insbesondere Carboxyl- und Amino-, ferner Hydroxy- und Sulfogruppen, sind gegebenenfalls durch Schutzgruppen geschützt, die in der Penicillin-, Cephalosporin- und Peptidchemie verwendet werden.

Solche Schutzgruppen sind leicht, das heisst ohne dass unerwünschte Nebenreaktionen stattfinden, beispielsweise solvolytisch, reduktiv, photolytisch oder auch unter physiologischen Bedingungen, abspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lübke, Academic Press, London, New York, 1965, sowie in "Methoden der organischen Chemie", Houben-Weyl, 4. Auflage, Bd. 15/1, Georg Thieme Verlag, Stuttgart, 1974.

So sind Carboxylgruppen z.B. üblicherweise in veresterter Form geschützt, wobei solche Estergruppierungen unter schonenden Bedingungen leicht spaltbar sind.

Geeignete geschützte Carboxylgruppen sind beispielsweise Niederalkoxycarbonyl, z.B. Methoxy- oder Aethoxycarbonyl, oder insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls, z.B. wie oben erwähnt substituiertes Benzyloxycarbonyl, z.B. 4-Nitro-benzyloxycarbonyl oder 4-Methoxybenzyloxycarbonyl, oder gegebenenfalls, z.B. wie oben erwähnt substituiertes Diphenylmethoxycarbonyl, z.B. Diphenylmethoxycarbonyl oder Di-(4-methoxyphenyl)-methoxycarbonyl, oder 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, 2-$(S_1)(S_2)(S_3)$-Silyläthoxycarbonyl, worin die Substituenten $S_1$, $S_2$ und $S_3$ unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit z.B. bis zu 15 C-Atomen, wie einen entsprechenden gegebenenfalls substituierten Niederalkyl-, Arylniederalkyl-, Cycloalkyl- oder Arylrest, bedeuten, z.B. 2-Triniederalkylsilyläthoxycarbonyl, wie 2-Trimethylsilyläthoxycarbonyl oder 2-(Dibutyl-methylsilyl)-äthoxycarbonyl. oder 2-Triarylsilyläthoxycarbonyl, wie 2-Triphenylsilyläthoxycarbonyl, oder 1-Niederalkoxyniederalkoxycarbonyl. wie Methoxymethoxycarbonyl. 1-Methoxyäthoxycarbonyl oder 1-Aethoxymethoxycarbonyl. oder 1-Niederalkylthioniederalkoxycarbonyl, wie 1-Methylthiomethoxycarbonyl oder 1-Aethylthioäthoxycarbonyl

oder Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, oder Polyhalogenaryloxycarbonyl, wie Pentachlorphenyloxycarbonyl. Veresterte Carboxylgruppen sind ebenfalls entsprechende Silyloxycarbonyl-, insbesondere organische Silyloxycarbonylgruppen oder entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.butylsilyl, Niederalkoxy-niederalkyl-halogen-silyl, z.B. Methoxy-methyl-chlor-silyl, oder Diniederalkyl-halogensilyl, z.B. Dimethylchlor-silyl, oder entsprechend substituierte Stannylverbindungen, z.B. Tri-n-butyl-stannyl.

Bevorzugte geschützte Carboxylgruppen sind tert.-Butyloxycarbonyl, gegebenenfalls, z.B. wie erwähnt substituiertes Benzyloxycarbonyl, z.B. 4-Nitrobenzyloxycarbonyl und Diphenylmethoxycarbonyl.

Eine unter physiologischen Bedingungen spaltbare, veresterte Carboxylgruppe ist in erster Linie eine Acyloxymethoxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet. Solche Gruppen sind Niederalkanoyloxymethoxycarbonyl, z.B. Acetyloxymethyloxycarbonyl oder Pivaloyloxymethoxycarbonyl, Aminoniederalkanoyloxymethoxycarbonyl, insbesondere α-Amino-niederalkanoyloxymethoxycarbonyl, z.B. Glycyloxymethoxycarbonyl, L-Valyloxymethoxycarbonyl oder

L-Leucyloxymethoxycarbonyl, ferner Phthalidyloxycarbonyl, z.B. 2-Phthalidyloxycarbonyl, oder Indanyloxycarbonyl, z.B. 5-Indanyloxycarbonyl.

Eine geschützte Aminogruppe kann z.B. in Form einer leicht spaltbaren Acylamino, Mono-, Di- oder Triarylmethylamino-, verätherten Mercaptoamino-, 1-Acyl-2-niederalkylidenamino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 C-Atomen, insbesondere einer gegebenenfalls, z.B. durch Halogen oder Aryl, substituierten aliphatischen Carbonsäure oder gegebenenfalls, z.B. durch Halogen, Niederalkoxy oder Nitro, substituierten aromatischen Carbonsäure, oder eines Kohlensäurehalbesters. Solche Acylgruppen sind beispielsweise Niederalkanoyl, wie Formyl, Acetyl, Propionyl, Halogenniederalkanoyl, wie 2-Halogenacetyl, insbesondere 2-Chlor-, 2-Brom-, 2-Jod-, 2,2-Dichlor- oder 2,2,2-Trichloracetyl, Phenylacetyl, Phenoxyacetyl, Thienylacetyl, Benzoyl, 4-Chlor-, 4-Methoxy- oder 4-Nitrobenzoyl, Niederalkoxycarbonyl, insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl,

oder 2-Halogen-niederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, 2-$(S_1)(S_2)(S_3)$-Silyläthoxycarbonyl, worin die Substituenten $S_1$, $S_2$ und $S_3$ unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie einen entsprechenden gegebenenfalls substituierten Niederalkyl-, Arylniederalkyl-, Cycloalkyl- oder Arylrest, bedeuten, z.B. 2-Triniederalkylsilyläthoxycarbonyl, wie 2-Trimethylsilyläthoxycarbonyl oder 2-(Dibutyl-methyl-silyl)-äthoxycarbonyl, oder 2-Triarylsilyläthoxycarbonyl, wie 2-Triphenylsilyläthoxycarbonyl, oder Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl.

Weitere Acylgruppen in Acylaminogruppen sind Reste organischer Phosphor-, Phosphon- oder Phosphinsäuren der Formel $(R^1)(R^2)P(=O)-$, worin $R^1$ und $R^2$ unabhängig voneinander eine durch einen Kohlenwasserstoffrest verätherte Hydroxygruppe oder einen Kohlenwasserstoffrest bedeuten, wobei die Kohlenwasserstoffreste bevorzugt bis zu 15 C-Atome enthalten, beispielsweise aliphatischer, araliphatischer, cycloaliphatischer oder aromatischer Natur sind, gegebenenfalls z.B. durch Niederalkyl, Niederalkoxy, Aryl, Halogen oder Nitro substituiert sind, und beispielsweise Alkyl, insbesondere Niederalkyl, wie Methyl, Aethyl, Propyl, Isopropyl oder Butyl, Phenylniederalkyl, wie Benzyl, p-Nitro- oder p-Chlorbenzyl, Cycloalkyl, wie Cyclopropyl, Cyclopentyl,

Cyclohexyl oder Cycloheptyl, oder Homoaryl, wie Phenyl, o-, m- oder p-Tolyl, p-Methoxyphenyl, p-Biphenylyl, p-Chlorphenyl oder p-Nitrophenyl, bedeuten. Solche Reste sind beispielsweise Dimethylphosphoryl, Diäthylphosphoryl, Dipropylphosphoryl, Diisopropylphosphoryl, Dibenzylphosphoryl, Di-p-nitrobenzylphosphoryl, Dicyclohexylphosphoryl, Diphenylphosphoryl, Phenoxy-phenyl-phosphonyl, Diäthylphosphinyl und Diphenylphosphinyl.

In einer Mono-, Di- oder Triarylmethylaminogruppe sind die Arylreste insbesondere gegebenenfalls substituierte Phenylreste. Solche Gruppen sind beispielsweise Benzyl-, Diphenylmethyl- oder Tritylamino.

Eine verätherte Mercaptogruppe in einer mit einem solchen Rest geschützten Aminogruppe is in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 1-Acyl-2-niederalkylidenrest ist Acyl vorzugsweise der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure oder eines Kohlensäurehalbesters, wie eines Kohlensäure-niederalkylhalbesters. Entsprechende Schutzgruppen sind in erster Linie 1-Niederalkanoyl-2-propyliden, z.B. 1-Acetyl-2-propyliden, oder 1-Niederalkoxycarbonyl-2-propyliden, z.B. 1-Aethoxycarbonyl-2-propyliden.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlor-silyl, oder entsprechend substituiertes Stannyl, z.B. Tri-n-butylstannyl.

Eine Aminogruppe kann auch in protonierter Form geschützt werden; als Anionen kommen in erster Linie diejenigen von starken anorganischen Säuren, wie von Halogenwasserstoffsäuren, z.B. das Chlor- oder Bromanion, oder von Sulfonsäure, wie p-Toluolsulfonsäure, in Frage.

Bevorzugte Aminoschutzgruppen sind die Acylreste von Kohlensäurehalbestern, insbesondere tert.-Butyloxycarbonyl, gegebenenfalls, z.B. wie angegeben substituiertes Benzyloxycarbonyl oder Diphenylmethoxycarbonyl, oder 2-Halogen-niederalkoxycarbonyl, wie 2,2,2-Trichloräthoxycarbonyl.

Hydroxyschutzgruppen sind z.B. Acylreste, wie 2,2-Dichloracetyl oder insbesondere die im Zusammenhang mit einer geschützten Aminogruppe genannten Acylreste von Kohlensäurehalbestern, insbesondere 2,2,2-Trichloräthoxycarbonyl, oder organischen Silyl- oder Stannylreste, ferner leicht abspaltbare 2-oxa- oder 2-thiaaliphatische oder -cycloaliphatische Kohlenwasserstoffreste, in erster Linie 1-Niederalkoxy-niederalkyl oder

1-Niederalkylthio-niederalkyl, z.B. Methoxymethyl, 1-Methoxyäthyl, 1-Aethoxy-äthyl, 1-Methylthiomethyl, 1-Methylthio-äthyl oder 1-Aethylthioäthyl, oder 2-Oxa- oder 2-Thiacycloniederalkyl mit 5-7 Ringatomen, z.B. 2-Tetrahydrofuryl oder 2-Tetrahydropyranyl oder entsprechende Thiaanaloge, sowie leicht abspaltbare, gegebenenfalls substituierte α-Phenylniederalkylreste, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine geschützte Sulfogruppe ist in erster Linie eine mit einem aliphatischen, cycloaliphatischen, cycloaliphatisch-aliphatischen, aromatischen oder araliphatischen Alkohol, wie einem Niederalkanol, oder mit einem Silyl- oder Stannylrest, wie Triniederalkylsilyl, veresterte Sulfogruppe. In einer Sulfogruppe kann die Hydroxygruppe beispielsweise wie die Hydroxygruppe in einer veresterten Carboxygruppe veräthert sein.

Salze sind insbesondere diejenigen von Verbindungen der Formel I mit sauren Gruppen, z.B. mit freien Carboxyl- und Sulfogruppen. Solche Salze sind in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-,Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder

Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethyl-piperidin, Cycloalkylamine, z.B. Dicyclohexylamin, oder Benzylamine, z.B. N,N'Dibenzyl-äthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren, wie Salzsäure, Schwefelsäure, oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono- oder Polysalze gebildet werden. Verbindungen der Formel I mit einer freien Carboxylgruppe und freien Aminogruppe können auch in Form von inneren Salzen, d.h. in zwitterionischer Form vorliegen, oder es kann ein Teil des Moleküls, z.B. die endständige Aminocarbonsäurefunktion, als inneres Salz, und ein anderer Teil, z.B. die 4-Carboxylgruppe, als normales Salz vorliegen.

Zur Isolierung oder Reinigung können auch pharmazeutisch ungeeignete Salze Verwendung finden. Zur therapeutischen Anwendung gelangen nur pharmazeutisch unbedenkliche Salze, die deshalb bevorzugt werden.

Der Acylrest an der 7β-Aminogruppe enthält ein oder gegebenenfalls zwei Asymmetriezentren. Das gegebenenfalls in A vorhandene Asymmetriezentrum, nämlich wenn A durch Amino, Hydroxyl, Carboxyl oder Sulfo substituiertes Methylen ist, liegt in der R,S- oder bevorzugt in der R-Konfiguration vor. Das Asymmetriezentrum an der endständigen Aminocarbonsäuregruppierung kann die R-, S- oder R,S-Konfiguration besitzen, wobei die R-Konfiguration bevorzugt ist.

Die Gruppe =N-O-$R^{O}$ kann in der syn- oder anti-Form vorliegen, wobei die syn-Form bevorzugt ist.

Die substituierte Aminothiazolylgruppe der Teilformel

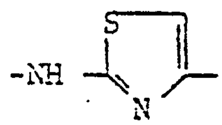

kann auch in ihrer tautomeren Form als Iminothiazolinylgruppe der Teilformel

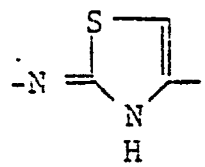

oder als Mischung beider Formen vorliegen. Das Gleichgewicht zwischen den beiden Tautomeren hängt von der Art der Substituenten und äusseren Faktoren, wie Temperatur, Lösungsmittel oder pH-Wert, ab. Im Rahmen der vorliegenden Erfindung wird diese Gruppe in der Beschreibung und in den Ansprüchen nur als "Aminothiazolyl" bezeichnet, obgleich die Iminothiazolinylform ebenfalls umfasst werden soll.

Die Verbindungen der Formel I, worin Carboxylgruppen gegebenenfalls in physiologisch spaltbarer Form verestert sind, und ihre pharmazeutisch verwendbaren, nichttoxischen Salze sind wertvolle, antibiotisch wirksame Substanzen, die insbesondere als antibakterielle Antibiotika verwendet werden können. Beispielsweise sind sie <u>in vitro</u> gegen gram-positive und gram-negative Mikroorganismen, wie gegen Kokken, z.B. Staphylokokken, Streptokokken und Mikrokokken, inklusive *Neisseria* Arten, in Minimalkonzentrationen von etwa 0,05 mcg/ml bis etwa 32 mcg/ml und gegen Enterobacteriaceae, z.B. gegen *Haemophilus*, in Minimalkonzentrationen von etwa 0,5 mcg/ml,

bis 128 mcg/ml , wirksam. In vivo, bei subcutaner Application an der Maus, sind sie beispielsweise gegen grampositive Infektionserreger (bei akuter Infektion in Minimaldosen von etwa 10 bis 30 mg/kg) und gegen gramnegative Erreger (bei akuter Infektion in Minimaldosen von etwa 0,8-100 mg/kg),und insbesondere gegen Enterobakterien, inklusive β-Lactamase bildende, z.B. gegen *Proteus morganii* und *Proteus mirabilis* (bei akuter Infektion $ED_{50}$ 8 mg/kg), wirksam. Die neuen Verbindungen können deshalb entsprechend, z.B. in Form von antibiotisch wirksamen Präparaten zur Behandlung von durch gram-positive oder gram-negative Bakterien und Kokken, insbesondere von durch Enterobakterien, wie *Proteus morganii* und *Proteus mirabilis*, verursachten Infektionen Verwendung finden.

Verbindungen der Formel I, worin die funktionellen Gruppen geschützt sind werden als Ausgangsmaterialien zur Herstellung von Verbindungen der Formel I verwendet.

Die vorliegende Erfindung betrifft in erster Linie diejenigen Verbindungen der Formel I, worin die Gruppe $-(C_nH_{2n})-$ unverzweigt ist und n die angegebene Bedeutung hat, X Sauerstoff, Schwefel oder -NH-, Y Sauerstoff, A Methylen, Aminomethylen, Hydroxymethylen, Carboxymethylen, Sulfomethylen, Hydroxyiminomethylen oder Methoxyiminomethylen, $R_1$ Wasserstoff, Niederalkyl, Niederalkoxy, Halogen oder eine Gruppe der Formel $-CH_2-R_2$, worin $R_2$ Niederalkanoyloxy, Carbamoyloxy, N-Niederalkylcarbamoyloxy, Triazolylthio, Tetrazolylthio, Thiazolylthio, Thiatriazolylthio, Thiadiazolylthio, Oxazolylthio, Oxadiazolylthio oder Pyridinio darstellt, worin die heterocyclischen Ringe gegebenenfalls beispielsweise durch Niederalkyl, N,N-Diniederalkylaminonieder-

alkyl, Carboxyniederalkyl, Sulfoniederalkyl, Amino, Carboxyniederalkanoylamino oder Carbamoyl substituiert sein können, und $R_3$ Wasserstoff oder Methoxy darstellen, pharmazeutisch verwendbare Salze von solchen Verbindungen, sowie die zu ihrer Herstellung verwendbaren Ausgangsstoffe und Zwischenprodukte.

Besonders hervorzuheben sind Verbindungen der Formel I, worin die Gruppe $-(C_nH_{2n})-$ unverzweigt ist und n die angegebene Bedeutung hat, X Sauerstoff, Schwefel oder -NH- bedeutet, Y Sauerstoff, A Methylen, Aminomethylen, Hydroxymethylen oder Methoxyiminomethylen ist, $R_1$ Wasserstoff, Methyl, Methoxy, Chlor, oder eine Gruppe der Formel $-CH_2-R_2$ bedeutet, worin $R_2$ Acetoxy, Carbamoyloxy, Tetrazolylthio, insbesondere 1-Methyl-1H-tetrazol-5-ylthio, 1-Sulfomethyl-1H-tetrazol-5-ylthio, 1-Carboxymethyl-1H-tetrazol-5-ylthio, oder 1-(2-Dimethyl-aminoäthyl)-1H-tetrazol-5-ylthio, oder Thiadiazolylthio, insbesondere 2-Methyl-1,3,4-thiadiazol-5-ylthio, oder Carbamoylpyridinio, insbesondere 4-Carbamoylpyridinio, darstellt, und $R_3$ Wasserstoff bedeutet, pharmazeutisch verwendbare Salze von solchen Verbindungen, sowie die zu ihrer Herstellung verwendbaren Ausgangsstoffe und Zwischenprodukte.

Die Erfindung betrifft insbesondere die in den Beispielen beschriebenen Verbindungen der Formel I, deren pharmazeutisch unbedenklichen Salze, sowie die dort beschriebenen Ausgangsstoffe und Zwischenprodukte.

Die Verbindungen der vorliegenden Erfindung werden nach an sich bekannten Verfahren erhalten.

Verbindungen der Formel I, worin die Carboxylgruppen gegebenenfalls in physiologisch spaltbarer Form verestert sind, sowie ihre Salze, werden hergestellt, indem man in einer der Formel I entsprechenden Ausgangsverbindung, worin mindestens eine der vorhandenen funktionellen Gruppen geschützt ist, die funktionelle(n) Gruppe(n) freisetzt, wenn erwünscht, in einer erhaltenen Verbindung einen Rest $R_1$ in einen anderen Rest $R_1$ überführt, und/oder, wenn erwünscht, eine freie Carboxylgruppe in eine physiologisch spaltbare veresterte Carboxylgruppe überführt, und/oder, wenn erwünscht, ein erhaltenes Isomerengemisch in die einzelnen Isomeren auftrennt, und/oder, wenn erwünscht, eine erhaltene Verbindung in ein Salz oder ein erhaltenes Salz in eine freie Verbindung oder in ein anderes Salz überführt.

In den Ausgangsverbindungen der Formel I, worin funktionelle Gruppen geschützt sind, werden diese, z.B. geschützte Carboxyl-, Amino-, Hydroxy-, Mercapto- und/oder Sulfogruppen, in an sich bekannter Weise, wie mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt.

So kann man z.B. eine gegebenenfalls in 2-Stellung durch eine Silylgruppe oder in 1-Stellung durch Niederalkoxy oder Niederalkylthio substituierte Niederalkoxycarbonylgruppe, eine Polycycloalkoxycarbonyl- oder eine Diphenylmethoxycarbonylgruppe durch Behandeln mit einem geeigneten sauren Mittel, wie Ameisensäure oder Trifluoressigsäure, gegebenenfalls unter Zugabe einer nucleophilen Verbindung, wie Phenol oder Anisol, in die freie Carboxylgruppe überführen. Eine gegebenenfalls substituierte Benzyloxycarbonylgruppe kann z.B. mittels Hydrogenolyse durch Behandeln mit Wasserstoff in Gegenwart eines Hydrierkatalysators, wie eines Palladiumkatalysators freigesetzt werden. Ferner kann man bestimmt substituierte Benzyloxycarbonylgruppen, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoffabgebenden Mittels, das zusammen mit dem Metall nascierenden Wasserstoff zu erzeugen vermag, wie einer Säure, in erster Linie einer Carbonsäure oder α-Hydroxycarbonsäure, wie insbesondere Essigsäure, Ameisensäure, Glycolsäure, Diphenylglycolsäure, Milchsäure, Mandelsäure, p-Chlormandelsäure, Weinsäure, und dergleichen, oder eines Alkohols oder Thiols, wobei man vorzusweise Wasser zugibt, in die freie Carboxylgruppe überführen. Durch Behandeln mit einem reduzierenden Metall oder Metallsalz, wie oben beschrieben, kann man auch eine 2-Halogen-niederalkoxycarbonylgruppe (gegebenenfalls nach Umwandlung einer 2-Brom-niederalkoxycarbonylgruppe in eine 2-Iodniederalkoxycarbonylgruppe) oder eine Aroylmethoxycarbonylgruppe in die freie Carboxylgruppe umwandeln, wobei eine Aroylmethoxycarbonylgruppe ebenfalls durch Behandeln mit einem nucleophilen, vorzugsweise salzbildenden Reagens, wie Natriumthiophenolat oder Natriumjodid gespalten werden kann. Eine substituierte Silyläthoxycarbonylgruppe kann auch durch Behandeln mit einem Salze der Fluorwasserstoffsäure, das Fluoridanion liefert, wie einem Alkalimetallfluorid, z.B. Natrium- oder Kaliumfluorid, in Anwesenheit eines macrocyclischen Polyäthers ("Kronenäther"), oder mit einem Fluorid einer or-

ganischen quaternären Base, wie Tetraalkylammoniumfluorid oder Trialkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in die freie Carboxylgruppe übergeführt werden. Eine Polyhalogenaryloxycarbonylgruppe, wie die Pentachlorphenyloxycarbonylgruppe,wird unter milden basischen Bedingungen, wie durch verdünnte Natronlauge oder organische Basen in Gegenwart von Wasser, zur freien Carboxylgruppe verseift.

Eine z.B. durch Silylierung oder Stannylierung geschützte Carboxylgruppe kann in üblicher Weise, z.B. durch Behandeln mit Wasser oder einem Alkohol, freigesetzt werden.

Eine geschützte Aminogruppe wird in an sich bekannter und je nach Art der Schutzgruppe in verschiedenartiger Weise, z.B. mittels Solvolyse oder Reduktion, freigesetzt. Eine 2-Halogen-niederalkoxycarbonylaminogruppe (gegebenenfalls nach Umwandlung einer 2-Brom-niederalkoxycarbonylgruppe in eine 2-Jod-niederalkoxycarbonylgruppe), eine Acylmethoxycarbonylaminogruppe oder eine 4-Nitrobenzyloxycarbonylaminogruppe kann z.B. durch Behandeln mit einem geeigneten chemischen Reduktionsmittel, wie Zink in Gegenwart einer Carbon- oder α-Hydroxycarbonsäure, wie wässriger Essigsäure, eine Arcylmethoxycarbonylaminogruppe auch durch Behandeln mit einem nucleophilen, vorzugsweise salzbildenden Reagens, wie Natriumthiophenolat, und eine 4-Nitro-benzyloxycarbonylaminogruppe auch durch Behandeln mit einem Alkalimetall-, z.B. Natriumdithionit, eine Diphenylmethoxycarbonylamino-, tert.-Niederalkoxycarbonylamino-, 2-($S_1$)($S_2$)($S_3$)-Silyläthoxycarbonylamino- oder Polycycloalkoxycarbonylaminogruppe durch Behandeln z.B. mit Ameisen- oder Trifluoressigsäure, eine gegebenenfalls substituierte Benzyloxycarbonylaminogruppe z.B.

mittels Hydrogenolyse durch Behandeln mit Wasserstoff in Gegenwart eines Hydrierkatalysators, wie eines Palladiumkatalysators, eine Triarylmethylgruppe z.B. durch Behandeln mit wässriger Mineralsäure, und eine mit einer organischen Silyl- oder Stannylgruppe geschützte Aminogruppe z.B. mittels Hydrolyse oder Alkoholyse freigesetzt werden. Eine durch 2-Halogenacetyl, wie 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs und anschliessende Solvolyse, wie Alkoholyse oder Hydrolyse, des entstandenen Kondensationsproduktes freigesetzt werden. Eine durch eine substituierte-Silyläthoxycarbonylgruppe geschützte Aminogruppe kann auch durch Behandeln mit einem Salz der Fluorwasserstoffsäure, das Fluoridanionen liefert, wie oben bei der Freisetzung einer entsprechend geschützten Carboxylgruppe angegeben, in die freie Aminogruppe übergeführt werden. Eine Phosphor-, Phosphon- oder Phosphinamidogruppe kann durch Behandeln mit einer Phosphor-haltigen Säure, wie einer Phosphor-Phosphon- oder Phosphinsäure, z.B. Orthophosphorsäure oder Polyphosphorsäure, einem sauren Ester davon, z.B. Monomethyl-, Monoäthyl-, Dimethyl- oder Diäthylphosphat oder Monomethylphosphonsäure, oder einem Anhydrid davon, wie Phosphorpentoxid, und dergleichen, in die freie Aminogruppe übergeführt werden.

Eine in Form einer Azidogruppe geschützte Aminogruppe wird in an sich bekannter Weise durch Reduktion, in die freie Aminogruppe übergeführt, beispielsweise durch katalytische Hydrierung mit Wasserstoff und einem Hydrierkatalysator, wie Platinoxid, Palladium. oder auch Raney-Nickel, oder auch durch Zink und Säure, wie Essigsäure. Die katalytische Hydrierung wird bevorzugt in einem inerten Lösungsmittel, wie einem halogenierten Kohlenwasserstoff, z.B. Methylenchlorid, oder auch in Wasser oder

einem Gemisch von Wasser und einem organischen Lösungsmittel, wie einem Alkohol oder Dioxan, bei etwa 20 bis 25°, oder auch bei erniedrigter oder erhöhter Temperatur, durchgeführt.

Eine durch eine Acylgruppe, eine Silyl- oder Stannylgruppe oder durch einen gegebenenfalls substituierten α-Phenylniederalkylrest geschützte Hydroxygruppe wird wie eine entsprechend geschützte Aminogruppe freigesetzt. Eine durch 2,2-Dichloracetyl geschützte Hydroxygruppe wird durch basische Hydrolyse und eine durch einen 2-oxa- oder 2-thia-aliphatischen oder -cycloaliphatischen Kohlenwasserstoffrest geschützte Hydroxygruppe wird durch Acidolyse freigesetzt.

Eine geschützte Sulfogruppe wird analog einer geschützten Carboxylgruppe freigesetzt.

Bevorzugt werden die Schutzgruppen so gewählt, dass sie alle gleichzeitig abgespalten werden können, beispielsweise acidolytisch, wie durch Behandeln mit Trifluoressigsäure oder Ameisensäure, oder reduktiv, wie durch Behandeln mit Zink und Eisessig, oder mit Wasserstoff und einem Hydrierkatalysator, wie einem Palladium/Kohle-Katalysator.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

In einer Verbindung der Formel I, worin eine Aminogruppe, wenn notwendig, geschützt ist, und worin die Carboxylgruppe in 4-Stellung des Cephemringes in freier Form vorliegt, kann man in an sich bekannter Weise eine Gruppe $R_1$ durch einen anderen Rest $R_1$ ersetzen oder in einen anderen Rest $R_1$ umwandeln. So ist es z.B. möglich, in einer Verbindung der Formel I, worin $R_1$ eine Gruppe der Formel $-CH_2-R_2$ bedeutet, und $R_2$ z.B. einen, durch

nucleophile Substituenten ersetzbaren Rest darstellt, oder in einem Salz davon, durch Behandeln mit einer entsprechenden Mercaptan- oder mit einer Thiocarbonsäureverbindung einen solchen Rest $R_2$ durch eine verätherte bzw. veresterte Mercaptogruppe $R_2$ zu ersetzen. Ein geeigneter, durch eine verätherte Mercaptogruppe ersetzbarer Rest ist beispielsweise eine durch eine niederaliphatische Carbonsäure veresterte Hydroxygruppe. Solche veresterten Hydroxygruppen sind inbesondere Acetyloxy, ferner Formyloxy.

Die Reaktion einer solchen Verbindung mit einer geeigneten Mercaptanverbindung kann unter neutralen oder schwach basischen Bedingungen in Gegenwart von Wasser und gegebenenfalls einem mit Wasser mischbaren organischen Lösungsmittel durchgeführt werden. Die basischen Bedingungen können beispielsweise durch Zugabe einer anorganischen Base, wie eines Alkalimetall- oder Erdalkalimetallhydroxids, -carbonats oder -hydrogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, oder Nitrile, z.B. Niederalkancarbonsäurenitrile, wie Acetonitril, und ähnliche verwendet werden.

Veresterte Hydroxygruppen $R_2$ in einer Verbindung der Formel I, worin $R_1$ die Gruppe $-CH_2-R_2$ bedeutet, wobei $R_2$ für eine, durch den Acylrest eines gegebenenfalls substituierten Halbamids der Kohlensäure veresterte Hydroxygruppe steht, kann man z.B. einführen, indem man eine entsprechende Verbindung der Formel I, worin $R_2$ für freies Hydroxy steht (das man z.B. durch Abspaltung des Acetylrestes aus einer Acetyloxygruppe $R_2$, z.B. durch Hydrolyse in schwach-basischem Medium, wie mit einer

wässrigen Natriumhydroxydlösung bei pH 9-10, oder durch Behandeln mit einer geeigneten Esterase, wie einem entsprechenden Enzym aus Rhizobium tritolii, Rhizobium lupinii, Rhizobium japonicum oder Bacillus subtilis, oder einer geeigneten Citrus-Esterase, z.B. aus Orangenschalen, freisetzen kann), mit einem geeigneten Kohlensäurederivat, insbesondere mit einer Isocyanat- oder Carbaminsäureverbindung, wie einem Silylisocyanat, z.B. Silyltetraisocyanat, einem Sulfonylisocyanat, z.B. Chlorsulfonylisocyanat, oder Carbaminsäurehalogenid, z.B. -chlorid (die zu N-unsubstituierten 3-Aminocarbonyloxymethyl-Verbindungen führen), oder dann mit einer N-substituierten Isocyanat- oder mit einer N-mono- oder N,N-disubstituierten Carbaminsäure-Verbindung , wie einem entsprechenden Carbaminsäurehalogenid, z.B. -chlorid, umsetzt, wobei man üblicherweise in Gegenwart eines Lösungs- oder Verdünnungsmittels und, wenn notwendig, unter Kühlen oder Erwärmen, in einem geschlossenen Gefäss und/oder in einer Inertgas-, z.B. Stickstoffatmosphäre, arbeitet.

Ferner kann man eine Verbindung der Formel I, worin $R_1$ eine Gruppe $-CH_2-R_2$ bedeutet, wobei $R_2$ z.B. den oben definierten, durch nucleophile Substitution ersetzbaren Rest darstellt, mit einer tertiären organischen Base, insbesondere einem gegebenenfalls substituierten Pyridin, unter neutralen oder schwach sauren Bedinungen, bevorzugt bei einem pH-Wert von etwa 6,5, in Gegenwart von Wasser und gegebenenfalls in einem mit Wasser mischbaren organischen Lösungsmittel umsetzen und so zu Verbindungen der Formel I gelangen, worin $R_1$ den Rest der Formel $-CH_2-R_2$ darstellt und $R_2$ für eine quaternäre Ammoniogruppe steht. Die schwach-sauren Bedingungen können durch Zugabe einer geeigneten organischen oder anorganischen Säure, beispielsweise Essigsäure, Chlorwasserstoffsäure, Phosphorsäure oder auch Schwefelsäure eingestellt werden. Als organische Lösungsmittel können beispielsweise die vorstehend genannten, mit Wasser mischbaren Lösungsmittel verwendet werden. Zur Erhöhung der Ausbeute können der Reaktionsmischung gewisse Salze zugesetzt werden, beispielsweise Alkalimetall-, wie Natrium- und insbesondere Kaliumsalze, von anorganischen Säuren, wie Halogenwasserstoffsäuren, z.B. Chlorwasserstoff- und insbesondere Jodwasserstoffsäure, sowie der Thiocyansäure, oder organischen Säuren, wie Niederalkancarbonsäuren, z.B. Essigsäure. Vertreter solcher Salze sind beispielsweise Kaliumjodid und Kaliumthiocyanat. Auch Salze von geeigneten Anionenaustauschern, z.B. flüssige Ionenaustauscher in Salzform, wie z.B. Amberlite LA-1 (flüssige sekundäre Amine mit einem Molekulargewicht von 351-393; Oel-löslich und Wasser-unlöslich; mAeq./g = 2,5-2,7, z.B. in Acetatform), mit Säuren, z.B. Essigsäure, können für diesen Zweck verwendet werden.

Quaternäre Ammoniogruppen $R_2$ können vorteilhafterweise unter Verwendung eines Zwischenprodukts der Formel I, in welchem $R_2$ für eine substituierte, insbesondere für eine aromatisch substituierte Carbonylthiogruppe und in erster Linie für die Benzoylthiogruppe steht, hergestellt werden. Ein solches Zwischenprodukt, das man z.B. durch Umsetzen einer Verbindung der Formel I, worin $R_2$ im Rest $R_1$ eine veresterte Hydroxygruppe, insbesondere eine Niederalkanoyloxy-, z.B. Acetyloxygruppe, bedeutet, mit einem geeigneten Salz, wie einem Alkalimetall-, z.B. Natriumsalz, einer Thiocarbonsäure, wie einer aromatischen Thiocarbonsäure, z.B. Thiobenzoesäure, erhalten kann, wird mit dem tertiären Amin, insbesondere einer tertiären heterocyclischen Base, wie einem gegebenenfalls substituierten Pyridin, umgesetzt, wobei man die quaternäre Ammonioverbindung erhält. Die Reaktion wird üblicherweise in Gegenwart eines geeigneten Entschwefelungsmittels, insbesondere eines Quecksilbersalzes, z.B.

Quecksilber-II-perchlorat, und eines geeigneten Lösungs- oder Verdünnungsmittels oder eines Gemisches davon, wenn notwending, unter Kühlen oder Erwärmen, in einem geschlossenen Gefäss und/oder in einer Inertgas-, z.B. Stickstoffatmosphäre, durchgeführt.

Die Ueberführung einer freien Carboxylgruppe in einer erhaltenen Verbindung der Formel I in eine veresterte Carboxylgruppe, die unter physiologischen Bedingungen spaltbar ist, erfolgt nach an sich bekannten Veresterungsmethoden, beispielsweise indem man eine Verbindung der Formel I, worin andere funktionelle Gruppen, wie Amino-, Hydroxy- oder Sulfogruppen, gegebenenfalls in geschützter Form vorliegen, oder ein bezüglich der zu veresternden Carboxylgruppe reaktionsfähiges funktionelles Derivat davon, oder ein Salz davon, mit einem entsprechenden Alkohol oder einem reaktionsfähigen funktionellen Derivat davon, verestert.

Salze von Verbindungen der Formel I können in an sich bekannter Weise hergestellt werden. So kann man Salze von Verbindungen der Formel I mit sauren Gruppen, z.B. durch Behandeln mit Metallverbindungen, wie Alkalimetallsalzen von geeigneten Carbonsäuren, z.B. dem Natriumsalz der α-Aethylcapronsäure oder Natriumbicarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche eine freie Carboxylgruppe enthalten, können z.B. durch Neutralisieren von Salzen, wie

Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Die Ausgangsverbindungen der Formel I, worin mindestens eine der funktionellen Gruppen in geschützter Form vorliegt, und Verfahren zu ihrer Herstellung sind ebenfalls Gegenstand der vorliegenden Erfindung. Diese Verbindungen können in an sich bekannter Weise hergesllt werden, indem man z.B.

a) in einer Verbindung der Formel

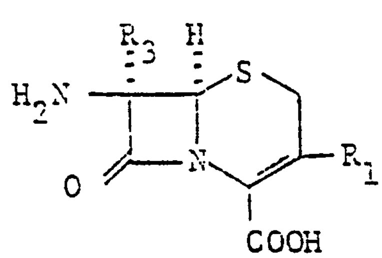

(II),

worin die Aminogruppe gegebenenfalls durch eine die Acylierung erlaubende Gruppe substituiert ist, und worin die 4-Carboxylgruppe und gegebenenfalls im Rest $R_1$ vorhandene weitere funktionelle Gruppen in geschützter Form vorliegen können, die Aminogruppe durch Behandeln mit einem den Acylrest einer Carbonsäure der Formel

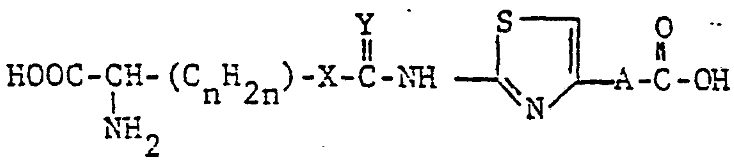

(III)

einführenden Acylierungsmittel, worin die Aminocarbonsäuregruppierung HOOC-CH($NH_2$)- und gegebenenfalls in der Gruppierung A vorhandene weitere funktionelle Gruppen in geschützter Form vorliegen, acyliert, oder

b) in einer Verbindung der Formel

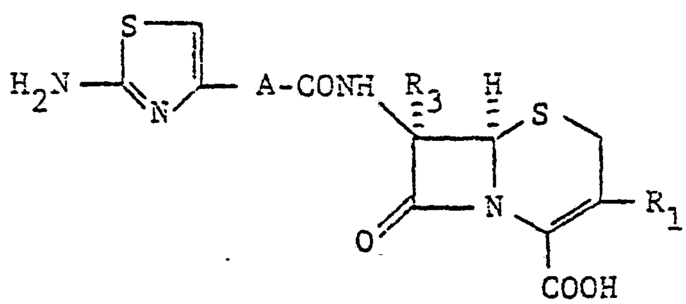

(IV),

worin die Aminogruppe gegebenenfalls durch eine die Acylierung erlaubende Gruppe substituiert ist, und worin die 4-Carboxylgruppe und gegebenenfalls im Rest $R_1$ und in der Gruppierung A vorhandene weitere funktionelle Gruppen in geschützter Form vorliegen können, die Aminogruppe durch Behandeln mit einem den entsprechenden Acylrest einer Carbonsäure der Formel

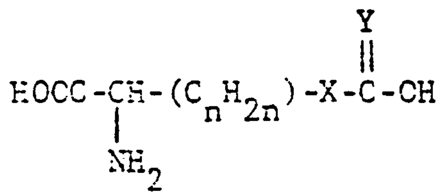 (V)

einführenden Acylierungsmittel, worin die Aminocarbonsäuregruppierung $HOOC-CH(NH_2)-$ in geschützter Form vorliegt, acyliert, oder

c) in einer Verbindung der Formel

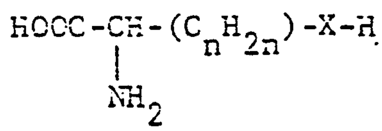 (VI),

worin X Sauerstoff, Schwefel oder die Gruppe -NH- bedeutet, und worin die Aminocarbonsäuregruppierung $HOOC-CH(NH_2)-$ in geschützter Form vorliegt, die Gruppe -X-H mit einem den entsprechenden Acylrest einer Carbonsäure der Formel

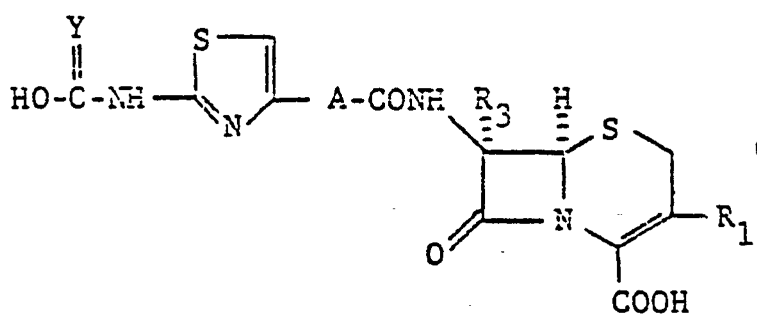 (VII)

einführenden Acylierungsmittel, worin die 4-Carboxylgruppe und gegebenenfalls im Rest $R_1$ und in der Gruppierung A vorhandene funktionelle Gruppen in geschützter Form vorliegen können, acyliert, oder

d) eine Verbindung der Formel

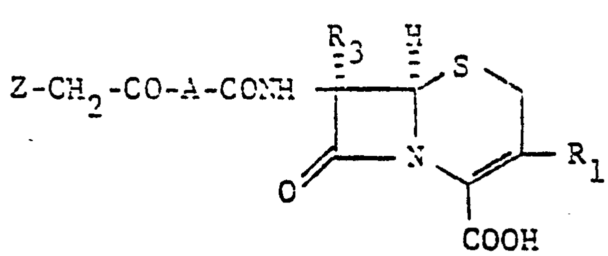 (VIII),

worin Z Halogen bedeutet, und worin funktionelle Gruppen gegebenenfalls geschützt sind, oder ein Salz davon, mit einem Thioharnstoff der Formel

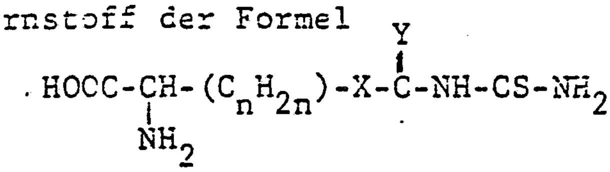

(IX),

worin funktionelle Gruppen geschützt sind, oder einem Salz davon, kondensiert, oder

e) zur Herstellung von Verbindungen der Formel I, worin $R_1$ Wasserstoff ist, in einer Verbindung der Formel

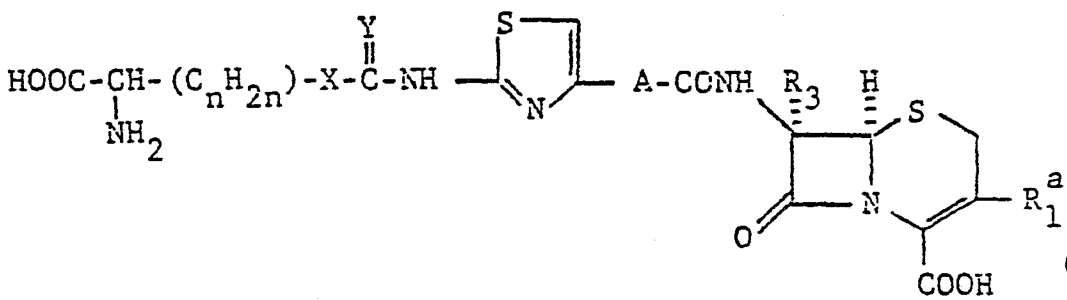

(X),

worin $R_1^a$ eine gegebenenfalls veresterte Hydroxygruppe oder eine sekundäre oder tertiäre Aminogruppe und $R_3$ Wasserstoff ist, und worin funktionelle Gruppen gegebenenfalls geschützt sind, die Gruppe $R_1^a$ durch Wasserstoff ersetzt oder

f) zur Herstellung von Verbindungen der Formel I, worin $R_1$ Wasserstoff ist und worin funktionelle Gruppen geschützt sind, eine Verbindung der Formel

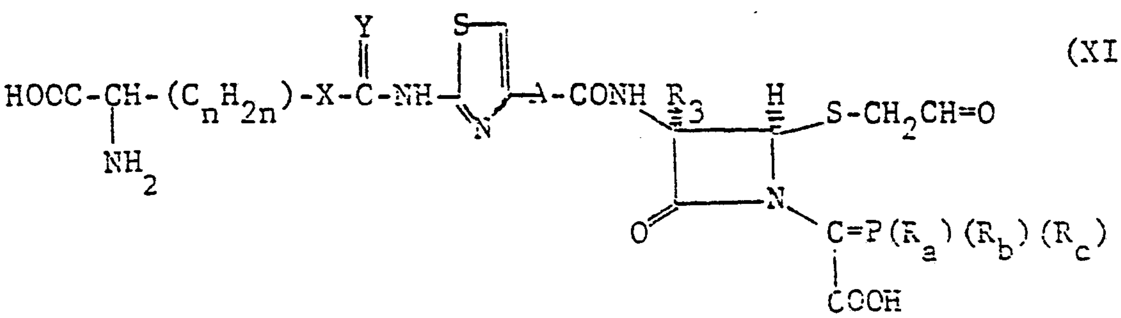

(XI),

worin $R_3$ Wasserstoff ist, jeder der Reste $R_a$, $R_b$ und $R_c$ einen gegebenenfalls substituierten Kohlenwasserstoffrest darstellt, und worin funktionelle Gruppen geschützt sind, unter

Abspaltung von $O=P(R_a)(R_b)(R_c)$ ringschliesst, oder

g) zur Herstellung von Verbindungen der Formel I, worin $R_1$ Niederalkyl, eine veresterte oder verätherte Hydroxygruppe oder Halogen bedeutet, und worin funktionelle Gruppen geschützt sind, eine Verbindung der Formel

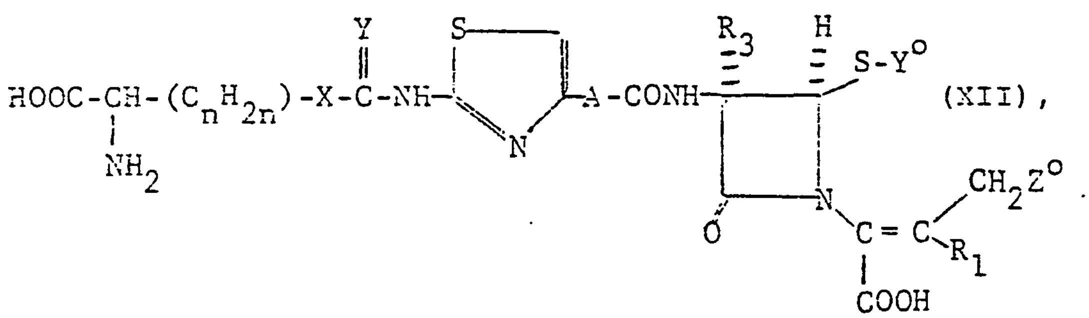

(XII),

worin $R_1$ die obige Bedeutung hat, $R_3$ Wasserstoff ist, $Y^o$ eine Abgangsgruppe darstellt und $Z^o$ Wasserstoff oder Halogen bedeutet, und worin funktionelle Gruppen geschützt sind, unter Abspaltung von $Y^o$ und $Z^o$ ringschliesst oder

h) eine 2-Cephemverbindung der Formel

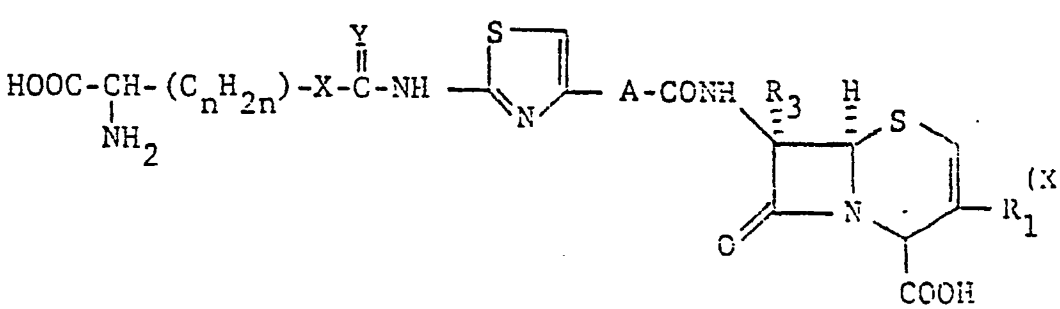

(XIII),

worin funktionelle Gruppen geschützt sind, zur entsprechenden 3-Cephemverbindung der Formel I isomerisiert, und, wenn gewünscht, in einer erhaltenen Verbindung noch

nicht geschützte funktionelle Gruppen schützt oder eine Schutzgruppe gegen eine andere austauscht, und/oder, wenn gewünscht, einen Rest A oder $R_1$ in einen anderen Rest A bzw. $R_1$ überführt, und/oder, wenn gewünscht, eine erhaltene Verbindung, worin $R_3$ Wasserstoff ist, in eine Verbindung überführt, worin $R_3$ Methoxy ist, und/oder, wenn erwünscht, eine erhaltene Verbindung in ein Salz oder ein erhaltenes Salz in eine freie Verbindung oder in ein anderes Salz überführt.

Verfahren a), b) und c) (Acylierungen): Gegebenenfalls vorhandene, die Aminogruppe substituierende und deren Acylierung erlaubende Reste in einem Ausgangsmaterial der Formel II oder IV sind beispielsweise organische Silyl- oder Stannylgruppen, ferner auch Ylidengruppen, die zusammen mit der Aminogruppe eine Schiff'sche Base bilden. Die genannten organischen Silyl- oder Stannylgruppen sind z.B. die gleichen, die auch mit der 4-Carboxylgruppe am Cephemring eine geschützte Carboxylgruppe zu bilden vermögen. Bei der Silylierung oder Stannylierung einer Carboxylgruppe in einem Ausgangsmaterial der Formel II oder IV, kann, bei Verwendung eines Ueberschusses des Silylierungs- oder Stannylierungsmittels, die Aminogruppe ebenfalls silyliert oder stannyliert werden.

Die genannten Ylidengruppen sind in erster Linie Arylmethylengruppen, worin Aryl insbesondere für einen carbocyclischen, in erster Linie monocyclischen Arylrest, z.B. für gegebenenfalls, wie durch Nitro oder Hydroxy, substituiertes Phenyl steht.

Die übrigen in den Ausgangsstoffen der Formeln II bis VII vorhandenen funktionellen Gruppen können durch die bereits unter den Verbindungen der Formel I genannten

Schutzgruppen geschützt sein. Bevorzugt sind alle nicht an der Acylierungsaktion teilnehmenden reaktionsfähigen funktionellen Gruppen, insbesondere aber gegebenenfalls vorhandene, acylierbare Amino-, Hydroxy- und Mercaptogruppen, geschützt.

Den Acylrest einer Carbonsäure der Formel III oder V einführende Acylierungsmittel sind beispielsweise die Carbonsäure selbst oder reaktionsfähige funktionelle Derivate davon.

Falls eine freie Säure der Formel III oder V, worin alle funktionellen Gruppen ausser der reagierenden Carboxylgruppe geschützt sind, zur Acylierung eingesetzt wird, verwendet man üblicherweise geeignete Kondensationsmittel, wie Carbodiimde, beispielsweise N,N'-Diäthyl-, N,N'-Dipropyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazoliniumsalze, beispielsweise N-Aethyl-5-phenyl-isoxazolinium-3'-sulfonat und N-tert.-Butyl-5-methyl-isoxazoliniumperchlorat, oder eine Acylaminoverbindung, z.B. 2-Aethoxy-1-äthoxycarbonyl-1,2-dihydrochinolin.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Ein reaktionsfähiges, d.h. Amid-bildendes, bzw. Esterbildendes, funktionelles Derivat einer Säure der Formel III, V oder VII, worin alle funktionellen Gruppen ausser der reagierenden Säuregruppe geschützt sind bzw.

sein können, ist in erster Linie ein Anhydrid einer solchen Säure, inklusive und vorzugsweise ein gemischtes Anhydrid, aber auch ein inneres Anhydrid, d.h. ein entsprechendes Keten oder, in der Säure V, wenn X die Gruppe -NH- ist, ein entsprechendes Isocyanat bzw. Isothiocyanat. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern , insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure. Von der Säure III, wenn A Hydroxymethylen bedeutet, kann auch ein gemischtes inneres Anhydrid mit dem Kohlensäurehalbester der α-Hydroxygruppe verwendet werden.

Weitere, zur Reaktion mit der Amino-, Hydroxy- oder Mercaptogruppe geeignete Säurederivate in einer Säure der Formel III, V, oder VII, worin alle funktionellen Gruppen ausser der reagierenden Carboxylgruppe geschützt sind, bzw. sein können, sind aktivierte Ester, wie Ester mit vinylogen Alkoholen (d.h. Enolen), wie vinylogen Niederalkenolen, oder Iminiomethylesterhalogeni-

den, wie Dimethyliminiomethylesterchlorid (hergestellt aus der Carbonsäure und Dimethyl-chlormethyliden-imminium-chlorid der Formel $[(CH_3)_2N{=}CHCl]^{\oplus}Cl^{\ominus}$), oder Arylester, wie 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthalyliminoester.

Die Acylierung mit einem Säurederivat, wie einem Anhydrid, insbesondere mit einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril,

oder Mischungen davon, bei Raumtemperatur, wenn notwendig, bei erniedrigter oder erhöhter Temperatur, etwa bei -40° bis etwa 100°, bevorzugt bei -10° bis +40°, und/oder in einer Inertgas-, z.B. Stickstoffatmosphäre.

Die Acylierung kann auch in Gegenwart einer die gewünschte Amidbindung herstellenden Acylase erfolgen. Solche Acylasen sind bekannt und werden durch eine Reihe von Mikroorganismen gebildet, z.B. durch Acetobacter, wie Acetobacter aurantium, Achromobacter, wie Achromobacter aeris, Aeromonas, wie Aeromonas hydrophila, oder Bacillus megaterium, z.B. 400. In die enzymatische Acylierung werden insbesondere Amide, Ester oder Thioester, wie entsprechende Niederalkyl-, z.B. Methyl- oder Aethylester, der Carbonsäure der Formel III eingesetzt. Die enzymatische Acylierung wird in einem den Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden Medium bei etwa 30-40°, bevorzugt bei etwa 37°, durchgeführt.

In einer acylierenden Säure der Formel III, V oder VII oder in einem Säurederivat davon kann eine geschützte Aminogruppe auch in ionischer Form vorliegen, d.h. das Ausgangsmaterial der Formel III, V oder VII kann in Form eines Säureadditionssalzes, vorzugsweise mit einer starken anorganischen Säure, wie einer Halogenwasserstoffsäure, z.B. Salzsäure, oder Schwefelsäure verwendet werden.

Ferner kann ein Säurederivat, wenn erwünscht, in situ gebildet werden. So erhält man z.B. ein gemischtes Anhydrid durch Behandeln einer Säure der Formel III, oder einer Säure der Formel V, worin X die direkte Bindung bedeutet, mit entsprechend geschützten funktionellen Gruppen, oder eines geeigneten Salzes davon, wie eines Ammoniumsalzes, z.B. mit einem organischem Amin, wie 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, oder mit einem Halbester eines Kohlensäurehalbhalogenids, z.B. Chlorameisensäureäthylester oder -isobutylester, und verwendet das so erhältliche gemischte Anhydrid ohne Isolierung.

Ein Säurechlorid einer Säure der Formel V, woring X Sauerstoff, Schwefel oder die Gruppe -NH- ist und worin die Aminocarbonsäuregruppierung HOOC-CH($NH_2$)- in geschützter Form vorliegt, kann, z.B. in situ, gebildet werden, indem man eine Aminocarbonsäure der Formel VI, worin X Sauerstoff, Schwefel oder die Gruppe -NH- bedeutet, und die Gruppierung HOOC-CH($NH_2$)- in geschützter Form vorliegt, in Gegenwart eines Salzsäureakzeptors in einem inerten organischen Lösungsmittel oder Lösungsmittelgemisch mit Phosgen behandelt. Die Salzsäureakzeptoren, Lösungsmittel und Reaktionsbedingungen sind die gleichen, die für die Acylierung von Verbindungen der Formel II oder IV genannt wurden; beispielsweise kann die Reaktion in Gegenwart von Pyridin in Methylenchlorid und Toluol bei etwa 0° bis etwa +10° stattfinden.

Auf die gleiche Weise kann, z.B. in situ, ein Säurechlorid einer Säure der Formel VII, worin die 4-Carboxylgruppe und in den Gruppen -A- und $R_1$ gegebenenfalls vorhandene weitere funktionelle Gruppen geschützt sind, aus einer entsprechend geschützten Verbindung der Formel IV, durch Behandlung mit Chlorsulfonyl-

isocyanat hergestellt werden.

Verfahren d) (Kondensation): In einer Ausgangsverbindung der Formel VIII ist Z Halogen, insbesondere Chlor, aber auch Brom oder Jod. Das Thioharnstoffderivat IX, worin die Aminocarbonsäuregruppierung bevorzugt geschützt ist, kann als solches oder als Salz, insbesondere als Thiolat eines Alkalimetalls, wie Lithium, Natrium oder Kalium, oder auch als Thiolat einer Ammoniumverbindung, in äquivalenter Menge oder im Ueberschuss, eingesetzt werden. Eine Verbindung der Formel VIII, die saure Gruppen enthält, z.B. wenn die 4-Carboxylgruppe ungeschützt ist oder wenn A Sulfomethylen bedeutet, kann ebenfalls als Salz, z.B. als Alkalimetall- oder als Ammoniumsalz, z.B. als Lithium-, Natrium-, Kalium-, Triniederalkyl-, wie Trimethyl- oder Triäthylammoniumsalz, eingesetzt werden.

Die Reaktion wird im allgemeinen in einem Lösungsmittel, wie Wasser oder einem organischen, inerten Lösungsmittel oder einer Mischung davon, durchgeführt. Als organische Lösungsmittel geeignet sind Alkohole, wie Methanol, Aethanol oder Isopropanol, Ketone, wie Aceton, Aether, wie Dioxan oder Tetrahydrofuran, Nitrile, wie Acetonitril, halogenierte Kohlenwasserstoffe, wie Methylenchlorid, Chloroform oder Tetrachlorkohlenstoff, Ester, wie Aethylacetat, oder Amide, wie Dimethylformamid oder Dimethylacetamid, und ähnliche. Die Reaktion kann, falls die freien Verbindungen eingesetzt werden, in Gegenwart einer Base durchgeführt werden. Geeignete Basen sind Alkalimetallhydroxide, wie Natrium- oder Kaliumhydroxid, Alkalimetallcarbonate, wie Natrium- oder Kaliumcarbonat, oder organische tertiäre Stickstoffbasen, wie Triniederalkylamine, z.B. Trimethylamin, Triäthylamin, Aethyl-diisopropylamin, Pyridin und dergleichen.

Die Reaktionstemperatur liegt bei Raumtemperatur, oder auch tiefer oder höher, vorzugsweise zwischen -10 bis +100° C, insbesondere zwischen 0 bis 40° C.

Die Reaktion kann auch stufenweise erfolgen, indem zunächst das ringoffene Zwischenprodukt mit der Teilformel

$$-X-\overset{\overset{\displaystyle Y}{\|}}{C}-NH-C(=NH)-S-CH_2-CO-A-$$

entsteht, das dann in der zweiten Stufe dehydratisiert wird.

Verfahren e) (Ersatz von $R_1^a$ durch Wasserstoff): Die Gruppe $R_1^a$ in einem Ausgangsmaterial der Formel X kann freies Hydroxy sein, steht aber vorzugsweise für verestertes Hydroxy. Eine veresterte Hydroxygruppe $R_1^a$ kann durch eine anorganische oder organische Säure, wie eine starke Mineralsäure, z.B. eine Halogenwasserstoffsäure, wie Chlorwasserstoff-, Bromwasserstoff- oder Jodwasserstoffsäure, oder eine organische Carbon- oder Sulfonsäure, inklusive Ameisensäure, wie eine entsprechende aliphatische, cycloaliphatische, cycloaliphatisch-aliphatische, aromatische, araliphatische, heterocyclische oder heterocyclischaliphatische Säure, ferner durch ein Kohlensäurehalbderivat verestert sein. So stellt $R_1^a$ z.B. Halogen, wie Chlor, Brom oder Jod, Niederalkylsulfonyloxy, z.B. Methylsulfonyloxy oder Aethylsulfonyloxy, Arylsulfonyloxy, z.B. 4-Methylphenylsulfonyloxy, Niederalkanoyloxy, z.B. Acetyloxy oder Propionyloxy, Arylcarbonyloxy, z.B. Benzoyloxy, oder Niederalkoxycarbonyloxy, z.B. Methoxycarbonyloxy oder Aethoxycarbonyloxy, dar.

Eine sekundäre Aminogruppe $R_1^a$ ist eine Gruppe $-N(R_4^a)(R_4^b)$, worin einer der Substituenten $R_4^a$ und $R_4^b$ Wasserstoff und der andere einen aliphatischen oder cycloaliphatischen Kohlenwasserstoffrest bedeutet, der bis zu 18, insbesondere bis zu 12 und bevorzugt bis zu 7 Kohlenstoffatome enthält. Aliphatische Kohlenwasserstoffreste $R_4^a$ oder $R_4^b$ sind beispielsweise gegebenenfalls substituierte, z.B. durch Niederalkoxy, wie Methoxy, Niederalkylthio, wie Methylthio, Cycloalkyl, wie Cyclohexyl, Aryl, wie Phenyl oder Heterocyclyl, wie Thienyl, substituierte Alkyl-, insbesondere Niederalkylgruppen, z.B. Methyl, Aethyl, Propyl, Isopropyl, Butyl, Isobutyl, Pentyl, Hexyl, 2-Aethoxyäthyl, 2-Methylthioäthyl, Cyclohexylmethyl, Benzyl oder Thienylmethyl. Cycloaliphatische Kohlenwasserstoffreste $R_4^a$ oder $R_4^b$ sind beispielsweise gegebenenfalls substituierte, z.B. durch Niederalkyl, wie Methyl, Niederalkoxy, wie Methoxy, Niederalkylthio, wie Methylthio, Cycloalkyl, wie Cyclohexyl, Aryl, wie Phenyl oder Heterocyclyl, wie Furyl, substituierte Cycloalkylgruppen, wie gegebenenfalls wie angegeben substituiertes Cyclopropyl, Cyclopentyl, Cyclohexyl oder Cycloheptyl.

Eine tertiäre Aminogruppe $R_1^a$ ist eine Gruppe $N(R_4^a)(R_4^b)$, worin jeder der Substituenten $R_4^a$ und $R_4^b$ einen der angegebenen aliphatischen oder cycloaliphatischen Kohlenwasserstoffreste bedeutet, wobei $R_4^a$ und $R_4^b$ gleich oder verschieden sein können, und wobei beide Substituenten $R_4^a$ und $R_4^b$ durch eine Kohlenstoff-Kohlenstoff-Bindung oder über ein Sauerstoff-, Schwefel- oder gegebenenfalls substituiertes, wie niederalkyliertes, z.B. methyliertes Stickstoffatom miteinander verbunden sein können.

Geeignete tertiäre Aminogruppen $N(R_4^a)(R_4^b)$ sind beispielsweise Dimethylamino, Diäthylamino, N-Methyl-äthylamino, Di-isopropylamino, N-Methyl-isopropylamino, Dibutylamino, N-Methyl-isobutylamino, Dicyclopropylamino, N-Methylcyclopropylamino, Dicyclopentylamino, N-Methyl-cyclopentylamino, Dicyclohexylamino, N-Methyl-cyclohexylamino, N-Methyl-

cyclopentylamino, N-Methyl-cyclohexylmethylamino, Dibenzylamino, N-Methylbenzylamino, N-Cyclopropyl-benzylamino, 1-Aziridinyl, 1-Pyrrolidinyl, 1-Piperidyl, 1H-2,3,4,5,6,7-Hexahydroazepinyl, 4-Morpholinyl, 4-Thiomorpholinyl, 1-Piperazinyl oder 4-Methyl-1-piperazinyl.

Der Ersatz der Gruppe $R_1^a$ in einer Verbindung der Formel X durch Wasserstoff erfolgt entweder durch Reduktion der $C_3$-$C_4$-Doppelbindung und Abspaltung von $R_1^a$-H oder durch unmittelbare Abhydrierung von $R_1^a$.

Die Reduktion von Ausgangsstoffen der Formel X kann unter Anwendung von geeigneten Reduktionsmitteln vorgenommen werden. So kann man katalytisch aktivierten Wasserstoff verwenden, wobei man den Wasserstoff in Gegenwart eines Hydrierkatalysators, wie eines Palladium-, Nickel-, Rhodium-, Ruthenium- oder Platinkatalysators anwendet, und z.B. Wasserstoff zusammen mit Platin oder Platinoxyd in Gegenwart eines Lösungsmittels, wie eines Alkohols, wie eines Niederalkanols, z.B. Methanol oder Aethanol, oder Essigsäure und gegebenenfalls unter Druck verwendet.

Vorzugsweise reduziert man mit Hilfe von metallischen Reduktionsmitteln ("naszierender Wasserstoff"), in erster Linie aber mit Hydrid-Reduktionsmitteln. Metallische Reduktionsmittel sind z.B. reduzierende Metalle, wie Natrium, Kalium, Calcium, Magnesium, Aluminium oder insbesondere Zink, oder reduzierende Metallverbindungen, z.B. -legierungen, -amalgame oder -salze davon, wie Aluminiumamalgam, die man üblicherweise in Gegenwart von Wasserstoff-abgebenden Mitteln, wie Wasser, Alkohol (z.B. Methanol oder Aethanol) oder einer Säure, z.B. Essigsäure anwendet. Zink wendet man beispielsweise in Gegenwart von Eisessig, ein Amalgam z.B. in Gegenwart eines wasserhaltigen inerten organischen Lösungsmittels, wie eines Aethers, an. Hydrid-Reduktionsmittel sind in erster Linie komplexe Metallhydride, vorzugsweise entsprechende Borhydride, wie Diboran oder Alkalimetallborhydride, z.B. Natriumborhydrid oder Lithiumborhydrid, ferner Zinkborhydrid, sowie organische Alkalimetallaluminiumhydride, wie Triniederalkoxy-alkalimetallaluminiumhydride, z.B. Tri-tert.-butyloxy-lithiumaluminiumhydrid, die man üblicherweise in Gegenwart von Lösungsmitteln, insbesondere von relativ polaren Lösungsmitteln, wie Alkoholen, z.B. Niederalkanolen, wie Methanol oder Aethanol, oder Aethern, wie aliphatischen Aethern, z.B. Glycol- und Polyglycoläthern, wie Aethylenglycoldimethyläther oder Diäthylenglycoldimethyläther, oder cyclischen Aethern, wie Tetrahydrofuran oder Dioxan, oder Lösungsmittelgemischen, auch von wässrigen Lösungsmitteln, anwendet, wobei man bei Temperaturen von etwa -20° C bis etwa +80° C, wenn notwendig, in einem geschlossenen Gefäss und/oder in einer Inertgas-, z.B. Stickstoffatmosphäre arbeitet.

Falls die Reduktion in Gegenwart geeigneter Wasser-, Säure oder Amin-abspaltender Mittel durchgeführt wird, kann die gewünschte 3-unsubstituierte-3- Cephem-Verbindung erhalten werden. Es kann aber auch zunächst eine 3-$R_1^a$-Cepham-Verbindung entstehen, die entweder isoliert oder in situ weiterverarbeitet werden kann.

Die Abspaltung der Elemente einer Verbindung der Formel $R_1^a$-H, d.h. von Wasser, einer Säure oder eines Amins, aus einer erhaltenen 3-$R_1^a$-Cepham-Verbindung, wird vorzugsweise durch Behandeln mit geeigneten Wasser-, Säure- oder Amin-abspaltenden Mitteln durchgeführt. Wasser und Amine werden vorzugsweise in Gegenwart eines sauren Mittels, z.B. einer Säure, vorzugsweise einer starken organischen Carbon- oder Sulfonsäure, wie einer Halogen-niederalkancarbonsäure, z.B. p-Toluolsulfonsäure, abgespalten. Wasser wird auch in Gegenwart eines geeigneten Säurederivats, wie eines Anhydrids oder insbesondere eines Halogenids, wie Chlorids, in erster Linie einer anorganischen, z.B. Phosphor- oder Schwefelhaltigen, Säure, z.B. Phosphoroxychlorid oder Thionylchlorid, wobei man ein solches Derivat, üblicherweise in

Gegenwart einer Base, wie einer tertiären organischen Base, z.B. Pyridin, verwendet, oder eines geeigneten sauren Ionenaustauschers, wie eines Ionenaustauschers auf der Sulfonsäure-Basis, z.B. eines sulfonierten Polystyrolionenaustauschers, abgespalten. Ferner kann man zur Wasserabspaltung auch dehydratisierende Carbodiimidverbindungen, z.B. Dicyclohexylcarbodiimid, oder dehydratisierende, über Stickstoffatome disubstituierte Carbonylverbindungen, z.B. Carbodiimidazol, verwenden. Dabei verwendet man diese Mittel üblicherweise in Gegenwart eines Lösungsmittels, wie eines gegebenenfalls halogenierten aliphatischen, cycloaliphatischen oder aromatischen Kohlenwasserstoffs, z.B. Benzol oder Toluol, oder eines Lösungsmittelgemisches, wobei man geeignete saure Mittel, wie Trifluoressigsäure gleichzeitg auch als Lösungsmittel verwenden kann. Wenn notwendig, verwendet man zusätzlich ein wasser-adsorbierendes Mittel oder einen Wasserabscheider und arbeitet unter Kühlen oder Erwärmen und/oder in einer Inertgas-, z.B. Stickstoffatmosphäre.

Vorzugsweise bedeutet $R_1^a$ in einem Ausgangsmaterial der Formel X eine veresterte Hydroxygruppe und man spaltet nach der Reduktion oder gleichzeitig eine Säure der Formel $R_1^a$-H ab. Ueblicherweise verwendet man zu diesem Zweck basische Säure-abspaltende und/oder -neutralisierende Mittel, wie z.B. anorganische Basen, wie verdünnte Alkalimetallhydroxyde, z.B. Natrium- oder Kaliumhydroxyd, wobei man neben Wasser auch organische Lösungsmittel, wie geeignete Ketone, z.B. Aceton, oder Aether, z.B. Dioxan, oder wässrige Gemische davon verwenden und bei einem pH-Wert von höchstens etwa 9, wenn notwendig, unter Kühlen oder Erwärmen und/oder in einer Inertgas-, z.B. Stickstoffatmosphäre arbeiten kann. Vorzugsweise verwendet man als Säure-abspaltende Mittel tert.-Amine, insbesondere gute Protonenakzeptoren, die den Lactam-

ring nicht angreifen, in erster Linie tert.-aliphatische oder tert.-cycloaliphatische Mono- und Diamine, wie Triniederalkylamine, z.B. Trimethylamin, Triäthylamin oder Aethyl-diisopropylamin, oder bicyclische Diazaverbindungen mit einer Amidin-artigen Anordnung der Ringstickstoffatome, z.B. 1,5-Diazabicyclo[4,3,0]non-4-en oder 1,5-Diazabicyclo[5.4.0]undec-5-en. Ferner kann man basische Ionenaustauscher, z.B. auf der Ammonium-hydroxid-Basis, als Säure-abspaltende Mittel einsetzen. Gewisse veresterte Hydroxygruppen $R_1^a$, insbesondere Sulfonyloxy, z.B. Methylsulfonyloxygruppen lassen sich in Form einer Säure der Formel $R_1^a$-H auch durch Adsorption, z.B. an Silikagel. Aluminiumoxyd, etc., und Elution (Chromatographie), abspalten.

Die Säureabspaltungen werden in Abwesenheit, üblicherweise aber in Gegenwart eines Lösungsmittels, wie eines gegebenenfalls halogenierten Kohlenwasserstoffs aliphatischen, cycloaliphatischen oder aromatischen Charakters, wie Methylenchlorid, eines Niederalkanons, z.B. Aceton, oder Aethers, z.B. Tetrahydrofuran oder Dioxan, oder eines Lösungsmittelgemisches, inkl. eines wässrigen Gemisches, wenn notwendig unter Kühlen oder Erwärmen und/oder in einer Inertgas-, z.B. Stickstoffatmosphäre, vorgenommen.

Bevorzugt wird in einer Verbindung der Formel X eine veresterte Hydroxygruppe $R_1^a$, wie eine 3-Halogen-, wie 3-Chlor- oder 3-Brom-, eine 3- Toluolsulfonyloxy- oder eine 3-Methansulfonyloxygruppe durch Behandlung mit Zink in Eisessig, und eine tertiäre Aminogruppe $R_1^a$, wie 3-Morpholino, 3-Pyrrolidino oder 3-N-Methylcyclohexylamino, durch Behandlung mit Diboran und anschliessend mit Eisessig, durch Wasserstoff ersetzt.

Verfahren f) (Ringschluss nach Wittig):

Im Ausgangsmaterial der Formel XI bedeutet jede der Gruppen $R_a$, $R_b$ und $R_c$ in erster Linie einen gegebenenfalls durch funktionelle Gruppen, z.B. durch gegebenenfalls verätherte oder veresterte Hydroxygruppen, wie Niederalkoxygruppen und/oder Halogenatome, substituierten Niederalkylrest oder einen gegebenenfalls , z.B. durch aliphatische Kohlenwasserstoffreste, wie Niederalkylgruppen, und/oder durch funktionelle Gruppen, wie gegebenenfalls verätherte oder veresterte Hydroxygruppen, wie Niederalkoxygruppen oder Halogenatome, oder Nitrogruppen, substituierten Phenylrest. Bevorzugt sind $R_a$, $R_b$ und $R_c$ Phenylgruppen.

Der Ringschluss erfolgt in einem inerten organischen Lösungsmittel, bei Temperaturen zwischen etwa -20° C und etwa 100° C, üblicherweise unmittelbar nach der oxidativen Bildung der endständigen Oxogruppe aus einer entsprechenden endständigen Hydroxygruppe.

Verfahren g) (Ringschluss unter Abspaltung von $Y^o$ und $Z^o$):

In einer Ausgangsverbindung der Formel XII ist $Z^o$ Wasserstoff oder Halogen, wie Chlor, Brom oder Jod, und $Y^o$ ist beispielsweise eine Gruppe $-S-R_4$, eine mit dem Schwefelatom an die Thiogruppe -S- gebundene Gruppe $-SO_2-R_5$ oder auch eine Gruppe $-S-SO_2-R_5$.

In der Gruppe $-S-R_4$ ist $R_4$ ein gegebenenfalls substituierter aromatischer heterocyclischer Rest mit bis zu 15, bevorzugt bis zu 9 Kohlenstoffatomen, und mindestens einem Ringstickstoffatom und gegebenenfalls einem weiteren Ringheteroatom, wie Sauerstoff oder Schwefel, welcher Rest mit einem seiner Ringkohlenstoffatome, das mit einem Ringstickstoffatom durch eine Doppelbindung verbunden ist, an die Thiogruppe -S- gebunden ist, oder ein Acylrest einer organischen Carbon- oder Thiocarbonsäure, wie einer gegebenenfalls substituierten, aliphatischen, cycloaliphatischen, araliphatischen oder aromatischen, Carbon- oder Thiocarbonsäure mit bis zu 18, vorzugsweise bis zu 10, Kohlenstoff-

atomen.

Solche Reste $R_4$ sind z.B. monocyclische fünfgliedrige thiatriazacyclische, oxadiazacyclische oder oxatriazacyclische Reste aromatischen Charakters, insbesondere aber monocyclische fünfgliedrige diazacyclische, oxazacyclische und thiazacyclische Reste aromatischen Charakters, und oder in erster Linie die entsprechenden benzdiazacyclischen, benzoxazacyclischen oder benzthiazacyclischen Reste, worin der heterocyclische Teil fünfgliedrig ist und aromatischen Charakter aufweist, wobei in Resten $R_4$ ein substituierbares Ringstickstoffatom z.B. durch Niederalkyl substituiert sein kann. Repräsentativ für solche Gruppen $R_4$ sind 1-Methyl-imidazol-2-yl, 1,3-Thiazol-2-yl, 1,3,4-Thiadiazol-2-yl, 1,3,4,5-Thiatriazol-2-yl, 1,3-Oxazol-2-yl, 1,3,4-Oxadiazol-2-yl, 1,3,4,5-Oxatriazol-2-yl, 2-Chinolyl, 1-Methyl-benzimidazol-2-yl, Benzoazol-2-yl und insbesondere Benthiazol-2-yl.

Representative Acyl- oder Thioacylreste $R_4$ sind beispielsweise Niederalkanoyl, inklusive Formyl, Acetyl und Propionyl, Niederthioalkanoyl, z.B. Thioacetyl oder Thiopropionyl, Cycloalkancarbonyl, z.B. Cyclohexancarbonyl, Cycloalkanthiocarbonyl, z.B. Cyclohexanthiocarbonyl, Benzoyl, Thiobenzoyl, Naphthylcarbonyl, Naphthylthiocarbonyl, heterocyclisches Carbonyl oder Thiocarbonyl, wie 2-, 3- oder 4-Pyridylcarbonyl, 2- oder 3-Thenoyl, 2- oder 3-Furoyl, 2-, 3- oder 4-Pyridylthiocarbonyl, 2- oder 3-Thiothenoyl, 2- oder 3-Thiofuroyl, oder entsprechende substituierte beispielsweise durch Niederalkyl, wie Methyl, Halogen, wie Fluor oder Chlor, Niederalkoxy, wie Methoxy, Aryl, wie Phenyl, Aryloxy, wie Phenyloxy, mono- oder polysubstituierte Acyl- oder Thioacylgruppen.

In den Gruppen $-SO_2-R_5$ und $-S-SO_2-R_5$ ist $R_5$ ein gegebenenfalls substituierter, insbesondere aliphatischer, cycloaliphatischer, araliphatischer oder aromatische, Kohlenwasserstoffrest mit bis zu 18, vorzugsweise bis zu 10, Kohlenstoffatomen. Geeignete Gruppen $R_5$ sind beispielsweise

gegebenenfalls substituierte, wie durch Niederalkoxy, wie Methoxy, Halogen, wie Fluor, Chlor oder Brom, Aryl, wie Phenyl, Aryloxy, wie Phenyloxy, mono- oder polysubstituierte Alkyl-, insbesondere Niederalkyl-, wie Methyl-, Aethyl oder Butylgruppen, Alkenyl-, wie Allyl- oder Butenylgruppen, Cycloalkyl-, wie Cyclopentyl- oder Cyclohexylgruppen, oder gegebenenfalls durch Niederalkyl, wie Methyl, Niederalkoxy, wie Methoxy, Halogen, wie Fluor, Chlor oder Brom, Aryl, wie Phenyl, Aryloxy, wie Phenyloxy, oder Nitro, mono- oder polysubstituierte Naphthyl- oder insbesondere Phenylgruppen, beispielsweise Phenyl, o-, m- oder bevorzugt p-Tolyl, o-, m- oder bevorzugt p-Methoxyphenyl, o-, m- oder p-Chlorphenyl, p-Biphenylyl, p-Phenoxyphenyl, p-Nitrophenyl oder 1- oder 2-Naphthyl.

In einer Verbindung der Formel XII kann die Gruppe $R_1$ in trans- (Crotonsäurekonfiguration) oder in cis-Stellung (Isocrotonsäurekonfiguration) zur Carboxylgruppe stehen. Der Ringschluss unter Abspaltung von $Y^o$ und $Z^o$ wird durch eine geeignete Base, insbesondere eine starke organische oder anorganische Base bewirkt. Hervorzuheben sind bicyclische Amidine, wie Diazabicycloalkene, z.B. 1,5-Diazabicyclo[4.3.0]non-5-en oder 1,5-Diazabicyclo[5.4.0]undec-5-en, substituierte, z.B. durch Niederalkyl mehrfach substituierte Guanidine, wie Tetramethylguanidin, ferner Metallbasen, wie Hydride, Amide oder Akoholate von Alkalimetallen, insbesondere von Lithium, Natrium oder Kalium, beispielsweise Natriumhydrid, Lithiumdiniederalkylamide, wie Lithiumdiisopropylamid, Kalium-niederalkanolate, wie Kalium-tert.-butylat, sowie tertiäre organische Stickstoffbasen, z.B. Triniederalkylamine, wie Triäthylamin.

Die Reaktion wird in einem geeigneten inerten Lösungsmittel durchgeführt, beispielsweise in einem aliphatischen, cycloaliphatischen oder aromatischen Kohlenwasserstoff, wie Hexan, Cyclohexan, Benzol oder Toluol, einem halogenierten Kohlenwasserstoff, wie Methylenchlorid, einem Aether, wie einem Diniederalkyläther, z.B. Diäthyläther, einem Diniederalkoxyniederalkan, wie Dimethyloxyäthan, einem cyclischen Aether, wie Dioxan oder Tetrahydrofuran oder auch einem Niederalkanol, z.B. Methanol, Aethanol oder tert.-Butanol, oder in einem Gemisch davon, bei Raumtemperatur oder unter leichtem Erwärmen auf 40 bis 50°, gewünschtenfalls in einer Inertgas-, wie Stickstoffatmosphäre.

Verfahren h) (Isomerisierung): In einer 2-Cephem-Verbindung der Formel XIII weist die gegebenenfalls geschützte Carboxylgruppe in 4-Stellung vorzugsweise die α-Konfiguration auf.

2-Cephem-Verbindungen der Formel XIII werden isomerisiert, indem man sie mit einem schwach-basischen Mittel behandelt und die entsprechende 3-Cephem-Verbindung isoliert. Geeignete Isomerisierungsmittel sind z.B. organische, stickstoffhaltige Basen, insbesondere tertiäre heterocyclische Basen aromatischen Charakters, in erster Linie Basen des Pyridin-Typs, wie Pyridin selber, sowie Collidine oder Lutidine, ferner Chinolin, tertiäre aromatische Basen, z.B. solche des Anilin-Typs, wie N,N-Diniederalkylaniline, z.B. N,N-Dimethylanilin oder N,N-Diäthylanilin, oder tertiäre aliphatische, azacycloaliphatische oder araliphatische Basen, wie N,N,N-Triniederalkylamine, z.B. N,N,N-Trimethylamin, N,N-Diisopropyl-N-äthylamin, N-Niederalkyl-azacycloalkane, z.B. N-Methyl-piperidin, oder N-Phenyl-niederalkyl-N,N-diniederalkyl-amine, z.B. N-Benzyl-N,N-dimethylamin, sowie Gemische davon, wie das Gemisch einer Base vom Pyridintyp und eines N,N,N-Tri-niederalkylamins, z.B. Pyridin und Triäthylamin. Ferner können auch anorganische oder organische Salze von Basen, insbesondere von mittelstarken bis starken Basen mit schwachen Säuren, wie Alkalimetall-, oder Ammoniumsalze von Niederalkancarbonsäuren, z.B. Natriumacetat, Triäthylammoniumacetat oder N-Methyl-piperidinacetat, sowie andere analoge Basen oder Gemische von solchen basischen Mitteln verwendet werden.

Bei Isomerisierung mit basischen Mitteln arbeitet man vorzugsweise in wasserfreiem Medium, in An- oder Abwesenheit eines Lösungsmittels, wie eines gegebenenfalls halogenierten, z.B. chlorierten, aliphatischen, cycloaliphatischen oder aromatischen Kohlenwasserstoffs, oder eines Lösungsmittelgemisches, wobei als Reaktionsmittel verwendete, unter den Reaktionsbedingungen flüssige Basen gleichzeitig auch als Lösungsmittel dienen können, unter Kühlen, bei Zimmertemperatur oder unter Erhitzen, vorzugsweise in einem Temperaturbereich von etwa -30°C bis etwa +100°C, in einer Inertgas-, z.B. Stickstoffatmosphäre, und/oder in einem geschlossenen Gefäss.

Die so erhältlichen 3-Cephem-verbindungen lassen sich in an sich bekannter Weise, z.B. durch Adsorption und/oder Kristallisation, von gegebenenfalls noch vorhandenen 2-Cephem-verbindungen abtrennen.

Die Isomerisierung von 2-Cephem-verbindungen der Formel XIII wird bevorzugt durchgeführt, indem man diese in 1-Stellung oxidiert, wenn erwünscht, ein erhältliches Isomerengemisch der 1-Oxide trennt, und die so erhältliches 1-Oxide der entsprechenden 3-Cephem-verbindungen reduziert.

Als geeignete Oxidationsmittel für die Oxidation in 1-Stellung von 2-Cephem-verbindungen kommen anorganische Persäuren, die ein Reduktionspotential von wenigstens +1,5 Volt aufweisen und aus nicht-metallischen Elementen bestehen, organische Persäuren oder Gemische aus Wasserstoffperoxid und Säuren, insbesondere organische

Carbonsäuren, mit einer Dissoziationskonstante von wenigstens $10^{-5}$ in Frage. Geeignete anorganische Persäuren sind Perjod- und Perschwefelsäure. Organische Persäuren sind entsprechende Percarbon- und Persulfonsäuren, die als solche zugesetzt oder durch Verwendung von wenigstens einem Aequivalent Wasserstoffperoxyd und einer Carbonsäure in situ gebildet werden können. Dabei ist es zweckmässig, einen grossen Ueberschuss der Carbonsäure zu verwenden, wenn z.B. Essigsäure als Lösungsmittel verwendet wird. Geeignete Persäuren sind z.B. Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

Die Oxidation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1-2% und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxid mit Essigsäure, Perchlorsäure oder Trifluoressigsäure.

Die obige Oxidation kann in Gegenwart von geeigneten Katalysatoren durchgeführt werden. So kann z.B. die Oxidation mit Percarbonsäuren durch die Anwesenheit einer Säure mit einer Dissoziationskonstante von wenigstens $10^{-5}$ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxidationsmittels, vorzugsweise einen geringen Ueberschuss von etwa 10% bis etwa 20%, wobei man auch grössere Ueberschüsse, d.h. bis zur 10-fachen Menge des

Oxidationsmittels oder darüber, verwenden kann. Die Oxidation wird unter milden Bedingungen, z.B. bei Temperaturen von etwa -50°C bis etwa +100°C, vorzugsweise von etwa -10°C bis etwa +40°C durchgeführt.

Die Reduktion der 1-Oxide von 3-Cephem-Verbindungen kann in an sich bekannter Weise durch Behandeln mit einem Reduktionsmittel, wenn notwendig, in Anwesenheit eines aktivierenden Mittels, durchgeführt werden. Als Reduktionsmittel kommen beispielsweise in Betracht: Katalytisch aktivierter Wasserstoff, wobei Edelmetallkatalysatoren verwendet werden, welche Palladium, Platin oder Rhodium enthalten, und die man gegebenenfalls zusammen mit einem geeigneten Trägermaterial, wie Kohle oder Bariumsulfat, einsetzt; reduzierende Zinn-, Eisen-, Kupfer- oder Mangankationen, welche in Form von entsprechenden Verbindungen oder Komplexen anorganischer oder organischer Art, z.B. als Zinn-II-chlorid, -fluorid, -acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanid-anionen, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden Säuren, wie Jodwasserstoffsäure, verwendet werden; reduzierende trivalente anorganische oder organische Phosphorverbindungen, wie Phosphine, ferner Ester, Amide und Halogenide der phosphinigen, phosphonigen oder phosphorigen Säure, sowie

diesen Phosphorsauerstoffverbindungen entsprechenden Phosphor-Schwefelverbindungen, worin organische Reste in erster Linie aliphatische, aromatische oder araliphatische Reste, z.B. gegebenenfalls substituierte Niederalkyl-, Phenyl oder Phenylniederalkylgruppen darstellen, wie z.B. Triphenylphosphin, Tri-n-butylphosphin,Diphenylphosphinigsäuremethylester, Diphenylchlorphosphin,Phenyldichlorphosphin, Benzolphosphonigsäuredimethylester, Butanphosphonigsäuremethylester, Phosphorigsäuretriphenylester, Phosphorigsäuretrimethylester, Phosphortrichlorid, Phosphortribromid, etc.; reduzierende Halogensilanverbindungen, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenenfalls substituierte Niederalkyl- oder Phenylgruppen aufweisen können, wie Chlorsilan, Bromsilan, Di- oder Trichlorsilan, Di- oder Tribromsilan, Diphenylchlorsilan, Dimethylchlorsilan, Trimethyljodsilan oder auch Halogensilanverbindungen, worin alle Wasserstoffe durch organische Reste ersetzt sind, wie Triniederalkylhalogensilan, z.B. Trimethyljodsilan, oder cyclische, schwefelhaltige Silane, wie 1,3-Dithia-2,4-disilacyclobutane oder 1,3,5-Trithia-2,4,6-trisila-cyclohexane, deren Siliciumatome durch Kohlenwasserstoffreste, wie insbesondere Niederalkylreste substituiert sind, z.B. 2,2,4,4-Tetramethyl-1,3-dithia-2,4-disilacyclobutan oder 2,2,4,4,6,6-Hexamethyl-1,3,5-trithia-2,4,6-trisilacyclohexan, etc.; reduzierende quaternäre Chlormethylen-iminiumsalze, insbesondere -chloride oder -bromide, worin die Iminiumgruppe durch einen bivalenten oder zwei monovalente organische Reste, wie gegebenenfalls substituierte Niederalkylen- oder Niederalkylgruppen substituiert ist, wie N-Chlormethylen-N,N-diäthyliminiumchlorid oder N-Chlor-methylen-pyrrolidiniumchlorid;und komplexe Metallyhdride, wie Natriumborhydrid, in Gegenwart von geeigneten Aktivierungsmitteln, wie Cobalt-II-chlorid, sowie Borandichlorid.

Als aktivierende Mittel, die zusammen mit denjenigen der obgenannten Reduktionsmittel verwendet werden, welche selber nicht Lewissäure-Eigenschaften aufweisen, d.h. die in erster Linie zusammen mit den Dithionit-, Jod- oder Eisen-II-cyanid- und den nicht-halogenhaltigen trivalenten Phosphor-Reduktionsmitteln oder bei der katalytischen Reduktion eingesetzt werden, sind inbesondere organische Carbon- und Sulfonsäurehalogenide, ferner Schwefel-, Phosphor- oder Siliciumhalogenide mit gleicher oder grösserer Hydrolysenkonstante zweiter Ordnung als Benzoylchlorid, z.B. Phosgen, Oxalylchlorid, Essigsäurechlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsäuredichlorid, Dimethylchlorsilan oder Trichlorsilan, ferner geeignete Säureanhydride, wie Trifluoressigsäureanhydrid, oder cyclische Sultone, wie Aethansulton, 1,3-Propansulton, 1,4-Butansulton oder 1,3-Hexansulton zu erwähnen.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels

bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B.Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20° C bis etwa 100° C, wobei bei Verwendung von sehr reaktionsfähigen Aktivierungsmitteln die Reaktion bei tieferen Temperaturen durchgeführt werden kann.

Nachoperationen:

In einem erhaltenen Zwischenprodukt der Formel I, worin mindestens eine der funktionellen Gruppen in geschützter Form vorliegt, können auf übliche, an sich bekannte Weise, noch nicht geschützte funktionelle Gruppen geschützt oder vorhandene Schutzgruppen gegen andere Schutzgruppen ausgetauscht werden, z.B. durch Abspalten der vorhandenen Schutzgruppe und Einführen der gewünschten anderen Schutzgruppe.

1. Veresterung der 4-Carboxylgruppe: Die Ueberführung einer freien Carboxylgruppe in einer erhaltenen Verbindung der Formel I in eine veresterte Carboxylgruppe,

die unter physiologischen Bedingungen spaltbar ist, erfolgt nach an sich bekannten Veresterungsmethoden, beispielsweise indem man eine Verbindung der Formel I , worin andere funktionelle Gruppen, wie Amino-, Hydroxy- oder Sulfogruppen, gegebenenfalls in geschützter Form vorliegen, oder ein bezüglich der zu veresternden Carboxylgruppe reaktionsfähiges funktionelles Derivat davon, oder ein Salz davon, mit einem entsprechenden Alkohol oder einem reaktionsfähigen funktionellen Derivat davon, verestert.

2. <u>Oxidation der Hydroxymethylengruppe A:</u> In einer erhaltenen Verbindung der Formel I, worin funktionelle Gruppen geschützt sind, kann auf an sich bekannte, d.h. wie für die Oxidation von Hydroxy- zu Oxogruppen bekannte Weise durchgeführt werden. Als Oxidationsmittel kommen oxidierende Oxide, wie solche des Mangans, Chroms, Stickstoffs oder Schwefels, wie Mangandioxid, Chromtrioxid, z.B. Jones Reagens oder Chromtrioxid in Gegenwart von Essigsäure, Schwefelsäure oder Pyridin, Distickstofftetroxid, Dimethylsulfoxid gegebenenfalls in Gegenwart von Dicyclohexylcarbodiimid oder Sauerstoff, und Peroxide, wie Wasserstoffperoxid, sauerstoffhaltige Säuren, wie Permangansäure, Chromsäure oder Hypochlorsäure oder Salze davon, wie Kaliumpermanganat, Natrium- oder Kaliumdichromat oder Kaliumhypochlorit, in Frage. Die Hydroxymethylengruppe kann auch durch die Oppenauer-Oxidation in die Oxomethylengruppe übergeführt werden, d.h. durch Behandeln mit dem Salz eines sterisch gehinderten Alkohols, wie Aluminium- oder Kalium-tert.-butoxid, -isopropoxid oder -phenoxid in Gegenwart eines Ketons, wie Aceton, Cyclohexanon oder Fluorenon. Eine weitere Möglichkeit die Hydroxymethylengruppe in die Oxomethylengruppe überzuführen besteht in der Dehydrierung, z.B. mit Raney-Nickel.

Die Oxidation wird je nach Oxidationsmittel in Wasser oder einem gegebenenfalls wasserhaltigen Lösungsmittel bei Temperaturen von etwa 0° bis etwa 100° durchgeführt.

3. Umsatz der Oxomethylengruppe A mit Hydroxylaminen: In einer erhaltenen Verbindung der Formel I, worin funktionelle Gruppen geschützt sind, und worin A eine Oxomethylengruppe bedeutet, kann diese durch Behandeln mit Hydroxylamin oder einem substituierten Hydroxylamin der Formel $R^o-O-NH_2$ in eine Hydroxyiminomethylen- bzw. $R^o$-substituierte Hydroxyiminomethylengruppe übergeführt werden.

Die Reaktion der Oxomethylengruppe mit der Hydroxylaminverbindung wird auf übliche Weise ausgeführt, z.B. indem man die beiden Reaktionspartner in einem Lösungsmittel, wie Wasser oder einem organischen Lösungsmittel, wie einem Alkohol, z.B. Methanol, bei leicht erhöhter oder erniedrigter Temperatur, gegebenenfalls in einer Inertgas-, wie Stickstoffatmosphäre, reagieren lässt. Die Hydroxylaminverbindung kann, auch in situ, aus einem ihrer Salze, beispielsweise einem Hydrohalogenid, wie Hydrochlorid, durch Behandeln mit einer anorganischen Base, wie einem Alkalimetallhydroxid, z.B. Natriumhydroxid, oder einer organischen Base, wie einem tertiären Amin, z.B. einem Triniederalkylamin, wie Triäthylamin oder Aethyl-diisopropylamin, oder einer heterocyclischen tertiären Base, wie Pyridin, in Freiheit gesetzt werden.

4. Nitrosierung und Sulfonylierung der Methylengruppe A: In einer erhaltenen Verbindung der Formel I, worin funktionelle Gruppen geschützt sind und worin A Methylen bedeutet, kann die Methylengruppe durch Nitrosierung, z.B. durch Behandeln mit einem Nitrit, wie Amylnitrit, in Gegenwart

einer Base, z.B. analog DE-PS 855 551, in eine Hydroxyiminomethylengruppe übergeführt werden, die anschliessend, falls erwünscht, durch Alkylierung in eine Gruppe $R^o$-O-N=C=, z.B. mit Dimethylsulfat in Gegenwart einer Base, in eine Methoxyiminomethylengruppe, übergeführt werden kann. Ferner kann eine Methylengruppe A zu einer Sulfomethylengruppe sulfonyliert werden, z.B. durch Behandeln mit dem $SO_3$/Dioxan Komplex, z.B. analog der DT-OS 2 638 028.

5. Veresterung der 3-Hydroxygruppe: In einer erhaltenen Verbindung der Formel I, worin funktionelle Gruppen geschützt sind, und worin $R_1$ eine geschützte Hydroxygruppe ist, kann diese Hydroxyschutzgruppe abgespalten und durch Wasserstoff ersetzt werden. Die erhaltene freie Hydroxygruppe kann nach konventionellen Methoden in eine veresterte oder verätherte Hydroxygruppe $R_1$ übergeführt werden.

Die Veresterung der freien Hydroxygruppe $R_1$ wird mit einem den gewünschten Acylrest einführenden Acylierungsmittel durchgeführt. Dabei verwendet man die entsprechende organische Carbonsäure oder ein reaktionsfähiges Säurederivat davon, wie ein Halogenid, z.B. Fluorid oder Chlorid, ferner ein Pseudohalogenid, wie eine der Carbonsäure entsprechende Cyancarbonylverbindung, oder ein Anhydrid (worunter auch ein inneres Anhydrid einer Carbonsäure, d.h. Keten, oder der Carbamin- oder Thiocarbaminsäure, d.h. ein Isocyanat, oder ein gemischtes Anhydrid, wie ein solches, das sich z.B. mit einem Halogenameisensäure-niederalkyl-, wie Chlorameisensäureäthylester oder -isobutylester, oder mit Trichloressigsäurechlorid bilden lässt, zu verstehen ist), oder einen aktivierten Ester, wobei man, wenn notwendig, in Gegenwart von geeigneten Kondensationsmitteln, bei Verwendung von Säuren z.B. von Carbodiimidverbindungen, wie Dicyclo-

hexylcarbodiimid, oder Carbonylverbindungen, wie Diimidazolylcarbonyl, bei Verwendung von reaktionsfähigen Säurederivaten z.B. von basischen Mitteln, wie Triniederalkylaminen, z.B. Triäthylamin, oder heterocyclischen Basen, z.B. Pyridin, arbeitet. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatmosphäre durchgeführt werden. Geeignete Lösungsmittel sind z.B. gegebenenfalls substituierte, insbesondere gegebenenfalls chlorierte, aliphatische, cycloaliphatische oder aromatische Kohlenwasserstoffe, wie Benzol oder Toluol, wobei man geeignete Veresterungsreagentien, wie Essigsäureanhydrid, auch als Verdünnungsmittel verwenden kann.

Durch organische Sulfonsäuren, z.B. Niederalkansulfonsäuren, wie Methansulfonsäure, oder aromatische Sulfonsäuren, wie p-Toluolsulfonsäure, veresterte Hydroxygruppen kann man vorzugsweise durch Behandeln mit einem reaktionsfähigen Sulfonsäurederivat, wie einem entsprechenden Halogenid, z.B. Chlorid, wenn notwendig, in Gegenwart eines Säure-neutralisierenden basischen Mittels, z.B. einer anorganischen oder organischen Base, z.B. in analoger Weise wie die Ester mit Carbonsäuren, bilden.

6. <u>Verätherung der 3-Hydroxygruppe:</u> Die Verätherung der freien Hydroxygruppe $R_1$ wird mit einem den gewünschten Kohlenwasserstoff einführenden konventionellen Verätherungsmittel durchgeführt.

Geeignete Verätherungsmittel sind beispielsweise entsprechende Diazoverbindungen, wie gegebenenfalls substituierte Diazoniederalkane, z.B. Diazomethan,

Diazoäthan, Diazo-n-butan oder Diphenyldiazomethan. Diese Reagentien werden in Gegenwart eines geeigneten inerten Lösungsmittel, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Aethers, wie eines Diniederalkyläthers, z.B. Diäthyläther, oder eines cyclischen Aethers, z.B. Tetrahydrofuran oder Dioxan, oder eines Lösungsmittelgemisches, und je nach Diazoreagens unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre zur Anwendung gebracht.

Weitere geeignete Verätherungsmittel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure oder Halogen-schwefelsäuren, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls, z.B. durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure, z.B. Diniederalkylsulfate, wie Dimethylsulfat, ferner Fluorsulfonsäureniederalkylester z.B. Fluorsulfonsäuremethylester oder gegebenenfalls Halogen-substituierte Methansulfonsäure-niederalkylester, z.B. Trifluormethansul-

fonsäuremethylester. Sie werden üblicherweise in Gegenwart eines inerten Lösungsmittels, wie eines gegebenenfalls halogenierten, wie chlorierten aliphatischen, cycloaliphatischen oder aromatischen Kohlenwasserstoffs, z.B. Methylenchlorid, eines Aethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organischen Basen, wie, üblicherweise sterische gehinderte, Triniederalkylamine, z.B. N,N-Diisopropyl-N-äthyl-amin (vorzugsweise zusammen mit Niederalkyl-halogensulfaten oder gegebenenfalls Halogensubstituierten Methansulfonsäure-niederalkylestern) an, wobei unter Kühlen, bei Raumtemperatur oder unter Erwärmen, z.B. bei Temperaturen von etwa -20°C bis etwa 50° C und, wenn notwendig, in einem geschlossenen Gefäss und/oder in einer Inertgas-, z.B. Stickstoffatmosphäre gearbeitet wird.

Weitere Verätherungsmittel sind entsprechende Acetale z.B. gem-Niederalkoxyniederalkane, wie 2,2-Dimethoxy-propan, die in Gegenwart einer starken organischen Sulfonsäure, wie p-Toluolsulfonsäure, und eines geeigneten Lösungsmittels, wie eines Diniederalkyl- oder Niederalkylensulfoxyds, z.B. Dimethylsulfoxyd, zur Anwendung gelangen, oder Orthoester, z.B. Orthoameisensäure-triniederalkylester, z.B. Orthoameisensäure-triäthylester, die in Gegenwart einer starken Mineralsäure, z.B. Schwefelsäure, oder einer starken organischen Sulfonsäure, wie p-Toluolsulfonsäure, und eines geeigneten Lösungsmittels, wie eines Aethers, z.B. Dioxan, verwendet werden.

Weitere Verätherungsmittel sind entsprechende Tri-$R_1^o$-oxoniumsalze (sogenannte Meerweinsalze), Di-$R_1^o$-O-

Carbeniumsalze oder Di-$R_1^O$-Haloniumsalze, worin $R_1^O$ den einzuführenden Rest darstellt, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate oder Hexachlorantimonate. Solche Reagentien sind z.B. Trimethyloxonium- oder Triäthyloxoniumhexafluorantimonat, -hexachlorantimonat, -hexafluorphosphat, oder -tetrafluorborat, Dimethoxycarbeniumhexafluorphosphat oder Dimethylbromonium-hexafluorantimonat. Man verwendet diese Verätherungsmittel vorzugsweise in einem inerten Lösungsmittel, wie einem Aether oder einem halogenierten Kohlenwasserstoff, z.B. Diäthyläther, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20° C bis etwa 50° C, wenn notwendig, in einem geschlossenen Gefäss und/oder in einer Inertgas-, z.B. Stickstoffatmosphäre.

Weitere Verätherungsmittel sind schliesslich entsprechende 1-$R_1^O$-3-Aryltriazenverbindungen, worin $R_1^O$ den einzuführenden Rest und Aryl vorzugsweise einen gegebenenfalls substituierten Phenylrest, z.B. Niederalkylphenyl, wie 4-Methylphenyl bedeutet. Solche Triazenverbindungen sind 3-Aryl-1-niederalkyl-triazene, z.B. 3-(4-Methylphenyl)-1-äthyl-triazen, 3-(4-Methylphenyl)-1-n-propyl-triazen oder 3-(4-Methylphenyl)-1-isopropyl-triazen. Diese Reagentien werden üblicherweise in Gegenwart

von inerten Lösungsmitteln, wie gegebenenfalls halogenierten Kohlenwasserstoffen oder Aethern, z.B. Benzol, oder Lösungsmittelgemischen, und unter Kühlen, bei Raumtemperatur und vorzugsweise bei erhöhter Temperatur, z.B. bei etwa 20° C bis etwa 100° C, wenn notwendig, in einem geschlossenen Gefäss und/oder in einer Inertgas-, z.B. Stickstoffatmosphäre verwendet.

7. Halogenierung der 3-Stellung: In einer erhaltenen Verbindung der Formel I, worin $R_1$ eine freie oder sulfonylierte Hydroxygruppe ist und worin andere funktionelle Gruppen geschützt sind, kann diese freie oder sulfonylierte Hydroxygruppe gegen Halogen ausgetauscht werden.

Der Austausch der freien Hydroxygruppe $R_1$ durch Halogen kann in verschiedenartiger Weise, üblicherweise durch Behandeln mit einem halogenierenden, d.h. fluorierenden, chlorierenden oder bromierenden Mittel, durchgeführt werden.

Geeignete Halogenierungsmittel sind beispielsweise Enolhydroxylgruppen durch Halogen ersetzende Phosphor-Reagentien, wie Di-halogen-triorganyl-phosphorane, Trihalogen-diorganyl-phosphorane oder ein Gemisch bestehend aus einem Triorganylphosphin und einem Tetrahalogenmethan.

Repräsentative Vertreter der genannten Phosphorane sind Difluor-triphenyl-, Trifluor-diphenyl-, Dichlor-triphenyl-, Trichlor-diphenyl, Dibrom-triphenyl- und Tribrom-diphenylphosphoran, worin eine der Phenylgruppen durch ein Polymer, wie einem mit Divinylbenzol vernetztem Polystyrol, oder durch Dimethylaminomethyl substituiert sein kann.

Repräsentative Vertreter der genannten Phosphine sind Triäthyl-, Methyl-propyl-phenyl-, Bis-(3-dimethylaminopropyl)-phenyl-, Tris-(dimethylamino)-, Bis-(dimethylamino)-phenyl- und insbesondere Triphenylphosphin, worin eine der Phenylgruppen durch ein Polymer, wie einem mit Divinylbenzol vernetztem Polystyrol, substituiert sein kann.

Tetrahalogenmethane sind z.B. Tetrabrom- und insbesondere Tetrachlorkohlenstoff.

Die Reaktion mit den halogenierenden Phosphorreagentien findet auf an sich bekannte Weise in einem inerten, aprotischen, bevorzugt polaren Lösungsmittel, wie einem chlorierten Kohlenwasserstoff, z.B. Methylenchlorid, Chloroform, Tetrachlorkohlenstoff, 1,2-Dichloräthan, einem Nitril, wie Acetonitril oder Benzonitril, oder einem N,N-disubstituierten Carbonsäureamid, wie Dimethylformamid oder N,N-Dimethylacetamid, oder Mischungen davon, je nach Reaktivität des eingesetzten Reagenses unter Kühlen oder Erwärmen, d.h. bei Temperaturen zwischen etwa -60° C bis zur Rückflusstemperatur des verwendeten Lösungsmittels, gegebenenfalls in einer Inertgas-, wie Stickstoffatmosphäre, statt. Bei Verwendung von Triniederalkyl- oder Tris-(diniederalkylamin)-phosphinen und Tetrachlorkohlenstoff oder Tetrabromkohlenstoff ist gewöhnlich Kühlen notwendig, etwa auf -60° bis -20° C.

Beim Halogenieren mit den genannten Phosphoranen kann dem Reaktionsmedium zum Abfangen entstehenden Halogenwasserstoffes eine schwache Base, wie Pyridin oder ein N,N-Diniederalkylanilin, wie N,N-Dimethylanilin, zugesetzt werden.

Weitere Halogenierungsmittel, sind entsprechende N,N-disubstituierte Halogen-iminiumhalogenid-Verbindungen, insbesondere der Formel $[(CH_3)_2N{=}CH\ Hal]^{\oplus}Hal^{\ominus}$,

worin Hal Chlor bzw. Brom ist.

Das obige Reagens wird üblicherweise in situ hergestellt, indem man ein geeignetes N,N-disubstituiertes Amid, in erster Linie Dimethylformamid, mit einem Chlorierungs- bzw. Bromierungsmittel, wie z.B. Phosgen, Carbonyldibromid, Oxalylchlorid, Oxalylbromid, Thionylchlorid, Thionylbromid, Phosphortrichlorid, Phosphoroxychlorid, Phosphortribromid, Phosphoroxybromid oder Phosphorpentachlorid, insbesondere mit Phosphortrichlorid oder Phosphortribromid, behandelt.

Die obige Reaktion wird üblicherweise in Gegenwart eines Lösungs- oder Verdünnungsmittels durchgeführt, wobei man neben dem normalerweise im Ueberschuss vorliegenden und als Lösungsmittel dienenden Amid, üblicherweise Dimethylformamid, ferner auch Dimethylacetamid, auch ätherartige Lösungsmittel, z.B. Tetrahydrofuran oder Dioxan, Halogenkohlenwasserstoffe, z.B. Methylenchlorid, oder Sulfoxide, z.B. Dimethylsulfoxid, verwenden kann.

Die Chlorierungs- und Bromierungsmittel werden üblicherweise in Mengen verwendet, die zwei Aequivalenten des 3-Hydroxy-3-cephem-Ausgangsmaterials entsprechen. Die Reaktion kann man z.B. so durchführen, dass man das Chlorierungs- bzw. Bromierungsmittel unter Kühlen zu einer Lösung des 3-Hydroxy-3-cephem-Ausgangsmaterials in Dimethylformamid zugibt, worauf man das Reaktionsgemisch während einigen Stunden bei Raumtemperatur stehen lässt.

Die Chlorierung oder Bromierung kann auch so durchgeführt werden, dass man zunächst das Chlorierungs- bzw. Bromierungsmittel mit dem Amid, insbesondere dem Dimethylformamid, mischt und so das Halogeniminiumhalogenid bildet, worauf man die Lösung des 3-Hydroxy-3-

cephem-Ausgangsmaterials im Amid, insbesondere in Dimethylformamid, dem noch ein zusätzliches Lösungsmittel zugegeben werden kann, oder in einem anderen Lösungsmittel, z.B. Tetrahydrofuran, zufügt. Falls notwendig, werden die Reaktionen in einer Inertgasatmosphäre durchgeführt.

Die Umwandlung der freien Hydroxygruppe $R_1$ in Fluor kann z.B. durch Behandeln mit einem Reagens der Formel $F_3S$-Am erfolgen, worin Am eine disubstituierte Aminogruppe darstellt; solche Reagentien sind u.a. von Markovskij et al., Synthesis, Bd. 1973, S. 787 beschrieben worden. Die Gruppe Am steht in erster Linie für Diniederalkylamino, z.B. Dimethylamino, Diäthylamino, Aethylmethyl-amino, Methyl-propylamino, Di-n-propyl-amino oder Diisopropylamino, Niederalkylphenyl-amino, z.B. Methylphenyl-amino oder Aethyl-phenyl-amino, Niederalkylenamino, z.B. Pyrrolidino oder Piperidino, Oxaniederalkylenamino, z.B. Morpholino, oder gegebenenfalls aza-Niederalkyl-substituiertes Amino, z.B. 4-Methyl-piperazino.

Die Reaktion wird vorzugsweise in einem geeigneten inerten Lösungsmittel, wie einem gegebenenfalls substituierten Kohlenwasserstoff, z.B. Cyclopentan, Cyclohexan, Benzol, Toluol, Xylol, Chlorbenzol, Dichlorbenzol, Pentan, Hexan, Chloroform, und vor allem Methylenchlorid oder einem Aether, wie Diäthyläther, Tetrahydrofuran oder vor allem Dioxan, falls notwendig, unter Kühlen oder Erwärmen, z.B. in einem Temperaturbereich von etwa -20° C bis etwa 80° C, vorzugsweise von etwa 0° C bis etwa 30° C, und/oder unter einer Inertgasatmosphäre durchgeführt.

Eine sulfonylierte Hydroxygruppe $R_1$ kann durch Behandeln mit einem anorganischen Fluorid in Gegenwart eines Kronenäthers in Fluor übergeführt werden.

Eine sulfonylierte Hydroxygruppe $R_1$ ist in erster Linie Niederalkylsulfonyloxy, insbesondere Methylsulfonyl-

oxy, kann aber auch Arylsulfonyloxy sein, worin Aryl vorzugsweise gegebenenfalls, z.B. durch Niederalkyl, wie Methyl, Halogen, z.B. Brom, oder Nitro substituiertes Phenyl ist, z.B. 4-Methylphenylsulfonyloxy.

Ein anorganisches Fluorid ist in erster Linie ein Metallfluorid, wobei man insbesondere ein Alkalimetallfluorid, z.B. Natriumfluorid, oder ein Schwermetallfluorid, z.B. Silberfluorid, verwendet.

Die zusammen mit dem anorganischen Fluorid verwendeten Kronenäther sind gegebenenfalls substituierte 18-Kronen-6-äther, wie der Dicyclohexyl-18-kronen-6-äther.

Die Reaktion wird in Gegenwart eines inerten Lösungsmittels, insbesondere eines Nitrils, z.B. Acetonitril oder Propionnitril, oder eines Nitroniederalkans, z.B. Nitromethan oder Nitroäthan, unter im wesentlichen wasserfreien Bedingungen und, falls notwendig, unter Kühlen, z.B. in einem Temperaturbereich von etwa -20° C bis etwa 25° C, vorzugsweise bei etwa Raumtemperatur und gegebenenfalls in einer Inertgasatmosphäre durchgeführt.

8. Thiolisierung der 3-Stellung: In einer erhaltenen Verbindung der Formel I, worin funktionelle Gruppen geschützt sind, kann eine durch ein Thiol substituierbare veresterte oder verätherte Hydroxygruppe $R_1$ durch Behandeln mit einem Thiol in eine verätherte Mercaptogruppe $R_1$ übergeführt werden.

Eine durch ein Thiol substituierbare veresterte Hydroxygruppe $R_1$ ist durch eine der genannten anorganischen oder organischen Säuren verestert, beispielsweise durch eine Halogenwasserstoffsäure, eine Schwefel oder Phosphor enthaltende Säure, wie eine Schwefel- oder

Sulfonsäure, eine Phosphor-; Phosphon- oder Phosphinsäure, oder eine Carbonsäure. Eine durch ein Thiol substituierbare verätherte Hydroxygruppe $R_1$ ist durch einen der genannten Kohlenwasserstoffreste veräthert, beispielsweise durch einen aliphatische, cycloaliphatischen, aromatischen oder araliphatischen Kohlenwasserstoffrest mit z.B. bis zu 15 C-Atomen, wie $C_1$-$C_4$-Niederalkyl, z.B. Methyl oder Aethyl, $C_2$-$C_6$ Niederalkenyl, z.B. Vinyl oder Propenyl, $C_3$-$C_7$-Cycloalkyl, z.B. Cyclopropyl oder Cyclohexyl, Phenyl, oder Phenylniederalkyl, wie Benzyl.

Bevorzugt ist der gegen das Thiol austauschbare Rest $R_1$ Chlor, Brom, Niederalkansulfonyloxy, wie Methan- oder Aethansulfonyloxy, oder Arylsulfonyloxy, wie Benzol-, p-Toluol-, p-Chlorbenzol- oder p-Nitrobenzolsulfonyloxy.

Die Substitution dieser Gruppe $R_1$ durch die verätherte Mercaptogruppe findet in an sich bekannter Weise unter Kühlen, bei Raumtemperatur oder unter Erwärmen, d.h. bei Temperaturen zwischen 0° C bis etwa 40° C, in einem geeigneten inerten Lösungsmittel und in Gegenwart einer Base statt. Geeignete Basen sind insbesondere sterisch gehinderte Amine, wie Triniederalkylamine, z.B. N,N-Diisopropyl-N-äthylamin, bicyclische Amidine, wie Diazabicycloalkane, z.B. 1,5-Diazabicyclo[4.3.0]non-5-en oder 1,5-Diazabicyclo[5.4.0]undec-5-en, sowie Alkalimetallhydride, -amide oder -niederalkanolate, wie Natriumhydrid, Natrium- oder Lithiumamid, Natriumäthylat oder Kalium-tert.-butylat. Die Reaktion kann in einer Inertgas-, wie Stickstoffatmosphäre durchgeführt werden, und, wenn notwendig, in einem geschlossenen Gefäss unter Druck.

Falls $R_1$ im Ausgangsmaterial eine verätherte Hydroxygruppe ist, findet unter den basischen Bedingungen zunächst eine Addition des Thiols an die Doppelbindung statt, worauf der Alkoholrest $H-R_1$ abgespalten werden muss.

Die Abspaltung des Alkohols $H-R_1$ findet in Gegenwart einer Säure, in An- oder Abwesenheit eines geeigneten, inerten Lösungsmittels und unter Kühlen, bei Raumtemperatur oder unter Erwärmen, d.h. bei Temperaturen zwischen etwa -70° C und +100° C, bevorzugt bei etwa 5° C bis etwa 40° C, statt. Zur Abspaltung des Alkohols $H-R_1$ geeignete Säuren sind starke organische Protonensäuren, insbesondere Mineralsäuren, wie Salzsäure, ferner Sulfonsäure, wie Niederalkansulfonsäuren, z.B. Methansulfonsäure, oder aromatische Sulfonsäuren, z.B. Benzol- oder Toluolsulfonsäure, halogenierte Niederalkancarbonsäuren wie Trifluor- oder Trichloressigsäure oder auch Ameisensäure. Geeignete Lösungsmittel sind die bei der basischen Addition genannten.

9. Ersatz der 3-Formylgruppe durch Wasserstoff: In einer erhaltenen Verbindung der Formel I, worin funktionelle Gruppen geschützt sind, und worin $R_1$ eine Formylgruppe darstellt, kann diese Formylgruppe durch Wasserstoff ersetzt werden.

Der Ersatz der Formylgruppe durch Wasserstoff wird durch Decarbonylieren vorgenommen. Diese Reaktion wird in erster Linie durch Behandeln mit einem Kohlenmonoxyd-aufnemenden Schwermetallkomplex durchgeführt. Solche Schwermetallkomplexe sind insbesondere Platinmetall-Komplexe, wobei man unter einem Platinmetall ausser Platin, auch Iridium, Rhodium, Palladium und Osmium versteht.

Vorzugsweise verwendet man Bis-trisubstituierte Phosphin-platinhalogenide, Bis-trisubstituierte Phosphin-carbonyliridiumhalogenide oder Tris-trisubstituierte Phosphin-iridiumhalogenide, in erster Linie Tris-trisubstituierte Phosphin-rhodiumhalogenide, worin die Substituenten des Phosphins vorzugsweise Niederalkyl, z.B. n-Butyl, und in erster Linie Phenyl darstellen, und die Halogenide in erster Linie Chloride sind. Solche Phosphinplatinmetall-Komplexe, welche Kohlenmonoxyd in kovalenter Bindung aufzunehmen vermögen, sind z.B. Bis-triphenylphosphin-platin-II-chlorid $[(C_6H_5)_3P]_2PtCl_2$ oder Bis-triphenyl-phosphin-carbonyliridium-II chlorid $[(C_6H_5)_3P]_2\ Ir(CO)Cl$, sowie Tris-triphenylphosphin-iridium-I-chlorid $[(C_6H_5)_3P]_3IrCl$, in erster Linie aber Tris-triphenyl-phosphin-rhodium-I-chlorid $[(C_6H_5)_3P]_3RhCl$.

Wenn erwünscht oder notwendig, kann die Decarbonylierung mit den obengenannten Schwermetallkomplexen in Gegenwart von geeigneten Katalysatoren oder Aktivierungsmitteln, z.B. Lewissäure, wie Bortrifluorid (das man z.B. zusammen mit dem Bis-triphenylphospin-platinchlorid verwenden kann), ferner eines Perchlorats, wie Alkalimetallperchlorates, z.B. Natriumperchlorat (das man z.B. zusammen mit Bis-triphenylphosphin-carbonyliridiumchlorid verwenden kann), durchgeführt werden.

Vorzugsweise arbeitet man in Gegenwart von inerten Lösungsmitteln, insbesondere von Kohlenwasserstoffen, wie aliphatischen oder cycloaliphatischen, insbesondere aromatischen Kohlenwasserstoffen, z.B. Benzol, Toluol oder Xylol, halogenierten Kohlenwasserstoffen, wie entsprechenden aliphatischen oder aromatischen Chlorkohlenwasserstoffen, z.B. Methylenchlorid oder Chlorbenzol, Aethern, wie aliphatischen, cycloaliphatischen oder aromatischen, ferner gemischten Aethern, z.B. Di-n-butyläther, Dioxan, Diphenyläther oder Anisol, Nitrilen, wie aliphatischen oder aromatischen Nitrilen, z.B.

Acetonnitril oder Benzonitril, oder Ketonen, insbesondere aliphatischen Ketonen, wie Niederalkanonen, z.B. Aceton, Aethylmethylketon oder Isobutylmethylketon, oder Gemischen von solchen Lösungsmitteln. Dabei wird die Reaktion unter Kühlen, bei Zimmertemperatur oder unter Erwärmen, z.B. bei etwa 10°C bis etwa 150°C, wie bei etwa 40°C bis etwa 120°C, ferner, wenn notwendig, in einem geschlossenen Gefäss und/oder in einer inerten Gasatmosphäre, z.B. unter Stickstoff oder Argon, durchgeführt.

10. Umwandlungen von $R_2$ in anderes $R_2$: In einem erhaltenen Zwischenprodukt der Formel I, worin mindestens eine der funktionellen Gruppen in geschützter Form vorliegt, kann eine im Rest $R_1$ vorhandene Gruppe $R_2$ gegen eine andere Gruppe $R_2$ ausgetauscht werden, wie für diese Reaktion bei den Endprodukten der Formel I angegeben ist.

11. Methoxylierung der 7α-Stellung: In einem erhaltenen Zwischenprodukt der Formel I, worin $R_3$ Wasserstoff ist und alle funktionellen Gruppen in geschützter Form vorliegen, kann die 7α-Methoxygruppe $R_3$ auf an sich bekannte Weise eingeführt werden, beispielsweise indem man das genannte Zwischenprodukt nacheinander mit einem Anionenbildenden Mittel, einem N-Halogenierungsmittel und Methanol behandelt.

Ein geeignetes Anion-bildendes Mittel ist in erster Linie eine metallorganische Base, insbesondere eine Alkalimetall-, in erster Linie eine Lithium-organische Base. Solche Verbindungen sind insbesondere entsprechende Alkoholate, wie geeignete Lithium-niederalkanolate, in erster Linie Lithiummethylat, oder entsprechende Metall-Kohlenwasserstoffbasen, wie Lithium-niederalkane und Lithiumphenyl. Die Umsetzung mit der Anion-bildenden metallorganischen Base wird üblicherweise unter Kühlen, z.B. von etwa 0° C bis etwa -80° C, und in Gegenwart eines geeigneten Lösungs- oder Verdünnungsmittels, z.B. eines Aethers, wie Tetrahydrofuran, bei Verwendung von Lithiummethylat auch in Gegenwart von Methanol, und, wenn erwünscht, in einem geschlossenen Gefäss und/oder in einer Inertgas-, z.B. Stickstoffatmosphäre, vorgenommen.

Als N-halogenierendes Mittel verwendet man üblicherweise ein sterisch gehindertes, organische Hypohalogenit, insbesondere -chlorit, und in erster Linie ein entsprechendes aliphatisches Hypohalogenit, z.B. -chlorit, wie ein tert.-Niederalkyl-hypohalogenit, z.B. -chlorit. In erster Linie wendet man das tert.-Butylhypochlorit an, das man mit dem nichtisolierten Produkt der Anionisierungsreaktion umsetzt.

Die N-halogenierte Zwischenverbindung wird bei Anwesenheit eines Ueberschusses der Anion-bildenden Base, insbesondere von Lithiummethylat, unter den Reaktionsbedingungen und ohne isoliert zu werden in eine 7-Acyliminocephemverbindung umgewandelt, und diese durch Zugabe von Methanol in eine 7α-Methoxy-cephem-Verbindung übergeführt. Falls notwendig, müssen aus dem N-halogenierten Zwischenprodukt die Elemente der Halogenwasserstoff-, insbesondere der Chlorwasserstoffsäure, abgespalten werden; dies geschieht unter Zugabe einer Halogenwasserstoff-abspaltenden Base, wie eines geeigneten Alkalimetall-niederalkanolats, z.B. Lithium-tert.-butylat, wobei diese Reaktion üblicherweise unter den Bedingungen der Anion- und N-Halogenverbindung-bildenden Reaktion stattfindet, wobei man in Gegenwart von Methanol arbeiten und anstelle der Acyliminoverbindung direkt die 7α-Methoxy-cephem-Verbindung erhalten kann. Man geht üblicherweise aus von einer Verbindung der Formel I, worin funktionelle Gruppen in geschützter Form vorliegen, setzt diese mit einem Ueberschuss des Anion-bildenden Mittels, z.B. Lithiummethylat oder Phenyllithium, in Gegenwart von Methanol um, behandelt dann mit dem N-Halogenierungsmittel, z.B. tert.-Butylhypochlorit, und erhält so direkt die gewünschte Verbindung der Formel I, worin funktionelle Gruppen geschützt sind. Man kann auch das Methanol nachträglich zugeben, wobei man die Dehydrohalogenierung und die Zugabe von Methanol bei etwas höheren Temperaturen als die Anion- und N-Halogenverbindung-bildenden Reaktionen, z.B. bei etwa 0° C bis etwa -20° C, wenn notwendig, in einem geschlossenen Gefäss und/oder in einer Inertgas-, z.B. Stickstoffatmosphäre, durchführen kann.

Bei vorstehenden Reaktionen, die unter basischen Bedingungen durchgeführt werden, können 3-Cephemverbindungen, gegebenenfalls teilweise, zu 2-Cephemverbindungen isomerisieren. Eine erhaltene 2-Cephemverbindung oder ein Gemisch aus einer 2- und einer 3-Cephemverbindung kann in an sich bekannter Weise zur gewünschten 3-Cephemverbindung isomerisiert werden.

Herstellung der Ausgangsverbindungen:
Ausgangsverbindungen der Formel II sowie entsprechende Verbindungen mit geschützten funktionellen Gruppen sind bekannt oder können auf an sich bekannte Weise hergestellt werden.

Verbindungen der Formel III, worin die Aminocarbonsäuregruppierung HOOC-CH($NH_2$)- und gegebenenfalls in der Gruppierung A vorhandene weitere funktionelle Gruppen in geschützter Form vorliegen, sind neu und bilden ebenfalls einen Gegenstand der vorliegenden Erfindung.

Solche Verbindungen der Formel III, mit entsprechend geschützten funktionellen Gruppen, werden beispielsweise hergestellt, indem man eine Verbindung der Formel

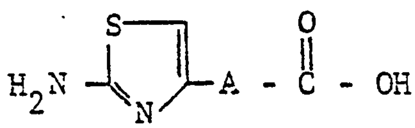 (XIV),

worin die Aminogruppe gegebenenfalls durch eine die Acylierung erlaubende Gruppe substituiert sein kann, und gegebenenfalls in der Gruppierung -A- vorhandene funktionelle Gruppen geschützt sind, unter intermediärem Schutz der Carboxylgruppe, mit einem den entsprechenden Acylrest einer Carbonsäure der Formel V einführenden Acylierungsmittel, worin die Aminocarbonsäuregruppierung HOOC-CH($NH_2$)- in geschützter Form vorliegt, acyliert, und wenn erwünscht, eine erhaltene Verbindung in eine andere Verbindung der Formel III mit entsprechend geschützten funktionellen Gruppen überführt.

Die die Acylierung erlaubenden Gruppen, sowie die Schutzgruppen der Aminocarbonsäuregruppierung HOOC-CH($NH_2$)- und der Gruppierung A, sind die gleichen wie die unter den Verbindungen der Formeln II oder IV,

sowie I erwähnten. Zum intermediären Schutz der Carboxylgruppe in der Verbindung der Formel XIV können an sich ebenfalls die bereits erwähnten Carboxylschutzgruppen verwendet werden, jedoch müssen sich die zum intermediären Schutz in der vorliegenden Acylierung benützten Carboxylschutzgruppen von den übrigen Schutzgruppen, die in den Verbindungen der Formel III notwendigerweise vorhanden bleiben sollen, in der Art ihrer Abspaltung unterscheiden, so dass sie nach der vorliegenden Acylierungsreaktion selektiv abgespalten werden können. Wenn beispielsweise zum intermediären Schutz der Carboxylgruppe eine hydrogenolytisch abspaltbare Schutzgruppe, wie eine der genannten gegebenenfalls substituierten Benzylgruppen, z.B. die Benzyl- oder p-Nitrobenzylgruppe, verwendet wird, dann dürfen die anderen Schutzgruppen hydrogenolytisch nicht abspaltbar sein; sie können beispielsweise die genannten, nur acidolytisch abspaltbaren tert.-Niederalkylgruppen, wie tert.Butyl, bzw. tert.-Niederalkoxycarbonylgruppen, wie tert.-Butoxycarbonyl, sein.

Die Acylierung kann im übrigen analog ausgeführt werden wie die Acylierung von Verbindungen der Formel IV mit einer Säure der Formel V, bzw. einem entsprechend geschützten und reaktionsfähigen funktionellen Derivat davon.

In einer erhaltenen Verbindung der Formel III, mit entsprechend geschützten funktionellen Gruppen, kann eine Schutzgruppe, gegebenenfalls selektiv, abgespalten, oder eine, gegebenenfalls bei der Acylierungsreaktion frei gewordene, funktionelle Gruppe geschützt werden. In einer erhaltenen Verbindung der Formel III, worin A Hydroxymethylen bedeutet, kann die Hydroxygruppe oxidativ, wie für die Oxidation von entsprechenden Verbindungen I, worin A eine Hydroxymethylengruppe ist, angegeben,

z.B., unter vorübergehendem Schutz der Carboxylgruppe als Ester, durch Behandeln mit Mangandioxid, in eine Oxomethylengruppe übergeführt werden und in einer erhältlichen Verbindung der Formel III, worin A die Oxomethylengruppe bedeutet, kann diese durch Behandeln mit einem Hydroxylamin der Formel $H_2N\text{-}O\text{-}R^o$, in die entsprechende Oxyiminogruppe übergeführt werden, welche Reaktion analog der Ueberführung von Verbindungen der Formel I, worin A Oxomethylen ist, in Verbindungen der Formel I, worin A eine entsprechende Oxyiminogruppe ist, ausgeführt werden kann.

Verbindungen der Formel IV, worin die Aminogruppe gegebenenfalls durch eine die Acylierung erlaubende Gruppe substituiert sein kann, und worin die 4-Carboxylgruppe und gegebenenfalls im Rest $R_1$ und in der Gruppierung -A- vorhandene weitere funktionelle Gruppen in geschützter Form vorliegen können, sind zum Teil bekannt und zum Teil neu. Sie können nach an sich bekannten Verfahren hergestellt werden.

Neue Verbindungen der Formel IV sind insbesondere solche, worin A Aminomethylen, $R_3$ Wasserstoff oder Methoxy und $R_1$ Wasserstoff, eine der genannten veräthertem Hydroxy- oder Mercaptogruppen oder Halogen bedeuten und ihre geschützten Derivate.

Diese neuen Verbindungen, worin die Carboxylgruppe gegebenenfalls in physiologisch spaltbarer Form verestert ist, und worin die Aminogruppen in freier Form vorliegen, sowie ihre pharmazeutisch verwendbaren Salze, haben ebenfalls antibiotische Wirksamkeit. Zusammen mit dem Verfahren zu ihrer Herstellung sind die neuen Verbindungen ebenfalls Gegenstand der vorliegenden Erfindung.

Die genannten neuen, antibiotisch wirksamen Verbindungen der Formel IV können als antibakterielle Antibiotika verwendet werden. Sie sind gegen eine Reihe von pathogenen Mikroorganismen, insbesondere grampositive und gramnegative Bakterien, wie Staphylococcus aureus, inclusive resistente Staphylococcus Stämme, Escherichia coli und Proteus vulgaris wirksam (z.B. werden im Plattentest gegen die genannten Keime Hemmzonen von etwa 11 bis 30 mm festgestellt), und können deshalb entsprechend, z.B. in Form von antibiotisch wirksamen Präparaten, zur Behandlung von durch solche Mikroorganismen verursachten Infektionen Verwendungen finden. Diese Verbindungen sind insbesondere auch oral wirksam.

Bevorzugt sind solche Verbindungen der Formel IV, worin A Aminomethylen, insbesondere in der D-Form, $R_1$ Wasserstoff, Methoxy oder Chlor und $R_3$ Wasserstoff ist, sowie pharmazeutisch annehmbare Salze davon.

Verbindungen der Formel IV, worin gegebenenfalls die Aminogruppe und die anderen funktionellen Gruppen wie angegeben substituiert bzw. geschützt sein können, sowie ihre Salze, können analog wie die Verbindungen I hergestellt werden, z.B. indem man eine Verbindung der Formel II, worin die Aminogruppe gegebenenfalls durch eine die Acylierung erlaubende Gruppe substituiert ist und worin die 4-Carboxylgruppe und gegebenenfalls im Rest $R_1$ vorhandene weitere funktionelle Gruppen in geschützter Form vorliegen können, mit einem den Acylrest einer Säure der Formel XIV einführenden Acylierungsmittel, worin die Aminogruppe gegebenenfalls geschützt sein kann, und gegebenenfalls in der Gruppierung -A- vorhandene funktionelle Gruppen geschützt sind, z.B. mit einem reaktionsfähigen funktionellen Derivat einer solchen Säure oder einem Salz davon, acyliert, wenn gewünscht in einer erhaltenen Verbindung die Schutzgruppen abspaltet und/oder, wenn gewünscht, eine erhaltene Verbindung, worin $R_3$ Wasserstoff ist, in eine Verbindung

überführt, worin $R_3$ Methoxy ist, und/oder wenn notwendig, eine erhaltene 2-Cephemverbindung oder ein erhaltenes Gemisch einer 2-Cephem- und 3-Cephemverbindung, zur 3-Cephemverbindung isomerisiert, und/oder, wenn gewünscht, ein erhaltenes Isomerengemisch in die Isomeren auftrennt, und/oder wenn gewünscht, eine erhaltene Verbindung mit salzbildender Gruppe in ein Salz oder ein erhaltenes Salz in die freie Verbindung überführt.

Die die Acylierung erlaubenden Gruppen in den Verbindungen der Formel II, sowie die Schutzgruppen, sind die gleichen, die bereits oben erwähnt wurden.

Die Acylierung von entsprechenden geschützten Verbindungen der Formel II,die Abspaltung der Schutzgruppen, und die Einführung der Methoxygruppe $R_3$, sowie die Salzbildung können analog ausgeführt werden, wie bei der Acylierung von entsprechend geschützten Verbindungen der Formel II oder IV mit einer Säure der Formel III bzw. V und den entsprechenden Nachoperationen angegeben ist.

Säuren der Formel V, reaktionsfähige funktionelle Derivate davon, die Vorstufen der Formel $HOOC-CH(NH_2)-(C_nH_{2n})-X-H$ (VI) und entsprechend geschützte Derivate sind bekannt oder können nach an sich bekannten Methoden, beispielsweise in situ, hergestellt werden.

Reaktionsfähige funktionelle Derivate von Säuren der Formel VII, worin die 4-Carboxylgruppe und gegebenenfalls im Rest $R_1$ und in der Gruppierung -A- vorhandene funktionelle Gruppen in geschützter Form vorliegen können, werden auf an sich bekannte Weise aus entsprechend geschützten Verbindungen der Formel IV hergestellt.

Säuren der Formel XIV und entsprechende reaktionsfähige funktionelle und geschützte Derivate davon sind bekannt oder können nach an sich bekannten Methoden hergestellt werden.

Ausgangsverbindungen der Formel VIII sind bekannt oder können auf an sich bekannte Weise, z.B. durch Acylierung von Verbindungen der Formel II, und gegebenenfalls entsprechende Nachoperationen, erhalten werden.

Ausgangsverbindungen der Formel IX, worin funktionelle Gruppen, insbesondere die Aminocarbonsäuregruppierung, gegebenenfalls geschützt sind, sind neu. Sie können beispielsweise hergestellt werden, indem man eine Verbindung der Formel VI, worin die Aminocarbonsäuregruppierung in geschützter Form vorliegt, in Gegenwart einer Base mit Phosgen oder Thiophosgen behandelt, das erhaltene Säurechlorid einer Verbindung der Formel V mit einem Salz der Rhodanwasserstoffsäure, z.B. mit einem Alkalimetall-, wie Kaliumrhodanid, behandelt und die erhaltene Verbindung der Formel

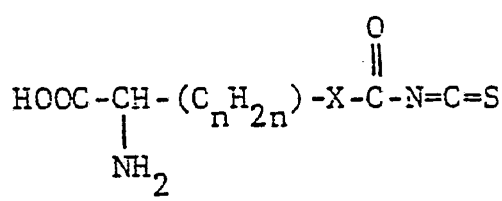

mit Ammoniak behandelt.

Ausgangsverbindungen der Formel X, worin $R_1^a$ eine gegebenenfalls veresterte Hydroxygruppe ist, fallen unter die Verbindungen der Formel I mit entsprechenden geschützten Gruppen, und können dementsprechend wie dort angegebenen hergestellt werden.

Ausgangverbindungen der Formel X, worin $R_1^a$ eine sekundäre oder tertiäre Aminogruppe $-N(R_4^a)(R_4^b)$ ist, können aus den Verbindungen der Formel X, worin $R_1^a$ eine gegebenenfalls veresterte Hydroxygruppe ist, durch Umsatz mit einem Amin der Formel $HN(R_4^a)(R_4^b)$, oder ausgehend von Verbindungen der Formeln II, IV, VII, VIII, XII oder XIII, in denen $R_1$ die Bedeutung einer sekundären oder tertiären Aminogruppe $-N(R_4^a)(R_4^b)$ hat, gegebenenfalls unter Schutz nicht an den Reaktionen teilnehmender Gruppen, analog den Verfahren a), b), c), d), f) und g), erhalten werden.

Die gegebenenfalls als Zwischenprodukte im Verfahren e) auftretenden Cephamverbindungen können auch analog den Acylierungs- und Kondensationsverfahren a) bis d), ausgehend von den bekannten oder auf an sich bekannte Weise herstellbaren Cephamverbindungen der Formel

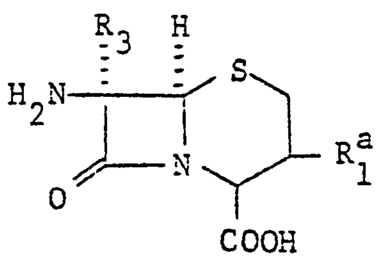

hergestellt werden.

Ausgangsverbindungen der Formel XI, mit geschützten funktionellen Gruppen, können auf an sich bekannte Weise hergestellt werden, z.B. analog der DT-OS 2.151.567, wobei in letzter Stufe eine entsprechende Hydroxyverbindung mit der Teilformel $-S-CH_2CH_2-OH$ zur Carbonylverbindung der Formel XI oxidiert wird.

Die Oxydation kann durch Behandeln mit einer oxidierenden organischen Sulfoxidverbindung in Gegenwart von Mitteln mit wasserentziehenden oder wasseraufnehmenden Eigenschaften durchgeführt werden. Als oxidierende Sulfoxidverbindungen kommen in erster Linie aliphatische Sulfoxidverbindungen in Frage, wie Diniederalkylsulfoxide,

in erster Linie Dimethylsulfoxid, oder Niederalkylensulfoxide, z.B. Tetramethylensulfoxid. Als Mittel mit wasserentziehenden oder -aufnehmenden Eigenschaften sind in erster Linie Säureanhydride zu nennen, insbesondere Anhydride von organischen, wie aliphatischen oder aromatischen Carbonsäuren, z.B. Anhydride von Niederalkancarbonsäuren, insbesondere Essigsäureanhydrid, ferner Propionsäureanhydrid, oder Benzoesäureanhydrid, sowie Anhydride von anorganischen Säuren, insbesondere von Phosphorsäuren, wie Phosphorpentoxyd. Die obigen Anhydride, in erster Linie von organischen Carbonsäuren, z.B. Essigsäureanhydrid, werden vorzugsweise in einem etwa 1:1-Gemisch mit dem Sulfoxid-oxidationsmittel verwendet. Weitere wasserentziehende oder -aufnehmende Mittel sind Carbodiimide, in erster Linie Dicyclohexylcarbodiimid, ferner Diisopropylcarbodiimid, oder Ketenimine, z.B. Diphenyl-N-p-tolylketenimin; diese Reagentien werden vorzugsweise in Gegenwart von sauren Katalysatoren, wie Phosphorsäure oder Pyridinium-trifluoracetat oder -phosphat verwendet. Schwefeltrioxyd kann ebenfalls als wasserentziehendes oder -aufnehmendes Mittel verwendet werden, wobei man es üblicherweise in Form eines Komplexes, z.B. mit Pyridin, zur Anwendung bringt.

Ueblicherweise verwendet man das Sulfoxid-oxidationsmittel im Ueberschuss. Unter den Reaktionsbedingungen flüssige Sulfoxidverbindungen, insbesondere das Dimethylsulfoxid, können z.B. gleichzeitig als Lösungsmittel dienen; als Lösungsmittel können zusätzlich inerte Verdünnungsmittel, wie gegebenenfalls halogenierte Kohlenwasserstoffe, vorzugsweise aliphatischen oder aromatischen Charakters, z.B. Benzol, oder Gemische von Lösungsmitteln verwendet werden.

Die obige Oxidationsreaktion wird, wenn erwünscht, unter Kühlen, meist aber bei Zimmertemperatur oder leicht erhöhter Temperatur, z.B. bei Temperaturen von etwa -20°C bis etwa 100°C durchgeführt.

Ausgangsverbindungen der Formel XII, mit geschützten funktionellen Gruppen, können auf an sich bekannte Weise hergestellt werden, z.B. analog der DT-OS 2.506.330 oder den belgischen Patentschriften 838.656 oder 844.344.

2-Cephemausgangsverbindungen der Formel XIII sind neu. Sie können, analog den Acylierungs- und Kondensationsverfahren a) bis d), ausgehend von den bekannten oder auf an sich bekannte Weise herstellbaren 2-Cephemverbindungen der Formel

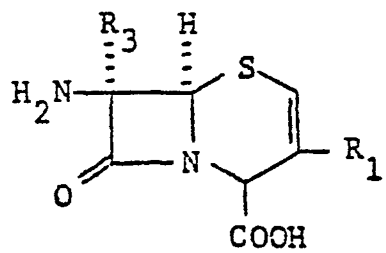

(XV),

hergestellt werden. Ausserdem können sie als Nebenprodukte bei den Verfahren a) bis e) entstehen, insbesondere wenn unter basischen Bedingungen gearbeitet wird.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich vorzugsweise zur parenteralen Verabreichung eignen.

Vorzugsweise verwendet man die pharmakologisch wirksamen Verbindungen der vorliegenden Erfindung in Form von injizierbaren, z.B. intravenös, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfstoffe, z.B. Konservier-, Stabilisier-, Netz und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes. Je nach Art der Infektion und Zustand des infizierten Organismus verwendet man tägliche Dosen von etwa 0,5 g bis etwa 5 g s.c. zur Behandlung von Warmblütern von etwa 70 kg Gewicht.

Die antibiotisch wirksamen Verbindungen der Formel IV, können auch oral verabreicht werden, z.B. in Form üblicher oral applizierbarer pharmazeutischer Präparate, wie Tablette Kapseln oder Lösungen, die eine wirksame Menge der Verbindung und gegebenenfalls einen üblichen pharmazeutischen akzeptablen Zusatzstoff enthalten.

Die folgenden Beispiele dienen zur Illustration der Erfindung; Temperaturen werden in Celsiusgraden angegeben.

In den Beispielen werden folgende Abkürzungen verwendet:

| | |
|---|---|
| BOC: | tert.-Butyloxycarbonyl |
| F: | Schmelzpunkt |
| DS: | Dünnschichtchromatogramm: auf Silicagel-Fertigplatten SL 254 der Fa. Antec, Birsfelden |
| $Rf_{96}$: | Rf-Wert im Lösungsmittelsystem sec. Butanol-Eisessig-Wasser 67:10:23 |

Beispiel 1: 7β-{2-[2-((2R)-2-Amino-2-carboxyäthoxycarbonylamino)thiazol-4-yl]acetylamino}-3-acetoxymethyl-3-cephem-4-carbonsäure-natriumsalz-trihydrat

a) Eine auf 0° gekühlte Lösung von 7 g (8.6 mMol) 7β-{2-[2-((2R)-2-BOC-Amino-2-diphenylmethoxycarbonyläthoxycarbonylamino)thiazol-4-yl]acetylamino}-3-acetoxymethyl-3-cephem-4-carbonsäure und 7 ml Anisol in Methylenchlorid (35 ml) werden mit 35 ml kalter Trifluoressigsäure versetzt und 30 Minuten bei 0° unter einer Stickstoffatmosphäre und Feuchtigkeitsausschluss gerührt. Nach Zugabe von Diäthyläther (700 ml) wird das in kristalliner Form anfallende Trifluoracetat der Titelverbindung abfiltriert, mit Diäthyläther gewaschen und am Hochvakuum bei Raumtemperatur getrocknet. Die wässrige Lösung (40 ml) des rohen Trifluoracetats wird mit konz. Natriumhydrogencarbonatlösung auf einen pH-Wert von 6 eingestellt, mit Essigsäureäthylester (2x20 ml) extrahiert und mit Aceton (200 ml) versetzt. Der gebildete Niederschlag wird abfiltriert, mit Aceton gewaschen, zweimal in wenig Wasser gelöst und am Rotationsverdampfer eingeengt, woraus nach Trocknung am Hochvakuum die Titelverbindung in Form eines amorphen Pulvers erhalten wird.

$[\alpha]_D = + 86 \pm 1^\circ (c = 1.127\ \%, H_2O)$

DS: $Rf_{96} = 0,17$

Die Ausgangsmaterialien können wie folgt erhalten werden:

b) Eine Suspension von 100 g (2R)-Serin in 1 l Wasser wird durch langsame Zugabe von 100 g festem, wasserfreiem Natriumcarbonat in Lösung gebracht, dann versetzt man mit 2 l Dioxan sowie 346 g Di-tert.-butyl-pyrocarbonat und rührt 30 Minuten bei +20°. Das Dioxan wird im Vakuum abgedampft und die wässrige Phase bei 0° und pH 2,0 mit

Essigester extrahiert. Man erhält 236,7 g amorphes, rohes (2R)-N-BOC-Serin, das ca. 195 g reines Produkt enthält. Beim Verreiben mit Petroläther und Kühlen kann daraus die reine Verbindung erhalten werden; F. 78-83° (Zersetzung).

c) Zu einer Lösung von 236,7 g des erhaltenen rohen (2R)-BOC-Serins in 700 ml Methylenchlorid tropft man bei 22° unter Kühlen eine Lösung von ca. 214 g Diphenyldiazomethan in 1 Liter Methylenchlorid und rührt 1½ Stunden bei 20°. Das Gemisch wird im Vakuum eingedampft, der Rückstand in Aethylacetat aufgenommen und bei 0° mit Phosphat-Puffer von pH 2,0 dann von pH 7,0 gewaschen. Die getrocknete, organische Phase gibt beim Eindampfen einen kristallinen Rückstand, der in Hexan digeriert wird und man erhält nach Filtrieren den (2R)-N-BOC-Serin-diphenylmethylester; F. 116-117° (korr.). $[\alpha]_D^{20} = +6° \pm 1°$ (c = 1, Chloroform).

d) Eine Lösung von 4.536 g (11 mMol) 7β-[2-(2-Aminothiazol-4-yl)acetylamino]-3-acetoxymethyl-3-cephem-4-carbonsäure in 100 ml absolutem Methylenchlorid wird unter einer Stickstoffatmosphäre bei Raumtemperatur mit 4.9 ml N,O-Bis-(trimethylsilyl)-acetamid versetzt und während einer Stunde gerührt (Lösung A). Zu einer auf 0° gekühlten Lösung von Phosgen in Toluol (5.2 ml; 20 Gew-%), verdünnt mit 30 ml Methylenchlorid, werden 3.71 g (10 mMol) (2R)-N-BOC-Serin-diphenylmethylester gelöst in 70 ml abs. Methylenchlorid, dann 0,85 ml (10,5 mMol) abs. Pyridin zugetropft und eine Stunde bei 0° unter einer Stickstoffatmosphäre gerührt (Lösung B). Darauf wird zur Lösung B die Lösung A und anschliessend 0,85 ml (10.5 mMol) abs. Pyridin unter Rühren bei 0° gegeben. Das Reaktionsgemisch wird 30 Min. bei 0° und zwei Stunden bei Raumtemperatur gerührt, mit Essigsäureäthylester (800 ml) verdünnt, mit Wasser (3x50 ml) und gesättigter Natriumchloridlösung (50 ml) gewaschen, über Natriumsulfat getrocknet und am

Rotationsverdampfer vom Lösungsmittel befreit. Der Rückstand enthält die 7β-{2-[2-((2R)-2-BOC-Amino-2-diphenylmethoxycarbonyläthoxycarbonylamino)thiazol-4-yl]acetylamino}-3-acetoxymethyl-3-cephem-4-carbonsäure als amorphes gelbes Pulver.

DS: $Rf_{96}$ = 0.59

Die gemäss Beispiel d) erhältliche Verbindung kann auch wie folgt hergestellt werden:

e) Eine auf -20° C gekühlte Lösung von 5,4 ml (11 mMol) Phosgen in Toluol (20-Gew %), verdünnt mit 10 ml absoluten Tetrahydrofuran, wird unter Rühren und Feuchtigkeitsausschluss innert ca. 15 Minuten mit 3,71 g (10 mMol) (2R)-N-BOC-Serin-diphenylmethylester und 0,81 absolutem Pyridin gelöst in 40 ml absolutem Tetrahydrofuran versetzt. Nach 15 Minuten wird der Niederschlag (Pyridin-Hydrochlorid) bei 0° abfiltriert und das Filtrat bei -10° zu einer Lösung von 1,07 g (11 mMol) Kaliumrhodanid in 20 ml absolutem Aceton gegeben. Nach 30 Minuten wird das Reaktionsgemisch mit 4,4 ml 2,5 molarer methanolischer Ammoniaklösung versetzt, innert einer Stunde auf Raumtemperatur gebracht und anschliessend am Rotationsverdampfer vom Lösungsmittel befreit. Der Rückstand,gelöst in Essigsäureäthylester (100 ml),wird mit Wasser (2x20 ml) gewaschen, über Natriumsulfat getrocknet und eingeengt. Das Rohprodukt wird an der 25-fachen Menge Kieselgel mit Chloroform-Aceton (9:1) als Eluiermittel gereinigt, woraus nach der Kristallisation aus Diäthyläther der N-((2R)2-BOC-Amino-2-diphenylmethoxycarbonyläthoxycarbonyl)thioharnstoff als farbloses Kristallisat vom Smp. 145-147° erhalten wird.

DS: Rf = 0,34 (Fliessmittel:Chloroform-Aceton 9:1).

f) Eine Lösung von 391 mg (1,0 mMol) 7β-(4-Chlor-3-oxo-butyramido)-cephalosporansäure, 84 mg (1,0 mMol) Natriumbicarbonat und 521 mg (1,1 mMol) N-((2R)2-BOC-Amino-2-diphenylmethoxycarbonyläthoxycarbonyl)-thioharnstoff in 5 ml absolutem Aethanol wird unter einer Stickstoffatmosphäre bei 70° C während fünf Stunden gerührt. Nach Entfernung des Lösungsmittels wird der Rückstand in Essigsäureäthylester (ca. 20 ml) gelöst und zweimal mit verdünnter Salzsäure (pH-2) gewaschen. Nach Trocknung der organischen Phase mit Natriumsulfat und Entfernung des Lösungsmittels am Rotationsverdampfer wird das Rohprodukt an Kieselgel mit Methylenchlorid-Essigsäureäthylester (2:1) gereinigt. Die erhaltene 7β{2-[2-((2R)-2-BOC-Amino-2-diphenylmethoxycarbonyläthoxycarbonylamino)thiazol-4-yl]acetylamino}-3-acetoxymethyl-3-cephem-4-carbonsäure ist mit der gemäss Beispiel 1d) hergestellten Verbindung identisch.

Beispiel 2: 7β-{2-[2-((2R)-2-Amino-2-carboxyäthoxycarbonylamino)thiazol-4-yl]-2-syn-methoxyiminoacetylamino}-3-acetoxymethyl-3-cephem-4-carbonsäure-natriumsalz

a) Eine auf 0° gekühlte Lösung von 4.5 g 7β-{2-[2-((2R)-2-BOC-Amino-2-diphenylmethoxycarbonyläthoxycarbonylamino)thiazol-4-yl]-2-syn-methoxyiminoacetylamino}-3-acetoxymethyl-3-cephem-4-carbonsäure und 4.5 ml Anisol in absolutem Methylenchlorid (23 ml) werden mit 23 ml Trifluoressigsäure versetzt und 30 Minuten bei 0° unter einer Stickstoffatmosphäre und Feuchtigkeitsausschluss gerührt. Nach der Zugabe von Diäthyläther (300 ml) wird das in kristalliner Form anfallende Trifluoracetat der Titelverbindung abfiltriert, mit Diäthyläther gewaschen und am Hochvakuum bei Raumtemperatur getrocknet. Die wässrige Lösung (20 ml) des erhaltenen rohen Trifluoracetates (pH~2) wird mit gesättigter Natriumhydrogencarbonatlösung auf einen pH-Wert von 7 eingestellt und an Amberlite XAD-2 mit Wasser-Isopropanol (12:88) als Eluiermittel chromatographiert. Die dünnschichtchromatographisch einheitlichen Fraktionen werden vereinigt, am Rotationsverdampfer eingeengt, der Rückstand wird aus 70 ml Wasser-Isopropanol 1:4 kristallisiert und nach der Filtration am Hochvakuum bei Raumtemperatur getrocknet. Man erhält die Titelverbindung als blassgelbes Kristallisat mit 1.6 Mol Kristallwasser.

F. ab 170° (Zersetzung); $[\alpha]_D = + 50 \pm 1°$ (c = 1.024%, $H_2O$)

DS: $Rf_{96} = 0.14$

Die Ausgangsmaterialien können wie folgt erhalten werden:

b) Zu einer Lösung von 28 g Kaliumhydroxid in 160 ml Wasser werden unter Rühren 21.5 g (0.1 Mol) 2-(2-Amino-thiazol-4-yl)-2-syn-methoxyiminoessigsäuremethylester gelöst in 900 ml Aethanol gegeben. Das Reaktionsgemisch wird während 3 Stunden bei Raumtemperatur gerührt. Nach Entfernung des Lösungsmittels wird der Rückstand in 200 ml Wasser gelöst und mit Essigsäureäthylester (3x100 ml) extrahiert. Die auf 0° gekühlte wässrige Phase wird mit 2N Salzsäure auf einen pH-Wert von 3 eingestellt und noch ca. 1 Stunde weitergerührt. Der gebildete Niederschlag wird abfiltriert, mit wenig Eiswasser und Aceton gewaschen und am Hochvakuum bei Raumtemperatur getrocknet. 2-(2-Amino-thiazol-4-yl)-2-syn-methoxyiminoessigsäure wird so als inneres Salz isoliert.

F. 119-122° (Zersetzung);DS: $Rf_{96}$ = 0.14.

c) Eine auf 0° gekühlte Lösung von 20.4 ml Phosgen in Toluol (20 Gew.%), verdünnt mit 20 ml Methylenchlorid, wird unter Rühren und Feuchtigkeitsausschluss tropfenweise mit 1 ml abs. Pyridin, anschliessend mit 4.45 g (12 mMol) (2R)-N-BOC-Serin-diphenylmethylester gelöst, in 20 ml Methylenchlorid versetzt und eine Stunde bei 0° gerührt. Die Suspension (Niederschlag von Pyridin-Hydrochlorid) wird mit Methylenchlorid (150 ml) verdünnt, mit Eiswasser (3x30 ml) gewaschen und die organische Phase über Natriumsulfat getrocknet. Nach der Entfernung des Lösungsmittels am Rotationsverdampfer wird das ölige Rohprodukt gut mit Petroläther verrührt, woraus nach Filtration und Trocknung am Hochvakuum (2R)-2-BOC-Amino-2-diphenylmethoxycarbonyl-äthoxycarbonylchlorid in kristalliner Form erhalten wird.

F. 78-80° (Zersetzung).

d) Eine Suspension von 4.02 g (0.02 Mol) 2-(2-Amino-thiazol-4-yl)-2-syn-methoxyimino-essigsäure in 100 ml

absolutem Tetrahydrofuran wird unter Rühren und Feuchtigkeitsausschluss mit 9.7 ml N,O-Bis-(trimethylsilyl)acetamid versetzt und eine Stunde bei Raumtemperatur reagieren lassen. Zu der auf $0^o$ gekühlten silylierten Essigsäure werden 8.67 g (0.02 Mol) (2R)-2-BOC-Amino-2-diphenylmethoxycarbonyläthoxycarbonylchlorid, gelöst in 100 ml abs. Tetrahydrofuran, innert ca. 10 Minuten getropft, gefolgt von 1.6 ml absolutem Pyridin. Nach einer Reaktionszeit von 1 Stunde bei $0^o$ und 2 Stunden bei Raumtemperatur wird das Lösungsmittel am Rotationsverdampfer entfernt, der ölige Rückstand in 200 ml kalter 0.5M Natriumhydrogencarbonatlösung gelöst und mit Diäthyläther (2x100 ml) extrahiert. Die wässrige Phase wird mit Essigsäureäthylester (400 ml) überschichtet, mit 2N Salzsäure auf einen pH-Wert von 2 eingestellt und nach Abtrennung der organischen Phase noch zweimal mit Essigsäureäthylester (200 ml) extrahiert. Die vereinigten organischen Phasen werden mit gesättigter Natriumchloridlösung gewaschen, über Natriumsulfat getrocknet und am Rotationsverdampfer vom Lösungsmittel befreit.
Die 2-[2-((2R)-BOC-Amino-2-diphenylmethoxycarbonyläthoxycarbonylamino)thiazol-4-yl]-2-syn-methoxyiminoessigsäure wird in amorpher Form isoliert. DS: $Rf_{96}$ = 0.65

e) Zu einer auf $0^o$ gekühlten Lösung von 5.98 g (10 mMol) 2-[2-((2R)-BOC-Amino-2-diphenylmethoxycarbonyläthoxycarbonylamino)thiazol-4-yl]-2-syn-methoxyiminoessigsäure und 1.35 g (10 mMol) 1-Hydroxy-benztriazol (angefeuchtet mit 10% Wasser) in 60 ml absolutem Tetrahydrofuran wird 2.27 g (11 mMol) N,N'-Dicyclohexylcarbodiimid, gelöst in 20 ml absolutem Tetrahydrofuran, getropft und das Reaktionsgemisch 2 Stunden bei $0^o$ weitergerührt. Zu der gelben Suspension wird eine Lösung von 4.38 g (10 mMol) 7β-Amino-3-acetoxymethyl-3-cephem-4-carbonsäure-diphenylmethylester in 40 ml Tetrahydrofuran getropft. Nach

4 Stunden bei 0° wird der entstandene Niederschlag (N,N'-Dicyclohexylharnstoff) abgenutscht, das Filtrat am Rotationsverdampfeer konzentriert, der Rückstand in Diäthyläther (300 ml) gelöst, dreimal mit verdünnter Natriumhydrogencarbonatlösung und Wasser gewaschen, über Natriumsulfat getrocknet, und das Lösungsmittel am Rotationsverdampfer entfernt. Das Rohprodukt wird an der 50-fachen Menge Kieselgel mit Diäthyläther als Eluiermittel gereinigt. Die dünnschichtchromatographisch einheitlichen Fraktionen an 7β-{2-[2-((2R)-2-BOC-Amino-2-diphenylmethoxycarbonyläthoxycarbonylamino)thiazol-4-yl]-2-syn-methoxyiminoacetylamino}-3-acetoxymethyl-3-cephem-4-carbonsäurediphenylmethylester werden vereinigt und direkt im nächsten Syntheseschritt eingesetzt. DS: Rf = 0.26 (Fliessmittel: Diäthyläther).

Beispiel 3: 7β-{2-[2-((2R)-Amino-2-carboxyäthoxycarbonylamino)thiazol-4-yl]-2-syn-methoxyiminoacetylamino}-3-carbamoyloxymethyl-3-cephem-4-carbonsäure-natriumsalz-trihydrat

a) In analoger Weise zu Beispiel 2a) werden durch Behandeln von 2.5 g (2.5 mMol) 7β-{2-[2-((2R)-2-BOC-Amino-2-diphenylmethoxycarbonyläthoxycarbonylamino)thiazol-4-yl]-2-syn-methoxyiminoacetylamino}-3-carbamoyloxy-3-cephem-4-carbonsäure-diphenylmethylester mit 12.5 ml Trifluoressigsäure in Gegenwart von 2.5 ml Anisol in 12.5 ml absolutem Methylenchlorid die Aminogruppe und die geschützten Carboxylgruppen freigesetzt, das erhaltene Trifluoressigsäure-Salz durch Behandeln mit gesättigter Natriumhydrogencarbonatlösung in Wasser auf pH = 7 gestellt und nach Chromatographie an Amberlite XAD-2 die Titelverbindung erhalten. F. ab 210° (Zersetzung); $[\alpha]_D = + 46 \pm 1°$ (c = 1.416%, $H_2O$); DS: $Rf_{96} = 0.14$

Das Ausgangsmaterial kann wie folgt hergestellt werden:

b) In analoger Weise zu Beispiel 2e) erhält man durch Behandeln des aktivierten Esters (hergestellt aus 2.99 g (5 mMol) 2-[2-((2R)-2-BOC-Amino-2-diphenylmethoxycarbonyläthoxycarbonylamino)thiazol-4-yl]-2-syn-methoxyiminoessigsäure und 0.743 g (5 mMol) 1-Hydroxy-benztriazol in Gegenwart von 1.13 g (5.5 mMol) N,N'-Dicyclohexylcarbodiimid in 40 ml Tetrahydrofuran bei 0° während 2 Stunden) mit 1.98 g (4.5 mMol) 7β-Amino-3-carbamoyloxymethyl-3-cephem-4-carbonsäure-diphenylmethylester, gelöst in 20 ml Tetrahydrofuran, den 7β-{2-[2-((2R)-2-BOC-Amino-2-diphenylmethoxycarbonyläthoxycarbonylamino)thiazol-4-yl]-2-syn-methoxyiminoacetylamino}-3-carbamoyloxy-3-cephem-4-carbonsäure-diphenylmethylester, der nach der chromatographischen Reinigung an Kieselgel mit Diäthyläther-Essigsäureäthylester 1:1 als Eluiermittel im nächsten Syntheseschritt eingesetzt wird. DS: Rf = 0,57 (Fliessmittel: Essigsäureäthylester); Infrarotspektrum in Methylenchlorid: charakteristische Banden bei 3400, 1785, 1730 und 1685 $cm^{-1}$.

<u>Beispiel 4: 7β-{2-[2-((2R)-2-Amino-2-carboxyäthoxycarbonylamino)thiazol-4-yl]-2-syn-methoxyiminoacetylamino}-3-methoxy-3-cephem-4-carbonsäure-natriumsalz-dihydrat</u>

a) In analoger Weise zu Beispiel 2a) werden durch Behandeln von 2.93 g (3 mMol) 7β-{2-[2-((2R)-2-BOC-Amino-2-diphenylmethoxycarbonyläthoxycarbonylamino)thiazol-4-yl]-2-syn-methoxyiminoacetylamino}-3-methoxy-3-cephem-4-carbonsäure-diphenylmethylester mit 15 ml Trifluoressigsäure in Gegenwart von 3 ml Anisol in 15 ml Methylenchlorid die Aminogruppe und die geschützten Carboxylgruppen freigesetzt, das erhaltene Trifluoressigsäure-Salz durch Behandeln mit 10%-iger Natriumhydrogencarbonatlösung in 15 ml Wasser auf pH = 7 gestellt und durch anschliessende Zugabe von Isopropanol (80 ml) die Titelverbindung erhalten. F. 176-179° (Zersetzung); $[\alpha]_D^{20} = +96 \pm 1^\circ$ (c = 1.044, $H_2O$); DS: $Rf_{96} = 0.12$.

Das Ausgangsmaterial kann wie folgt hergestellt werden:

b) In analoger Weise zu Beispiel 2e) erhält man durch Behandeln des aktivierten Esters (hergestellt aus 2.99 g (5 mMol) 2-[2-((2R)-2-BOC-Amino-2-diphenylmethoxycarbonyläthoxycarbonylamino)thiazol-4-yl]-2-syn-methoxyiminoessigsäure und 0.743 g (5 mMol) 1-Hydroxy-benztriazol in Gegenwart von 1.13 g (5.5 mMol) N,N'-Dicyclohexylcarbodiimid in 45 ml Tetrahydrofuran bei 0° während 2 Stunden) mit 2.17 g (5 mMol) 7β-Amino-3-methoxy-3-cephem-4-carbonsäurediphenylmethylester-hydrochlorid, gelöst in 20 ml Tetrahydrofuran, und anschliessend mit 0.56 ml (5 mMol) N-Methylmorpholin den 7β-{2-[2-((2R)-2-BOC-Amino-2-diphenylmethoxycarbonyläthoxycarbonylamino)thiazol-4-yl]-2-syn-methoxyiminoacetylamino}-3-methoxy-3-cephem-4-carbonsäure-diphenylmethylester, der nach der chromatographischen Reinigung an Kieselgel mit Diäthyläther-Essigsäureäthylester 1:1 als Eluiermittel im nächsten Syntheseschritt eingesetzt wird. DS: Rf = 0.47 (Fliessmittel: Diäthyläther-Essigsäureäthylester 1:1).

<u>Beispiel 5: 7β-{2-[2-((2R)-2-Amino-2-carboxyäthoxycarbonylamino)thiazol-4-yl]-2-syn-methoxyiminoacetylamino}-3-[(1-methyl-1H-tetrazol-5-yl)-thiomethyl]-3-cephem-4-carbonsäure-natriumsalz-dihydrat</u>

a) In analoger Weise zu Beispiel 2a) werden durch Behandeln von 2.65 g (2.47 mMol) 7β-{2-[2-((2R)-2-BOC-Amino-2-diphenylmethoxycarbonyläthoxycarbonylamino)thiazol-4-yl]-2-syn-methoxyiminoacetylamino}-3-[(1-methyl-1H-tetrazol-5-yl)-thiomethyl]-3-cephem-4-carbonsäure-diphenylmethylester

mit 13 ml Trifluoressigsäure in Gegenwart von 2.6 ml Anisol in 13 ml Methylenchlorid die Aminogruppe und die geschützten Carboxylgruppen freigesetzt, das erhaltene Trifluoracetat-Salz gelöst in 15 ml Wasser durch Zugabe von 10%-iger Natriumhydrogencarbonat-Lösung auf pH = 7 gestellt und durch anschliessende Zugabe von Isopropanol (80 ml) die Titelverbindung erhalten.
F. ab 150° Zersetzung; $[\alpha]_D = -17 \pm 1°$ (c = 0.92%, $H_2O$);
DS: $Rf_{96}$ = 0.13.

Das Ausgangsmaterial kann wie folgt hergestellt werden:

b) In analoger Weise zu Beispiel 2e) erhält man durch Behandeln des aktivierten Esters (hergestellt aus 2.99 g (5 mMol) 2-[2-((2R)-2-BOC-Amino-2-diphenylmethoxycarbonyläthoxycarbonylamino)thiazol-4-yl]-2-syn-methoxyiminoessigsäure und 0.743 g (5 mMol) 1-Hydroxy-benztriazol in Gegenwart von 1.13 g (5.5 mMol) N,N'-Dicyclohexylcarbodiimid in 45 ml Tetrahydrofuran bei 0° während 2 Stunden) mit 2.47 g (5 mMol) 7β-Amino-3-[(1-methyl-1H-tetrazol-5-yl)]-thiomethyl-3-cephem-4-carbonsäure-diphenylmethylester den 7β-{2-[2-((2R)-2-BOC-Amino-2-diphenylmethoxycarbonyläthoxycarbonylamino)thiazol-4-yl]-2-syn-methoxyiminoacetylamino}-3-[(1-methyl-1H-tetrazol-5-yl)-thiomethyl]-3-cephem-4-carbonsäure-diphenylmethylester, der nach der chromatographischen Reinigung an Kieselgel mit Diäthyläther-Essigsäureäthylester 1:1 als Eluiermittel im nächsten Syntheseschritt eingesetzt wird.
DS: Rf = 0.59 (Fliessmittel: Diäthyläther-Essigsäureäthylester 1:1)

Beispiel 6: 7β-{(2R,S)-2-[2-((2R)-2-Amino-2-carboxyäthoxycarbonylamino)thiazol-4-yl]-2-aminoacetylamino}-3-methoxy-3-cephem-4-carbonsäure.

a) Eine auf 0° gekühlte Lösung von 2,1 g (2 mMol) 7β-{(2R,S)-2-[2-((2R)-2-BOC-Amino-2-diphenylmethoxycarbonyl-äthoxycarbonylamino)thiazol-4-yl]-2-BOC-amino-acetylamino}-3-methoxy-3-cephem-4-carbonsäure-diphenylmethylester und 4,2 ml Anisol in absolutem Methylenchlorid (21 ml) werden mit 21 ml Trifluoressigsäure versetzt und eine Stunde bei 0° unter einer Stickstoffatmosphäre und Feuchtigkeitsausschluss gerührt. Nach Zugabe von Diäthyläther (210 ml) wird das in kristalliner Form anfallende Trifluoracetat abfiltriert, mit Diäthyläther gewaschen und am Hochvakuum bei Raumtemperatur getrocknet.

Die wässrige Lösung (10 ml) des rohen Trifluoracetats (pH 1,8) wird mit Essigsäureäthylester (2x5 ml) extrahiert und mit 1N Natronlauge auf pH = 5 gestellt. Durch Zugabe von Isopropanol wird die Titelverbindung in mikrokristalliner Form erhalten. DS: $Rf_{96}$ = 0.11.

Das Ausgangsmaterial kann wie folgt hergestellt werden:

b) Zu einer auf 0° gekühlten Lösung von 64,5 g (0,3 Mol) 2-(2-Aminothiazol-4-yl)-2-syn-methoxyimino-essigsäuremethylester und 42 ml absolutem Pyridin in 600 ml absolutem Dimethylformamid wird unter Rühren und Feuchtigkeitsausschluss innert ca. 30 Minuten eine Lösung von 64 ml 2,2,2-Trichloräthoxycarbonylchlorid in 150 ml absolutem Tetrahydrofuran gegeben. Nach 2 Stunden bei 0° wird das Reaktionsgemisch mit Diäthyläther (1800 ml) verdünnt, das ausgefallene Pyridinhydrochlorid abfiltriert und mit Wasser (4x300 ml) gewaschen. Die organische Phase wird über Natriumsulfat getrocknet und am Rotationsverdampfer eingeengt. Das Rohprodukt wird darauf an der 10-fachen Menge Kieselgel mit Hexan-Diäthyläther

1:1 als Eluiermittel gereinigt, woraus nach der Kristallisation aus Diäthyläther-Petroläther 1:1 der 2-[2-(2,2,2-Trichloräthoxycarbonylamino)thiazol-4-yl]-2-syn-methoxyiminoessigsäuremethylester isoliert wird. Smp. 105-107°; DS: Rf = 0,48 (Fliessmittel: Hexan-Diäthyläther 1:1).

c) Eine Lösung von 38,0 g (97 mMol) 2-[2,2,2-Trichloräthoxycarbonylamino)thiazol-4-yl]-2-methoxyiminoessigsäuremethylester in 1000 ml Methylalkohol, der 9% Salzsäuregas enthält, wird in Gegenwart von 38 g 10%-iger Palladiumkohle bei Raumtemperatur hydriert. Nach Aufnahme der theoretischen Menge Wasserstoff wird das Reaktionsgemisch über Celite filtriert und am Rotationsverdampfer eingeengt. Der Rückstand wird mit Diäthyläther gewaschen, in Essigsäureäthylester (800 ml) aufgeschlämmt, und mit 5%-iger Natriumhydrogencarbonatlösung (2x200 ml) und Wasser gewaschen. Die organische Phase wird getrocknet ($Na_2SO_4$) und eingeengt und ergibt den (2R,S)-2-[-2-(2,2,2-Trichloräthoxycarbonylamino)thiazol-4-yl]-2-aminoessigsäure - methylester als blassgelbes Oel. DS: $Rf_{96}$ = 0,48.

d) Eine Lösung von 23,0 g (57,5 mMol) (2R,S)-2-[2-(2,2,2-Trichloräthoxycarbonylaminothiazol-4-yl]-2-aminoessigsäuremethylester in 940 ml Aethanol wird mit 17,2 g Kaliumhydroxid gelöst in 172 ml Wasser versetzt und während zwei Stunden bei Raumtemperatur gerührt. Das Reaktionsgemisch wird auf ca. 100 ml eingeengt, mit 250 ml Wasser verdünnt, mit Essigsäureäthylester (2x100 ml) extrahiert, und die auf 0° gekühlte wässerige Phase mit 2N Salzsäure auf pH = 4 gestellt. Nach einer Stunde wird die farblose, kristalline (2 R,S)-2-[2-(2,2,2-Trichloräthoxycarbonylamino)thiazol-4-yl]-2-aminoessigsäure abfiltriert, mit wenig Wasser gewaschen und am Hochvakuum (16 Stunden, 0,05 Torr, Raumtemperatur, $P_2O_5$) getrocknet. F.: ab 160° (Zersetzung); DS: $Rf_{96}$ = 0,45.

e) Einer gut gerührten Suspension von 6,07 g (17 mMol) (2R,S)-2-[2-(2,2,2-Trichloräthoxycarbonylamino) thiazol-4-yl]-2-aminoessigsäure und 1,794 g (~17 mMol) Natriumcarbonat in 70 ml Wasser-Dioxan 1:2 werden bei Raumtemperatur 4,06 g Di-tert.-butyl-dicarbonat in einer Portion zugefügt. Nach zwei Stunden wird das Reaktionsgemisch nach Zugabe von 50 ml Wasser bei 0° mit 2N Salzsäure auf pH = 2 gestellt und mit Essigsäureäthylester (3x250 ml) extrahiert. Die vereinigten organischen Phasen werden getrocknet ($Na_2SO_4$) und eingeengt. Das Rohprodukt wird an der 30-fachen Menge Kieselgel mit Diäthyläther als Eluiermittel gereinigt. Nach Kristallisation aus Diäthyläther wird die (2R,S)-2-[2-(2,2,2-Trichloräthoxycarbonylamino)thiazol-4-yl]-2-BOC-aminoessigsäure in Form farbloser Kristalle isoliert. F: 183-184°, DS: $Rf_{96}$ = 0,78.

f) Eine auf -20° gekühlte Lösung von 4,48 g (10 mMol) (2R,S)-2-[2-(2,2,2-Trichloräthoxycarbonylamino)-thiazol-4-yl]-2-BOC-aminoessigsäure und 1,12 ml N-Methyl-morpholin in 100 ml absolutem Methylenchlorid wird unter einer Stickstoffatmosphäre und Feuchtigkeitsausschluss mit 1,3 ml (10 mMol) Chlorameisensäureisobutylester versetzt. Nach 40 Minuten bei -20° wird das so erhaltene gemischte Anhydrid in einer Portion mit 4,33 g (10 mMol) 7β-Amino-3-methoxy-3-cephem-4-carbonsäure-diphenylmethylester-hydrochlorid, gefolgt von 1,12 ml N-Methyl-morpholin umgesetzt. Nach einer Reaktionszeit von 30 Minuten bei 0° und 2 Stunden bei Raumtemperatur wird das Reaktionsgemisch mit Essigsäureäthylester (400 ml) verdünnt, und mit Wasser (2x100 ml) und konzentrierter Natriumchloridlösung gewaschen. Nach Trocknung ($Na_2SO_4$) der organischen Phase und Entfernung des Lösungs-

mittels am Rotationsverdampfer wird das so erhaltene Rohprodukt an der 30-fachen Menge Kieselgel mit Diäthyläther als Eluiermittel gereinigt, und der 7β-{(2R,S)-2-[2-(2,2,2-Trichloräthoxycarbonylamino)thiazol-4-yl]-2-BOC-aminoacetylamino}-3-methoxy-3-cephem-4-carbonsäure-diphenylmethylester in Form eines farblosen Pulvers erhalten. DS: Rf = 0,63 (Fliessmittel: Diäthyläther-Essigsäureäthylester 1:1). Infrarotspektrum in Methylenchlorid: charakteristische Banden bei 3400, 1780, 1750, 1715, 1695, 1550 und 1210 $cm^{-1}$.

g) Eine auf 0° gekühlte Lösung von 8 g (1,67 mMol) 7β-{(2R,S)-2-[2-(2,2,2-Trichloräthoxycarbonylamino)thiazol-4-yl]-2-BOC-aminoacetylamino}-3-methoxy-3-cephem-4-carbonsäure-diphenylmethylester in 80 ml Acetonitril-Essigsäure 1:1 wird unter starkem Rühren portionenweise mit 8 g Zinkstaub versetzt. Nach einer Stunde wird das Zink abfiltriert, mit Acetonitril gewaschen und das Filtrat eingeengt. Der Rückstand wird in Essigsäureäthylester (400 ml) aufgenommen, mit 1N Natriumhydrogencarbonatlösung (2x80 ml) und gesättigter Natriumchloridlösung gewaschen. Nach Trocknung der organischen Phase über Natriumsulfat wird das Lösungsmittel am Rotationsverdampfer entfernt, worauf der 7β-(2R,S)-2-(2-Aminothiazol-4-yl)-2-BOC-amino-acetylamino]-3-methoxy-3-cephem-4-carbonsäure-diphenylmethylester erhalten wird, der ohne weitere Reinigung im nächsten Syntheseschritt eingesetzt werden kann. DS: Rf = 0,69 (Fliessmittel:Essigsäureäthylester) Infrarotspektrum in Methylenchlorid: charakterisitsche Banden bei 3380, 3300, 1782, 1715 und 1602 cm-1.

h) Zu einer auf 0° gekühlten Lösung von 3,26 g (5 mMol) 7β-[(2R,S)-2-(2-Aminothiazol-4-yl)-2-BOC-amino-acetylamino]-3-methoxy-3-cephem-4-carbonsäure-diphenylmethylester in 75 ml absolutem Methylenchlorid werden unter Rühren und Feuchtigkeitsausschluss 2,38 g (5,5 mMol) (2R)-2-BOC-Amino-2-diphenylmethoxycarbonyl-äthoxycarbonylchlorid gelöst in 30 ml absolutem Methylenchlorid, gefolgt von 0,4 ml (5 mMol) absolutem Pyridin gegeben. Nach je einer Stunde bei 0° und Raumtemperatur wird das Reaktionsgemisch mit Methylenchlorid (100 ml) verdünnt, mit Wasser (2x30 ml) gewaschen, getrocknet ($Na_2SO_4$) und am Rotationsverdampfer eingeengt. Das Rohprodukt wird an der 30-fachen Menge Kieselgel mit Diäthyläther-Essigsäureäthylester als Eluiermittel gereinigt. Der in amorpher Form erhaltene 7β-{(2R,S)-2-[2-((2R)-2-BOC-Amino-2-diphenylmethoxycarbonyläthoxycarbonyl-amino)thiazol-4-yl]-2-BOC-amino-acetylamino}-3-methoxy-3-cephem-4-carbonsäure-diphenylmethylester wird direkt in den nächsten Syntheseschritt eingesetzt. DS: Rf = 0,68 (Fliessmittel: Essigsäureäthylester).

Beispiel 7: 7β-[(2 R,S)-2-(2-Aminothiazol-4-yl)-2-aminoacetylamino]-3-methoxy-3-cephem-4-carbonsäure. Eine auf $0^o$ gekühlte Lösung von 2,3 g 7β-[(2R,S)-2-(2-Aminothiazol-4-yl)-2-BOC-aminoacetylamino]-3-methoxy-3-cephem-4-carbonsäure-diphenylmethylester und 2,3 ml Anisol in 12 ml absolutem Methylenchlorid wird mit 12 ml Trifluoressigsäure versetzt und unter Feuchtigkeitsausschluss eine Stunde bei Raumtemperatur gerührt. Nach Zugabe von Diäthyläther (200 ml) bei $0^o$ wird das in fester Form anfallende Trifluoracetat abfiltriert, mit Diäthyläther gewaschen und am Hochvakuum bei Raumtemperatur getrocknet. Die wässrige Lösung (15 ml) von 7β-[(2R,S)-2-(2-Aminothiazol-4-yl)-2-aminoacetylamino]-3-methoxy-3-cephem-4-carbonsäure-di-trifluoracetat wird bei $0^o$ mit 2N Natriumhydroxidlösung auf pH = 5,5 gebracht, klar filtriert und an Amberlite XAD-2

mit Wasser als Eluiermittel gereinigt, worauf nach der Entfernung des Lösungsmittels am Rotationsverdampfer und der Trocknung am Hochvakuum (16 Stunden 0,05 Torr, Raumtemperatur) die Titelverbindung, die 0,8 Mol Kristallwasser enthält, in kristalliner Form isoliert wird. F.: ab 145° C (Zersetzung); DS: $Rf_{96} = 0,1$; $[\alpha]_D = +147 \pm 1°$ (c = 0,805%, 0,1 N Salzsäure).

Beispiel 8: In analoger Weise zu den vorhergehenden Beispielen können die folgenden Verbindungen hergestellt werden:

7β-{2-[2-((2R)-2-Amino-2-carboxyäthoxycarbonylamino) thiazol-4-yl]-2-syn-methoxyimino-acetylamino}-3-(4-carbamoylpyridinio)methyl-3-cephem-4-carboxylat;

7β-{2-[2-((2R)-2-Amino-2-carboxyäthoxycarbonylamino) thiazol-4-yl]-2-hydroxy-acetylamino}-3-acetoxymethyl-3-cephem-4-carbonsäure;

7β-{2-[2-((2R)-2-Amino-2-carboxyäthoxycarbonylamino) thiazol-4-yl]-2-hydroxy-acetylamino}-3-carbamoyloxymethyl-3-cephem-4-carbonsäure;

7β-{2-[2-((2R)-2-Amino-2-carboxyäthoxycarbonylamino) thiazol-4-yl]-2-hydroxy-acetylamino}-3-(1-methyl-1H-tetrazol-5-ylmethyl)-3-cephem-4-carbonsäure;

7β-{2-[2-((2R)-2-Amino-2-carboxyäthoxycarbonylamino) thiazol-4-yl]-2-sulfo-acetylamino}-3-acetoxymethyl-3-cephem-4-carbonsäure;

7β-{2-[2-((2R)-2-Amino-2-carboxyäthoxycarbonylamino) thiazol-4-yl]-2-sulfo-acetylamino}-3-carbamoyloxymethyl-3-cephem-4-carbonsäure;

7β-{2-[2-((2R)-2-Amino-2-carboxyäthylmercapto-carbonylamino)thiazol-4-yl]-2-syn-methoxyiminoacetylamino]-3-

acetoxymethyl-3-cephem-4-carbonsäure;

7β-{2-[2-((2R)-2-Amino-2-carboxyäthylmercapto-carbonylamino)thiazol-4-yl]-2-syn-methoxyimino-acetylamino]-3-carbamoyloxymethyl-3-cephem-4-carbonsäure;

sowie ihre Natriumsalze.

Beispiel 9: Trockenampullen oder Vials, enthaltend 0,5 g 7β-{2-[2-((2R)-2-Amino-2-carboxyäthoxycarbonylamino)thiazol-4-yl]-2-methoxyimino-acetylamino}-3-acetoxymethyl-3-cephem-4-carbonsäure-natriumsalz (1,6 $H_2O$) als Wirksubstanz werden wie folgt hergestellt:

| | |
|---|---|
| Wirksubstanz | 0,5 g |
| Mannit | 0,05 g |

Eine sterile wässrige Lösung der Wirksubstanz und des Mannits wird unter aseptischen Bedingungen in 5 ml.-Ampullen oder 5 ml.-Vials verschlossen und geprüft.

Beispiel 10: Trockenampullen oder Vials, enthaltend 0,5 g 7β-[(2R,S)-2-(2-Aminothiazol-4-yl)-2-aminoacetylamino]-3-methoxy-3-cephem-4-carbonsäure-hydrat als Wirksubstanz werden wie folgt hergestellt:

Zusammensetzung (für 1 Ampulle oder Vial)

| | |
|---|---|
| Wirksubstanz | 0,5 g |
| Mannit | 0,05 g |

Eine sterile wässrige Lösung der Wirksubstanz und des Mannits wird unter aseptischen Bedingungen in 5 ml.-Ampullen oder 5 ml.-Vials verschlossen und geprüft.

## Patentansprüche

1. Aminothiazolacetamido-3-cephem-4-carbonsäureverbindungen der Formel

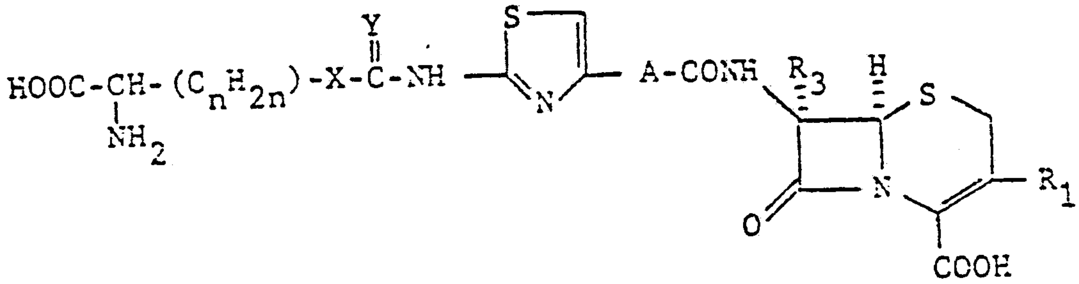

(I),

worin der Index n eine ganze Zahl von 1 bis 4, X Sauerstoff, Schwefel, die Gruppe -NH-, oder die direkte Bindung, Y Sauerstoff oder Schwefel, A Methylen oder durch Amino, Hydroxy, Carboxyl, Sulfo, Oxo, oder die Gruppe $=N-O-R^o$, worin $R^o$ Wasserstoff oder gegebenenfalls substituiertes Niederalkyl darstellt, substituiertes Methylen, $R_1$ Wasserstoff, Niederalkyl, eine veresterte oder verätherte Hydroxy- oder Mercaptogruppe, Halogen, Formyl oder eine Gruppe der Formel $-CH_2-R_2$, worin $R_2$ eine veresterte oder verätherte Hydroxy- oder Mercaptogruppe oder eine quaternäre Ammoniogruppe darstellt, und $R_3$ Wasserstoff oder Methoxy bedeuten, worin die Carboxylgruppen gegebenenfalls in physiologisch spaltbarer Form verestert sind, und Salze von solchen Verbindungen mit sauren und/oder basischen Gruppen, sowie die entsprechenden Verbindungen mit geschützten funktionellen Gruppen.

2. Verbindungen der Formel I nach Patentanspruch 1, worin die Gruppe $-(C_nH_{2n})-$ unverzweigt ist und n die angegebene Bedeutung hat, X Sauerstoff, Schwefel oder -NH-, Y Sauerstoff, A Methylen, Aminomethylen, Hydroxymethylen, Carboxymethylen, Sulfomethylen, Hydroxyiminomethylen oder Methoxyiminomethylen, $R_1$ Wasserstoff, Niederalkyl,

Niederalkoxy, Halogen oder eine Gruppe der Formel $-CH_2-R_2$, worin $R_2$ Niederalkanoyloxy, Carbamoyloxy, N-Niederalkylcarbamoyloxy, Triazolylthio, Tetrazolylthio, Thiazolylthio, Thiatriazolylthio, Thiadiazolylthio, Oxazolylthio, Oxadiazolylthio oder Pyridinio darstellt, worin die heterocyclischen Ringe gegebenenfalls beispielsweise durch Niederalkyl, N,N-Diniederalkylaminoniederalkyl, Carboxyniederalkyl, Sulfoniederalkyl, Amino, Carboxyniederalkanoylamino oder Carbamoyl substituiert sein können, und $R_3$ Wasserstoff oder Methoxy darstellen und pharmazeutisch verwendbare Salze von solchen Verbindungen, sowie die entsprechenden Verbindungen mit geschützten funktionellen Gruppen.

3. Verbindungen der Formel I nach Patentanspruch 1, worin die Gruppe $-(C_nH_{2n})-$ unverzweigt ist und n die angegebene Bedeutung hat, X Sauerstoff, Schwefel oder -NH- bedeutet, Y Sauerstoff, A Methylen, Aminomethylen, Hydroxymethylen oder Methoxyiminomethylen ist, $R_1$ Wasserstoff, Methyl, Methoxy, Chlor, oder eine Gruppe der Formel $-CH_2-R_2$ bedeutet, worin $R_2$ Acetoxy, Carbamoyloxy, Tetrazolylthio, insbesondere 1-Methyl-1H-tetrazol-5-ylthio, 1-Sulfomethyl-1H-tetrazol-5-ylthio, 1-Carboxymethyl-1H-tetrazol-5-ylthio, oder 1-(2-Dimethyl-aminoäthyl)-1H-tetrazol-5-ylthio, oder Thiadiazolylthio, insbesondere 2-Methyl-1,3,4-thiadiazol-5-ylthio, oder Carbamoylpyridinio, insbesondere 4-Carbamoylpyridinio, darstellt, und $R_3$ Wasserstoff bedeutet und pharmazeutisch verwendbare Salze von solchen Verbindungen, sowie die entsprechenden Verbindungen mit geschützten funktionellen Gruppen.

4. 7β-{2-[2-((2R)-2-Amino-2-carboxyäthoxycarbonylamino)thiazol-4-yl]acetylamino}-3-acetoxymethyl-3-cephem-

4-carbonsäure, gemäss Patentanspruch 1.

5. 7β-{2-[2-((2R-2-Amino-2-carboxyäthoxycarbonylamino)thiazol-4-yl]-2-syn-methoxyiminoacetylamino}-3-acetoxymethyl-3-cephem-4-carbonsäure, gemäss Patentanspruch 1.

6. 7β-2-[2-((2R)-Amino-2-carboxyäthoxycarbonylamino)thiazol-4-yl]-2-syn-methoxyiminoacetylamino-3-carbamoyloxymethyl-3-cephem-4-carbonsäure, gemäss Patentanspruch 1.

7. 7β-{2-[2-((2R)-2-Amino-2-carboxyäthoxycarbonylamino)thiazol-4-yl]-2-syn-methoxyiminoacetylamino}-3-methoxy-3-cephem-4-carbonsäure, gemäss Patentanspruch 1.

8. 7β-{2-[2-((2R)-2-Amino-2-carboxyäthoxycarbonylamino)-thiazol-4-yl]-2-syn-methoxyiminoacetylamino}-3-[(1-methyl-1H-tetrazol-5-yl)-thiomethyl]-3-cephem-4-carbonsäure, gemäss Patentanspruch 1.

9. 7β-{(2R,S)-2-[2-((2R)-2-Amino-2-carboxyäthoxycarbonylamino)thiazol-4-yl]-2-aminoacetylamino}-3-methoxy-3-cephem-4-carbonsäure, gemäss Patentanspruch 1.

10. Pharmazeutische annehmbare Salze der Verbindungen nach einem der Patentansprüche 1-9.

11. Die Natriumsalze der Verbindungen nach einem der Patentansprüche 1 bis 10.

12. Die in den Beispielen genannten Verbindungen gemäss einem der Patentansprüche 1 bis 11.

13. Pharmazeutische Präparate enthaltend eine der Verbindungen der Patentansprüche 1 bis 11.

14. Verwendung der Verbindungen der Patentansprüche 1-11 als antibakterielle Antibiotika.

15. Verfahren zur Herstellung von Aminothiazolacetamido-3-cephem-4-carbonsäureverbindungen der Formel

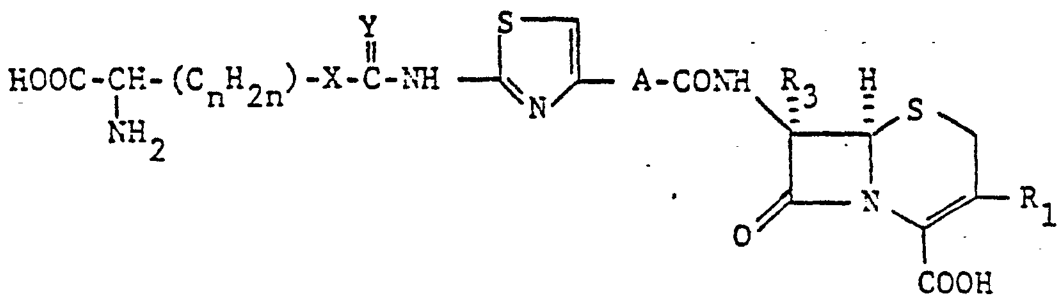

(I),

worin der Index n eine ganze Zahl von 1 bis 4, X Sauerstoff, Schwefel, die Gruppe -NH-, oder die direkte Bindung, Y Sauerstoff oder Schwefel, A Methylen oder durch Amino, Hydroxy, Carboxyl, Sulfo, Oxo, oder die Gruppe $=N-O-R^o$, worin $R^o$ Wasserstoff oder gegebenenfalls substituiertes Niederalkyl darstellt, substituiertes Methylen, $R_1$ Wasserstoff, Niederalkyl, eine veresterte oder verätherte Hydroxy- oder Mercaptogruppe, Halogen, Formyl oder eine Gruppe der Formel $-CH_2-R_2$, worin $R_2$ eine veresterte oder verätherte Hydroxy- oder Mercaptogruppe oder eine quaternäre Ammoniogruppe darstellt, und $R_3$ Wasserstoff oder Methoxy bedeuten, worin die Carboxylgruppen gegebenenfalls in physiologisch spaltbarer Form verestert sind, sowie ihrer Salze, dadurch gekennzeichnet, dass man in einer der Formel I entsprechenden Ausgangsverbindung, worin mindestens eine der vorhandenen funktionellen Gruppen geschützt ist, die funktionelle(n) Gruppe(n) freisetzt, wenn erwünscht, in einer erhaltenen Verbindung einen Rest $R_1$ in einen anderen Rest $R_1$ überführt, und/oder, wenn erwünscht, eine freie Carboxylgruppe in eine physiologisch spaltbare veresterte Carboxylgruppe überführt, und/oder, wenn erwünscht, ein erhaltenes Isomerengemisch in die einzelnen Isomeren

auftrennt, und/oder, wenn erwünscht, eine erhaltene Verbindung in ein Salz oder ein erhaltenes Salz in eine freie Verbindung oder in ein anderes Salz überführt.

16. Verfahren nach Patentanspruch 15, dadurch gekennzeichnet, dass man geschützte funktionelle Gruppen durch Solvolyse freisetzt.

17. Verfahren nach Patentanspruch 15, dadurch gekennzeichnet, dass man geschützte funktionelle Gruppen durch Reduktion freisetzt.

18. Verfahren nach Patentanspruch 15, dadurch gekennzeichnet, dass man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht.

19. Verfahren nach Patentanspruch 15, dadurch gekennzeichnet, dass man einen Ausgangsstoff in situ, gegebenenfalls unter den Reaktionsbedingungen, bildet, oder dass das Ausgangsmaterial als Salz eingesetzt wird.

20. Verfahren nach einem der Patentansprüche 15-19, zur Herstellung von Verbindungen der Formel (I), worin die Gruppe $-(C_nH_{2n})-$ unverzweigt ist und n die angegebene Bedeutung hat, X Sauerstoff, Schwefel oder -NH-, Y Sauerstoff, A Methylen, Aminomethylen, Hydroxymethylen, Carboxymethylen, Sulfomethylen, Hydroxyiminomethylen oder Methoxyiminomethylen, $R_1$ Wasserstoff, Niederalkyl,

Niederalkoxy, Halogen oder eine Gruppe der Formel $-CH_2-R_2$, worin $R_2$ Niederalkanoyloxy, Carbamoyloxy, N-Niederalkylcarbamoyloxy, Triazolylthio, Tetrazolylthio, Thiazolylthio, Thiatriazolylthio, Thiadiazolylthio, Oxazolylthio, Oxadiazolylthio oder Pyridinio darstellt, worin die heterocyclischen Ringe gegebenenfalls beispielsweise durch Niederalkyl, N,N-Diniederalkylaminoniederalkyl, Carboxyniederalkyl, Sulfoniederalkyl, Amino, Carboxyniederalkanoylamino oder Carbamoyl substituiert sein können, und $R_3$ Wasserstoff oder Methoxy darstellen, und pharmazeutisch verwendbaren Salzen davon.

21. Verfahren nach einem der Patentansprüche 15-19 zur Herstellung von Verbindungen der Formel (1), worin die Gruppe $-(C_nH_{2n})-$ unverzweigt ist und n die angegebene Bedeutung hat, X Sauerstoff, Schwefel oder -NH- bedeutet, Y Sauerstoff, A Methylen, Aminomethylen, Hydroxymethylen oder Methoxyiminomethylen ist, $R_1$ Wasserstoff, Methyl, Methoxy, Chlor, oder eine Gruppe der Formel $-CH_2-R_2$ bedeutet, worin $R_2$ Acetoxy, Carbamoyloxy, Tetrazolylthio, insbesondere 1-Methyl-1H-tetrazol-5-ylthio, 1-Sulfomethyl-1H-tetrazol-5-ylthio, 1-Carboxymethyl-1H-tetrazol-5-ylthio, oder 1-(2-Dimethyl-aminoäthyl)-1H-tetrazol-5-ylthio, oder Thiadiazolylthio, insbesondere 2-Methyl-1,3,4-thiadiazol-5-ylthio, oder Carbamoylpyridinio, insbesondere 4-Carbamoylpyridinio, darstellt, und $R_3$ Wasserstoff bedeutet, und pharmazeutisch verwendbaren Salzen davon.

22. Verfahren nach einem der Patentansprüche 15-19 zur Herstellung von 7β-{2-[2-((2R)-2-Amino-2-carboxyäthoxycarbonylamino)thiazol-4-yl]acetylamino}-3-acetoxymethyl-3-cephem-4-carbonsäure und pharmazeutisch verwendbaren Salzen davon.

23. Verfahren nach einem der Patentansprüche 15-19 zur Herstellung von 7ß-{2-[2-((2R)-2-Amino-2-carboxyäthoxycarbonylamino)thiazol-4 yl]-2-syn-methoxyiminoacetylamino}-3-acetoxymethyl-3-cephem-4-carbonsäure und pharmazeutisch verwendbaren Salzen davon.

24. Verfahren nach einem der Patentansprüche 15-19 zur Herstellung von 7ß-{2-[2-((2R)-Amino-2-carboxyäthoxycarbonylamino)thiazol-4-yl]-2-syn-methoxyiminoacetylamino}-3-carbamoyloxymethyl-3-cephem-4-carbonsäure und pharmazeutisch verwendbaren Salzen davon.

25. Verfahren nach einem der Patentansprüche 15-19 zur Herstellung von 7ß-{2-[2-((2R)-2-Amino-2-carboxyäthoxycarbonylamino)thiazol-4-yl]-2-syn-methoxyiminoacetylamino}-3-methoxy-3-cephem-4-carbonsäure und pharmazeutisch verwendbaren Salzen davon.

26. Verfahren nach einem der Patentansprüche 15-19 zur Herstellung von 7ß-{2-[2-((2R)-2-Amino-2-carboxyäthoxycarbonylamino)-thiazol-4-yl]-2-syn-methoxyiminoacetylamino}-3-[(1-methyl-1H-tetrazol-5-yl)-thiomethyl]-3-cephem-4-carbonsäure und pharmazeutisch verwendbaren Salzen davon.

27. Verfahren nach einem der Patentansprüche 15-19 zur Herstellung von 7ß-{(2R,S)-2-[2-((2R)-2-Amino-2-carboxyäthoxycarbonylamino)thiazol-4-yl]-2-aminoacetylamino}-3-methoxy-3-cephem-4-carbonsäure und pharmazeutisch verwendbaren Salzen davon.

28. Die in den Beispielen 1-8 genannten Verfahren nach einem der Patentansprüche 15-27.

29. Verfahren zur Herstellung von Aminothiazolacetamido-3-cephem-4-carbonsäureverbindungen der Formel

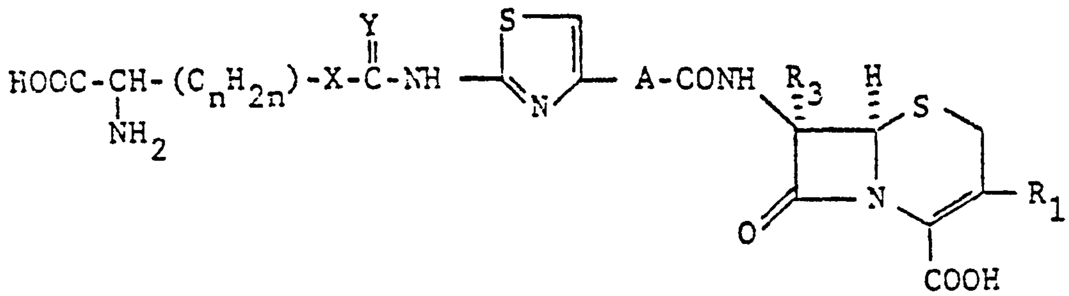

(I),

worin der Index n eine ganze Zahl von 1 bis 4, X Sauerstoff, Schwefel, die Gruppe -NH-, oder die direkte Bindung, Y Sauerstoff oder Schwefel, A Methylen oder durch Amino, Hydroxy, Carboxyl, Sulfo, Oxo, oder die Gruppe $=N-O-R^o$, worin $R^o$ Wasserstoff oder gegebenenfalls substituiertes Niederalkyl darstellt, substituiertes Methylen, $R_1$ Wasserstoff, Niederalkyl, eine veresterte oder verätherte Hydroxy- oder Mercaptogruppe, Halogen, Formyl oder eine Gruppe der Formel $-CH_2-R_2$, worin $R_2$ eine veresterte oder verätherte Hydroxy- oder Mercaptogruppe oder eine quaternäre Ammoniogruppe darstellt, und $R_3$ Wasserstoff oder Methoxy bedeuten, worin mindestens eine der funktionellen Gruppen in geschützter Form vorliegt, dadurch gekennzeichnet, dass man

a) in einer Verbindung der Formel

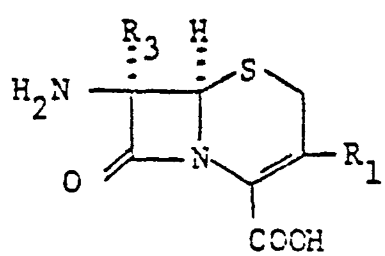

(II),

worin die Aminogruppe gegebenenfalls durch eine die Acylierung erlaubende Gruppe substituiert ist, und worin die 4-Carboxylgruppe und gegebenenfalls im Rest $R_1$

vorhandene weitere funktionelle Gruppen in geschützter Form vorliegen können, die Aminogruppe durch Behandeln mit einem den Acylrest einer Carbonsäure der Formel

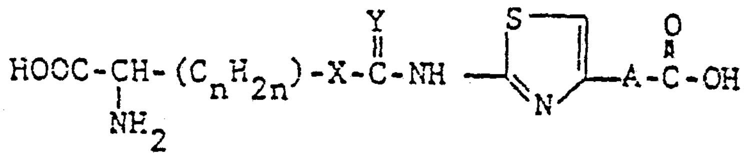

(III)

einführenden Acylierungsmittel, worin die Aminocarbonsäuregruppierung HOOC-CH($NH_2$)- und gegebenenfalls in der Gruppierung A vorhandene weitere funktionelle Gruppen in geschützter Form vorliegen, acyliert, oder

z) in einer Verbindung der Formel

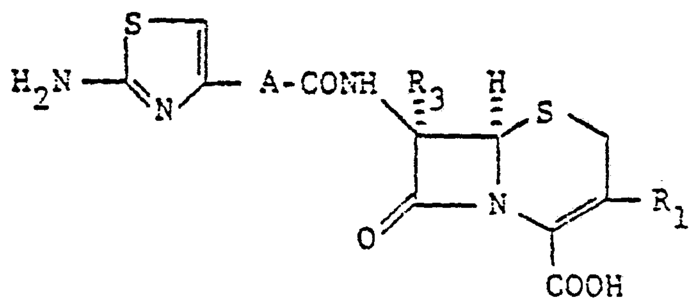

(IV),

worin die Aminogruppe gegebenenfalls durch eine die Acylierung erlaubende Gruppe substituiert ist, und worin die 4-Carboxylgruppe und gegebenenfalls im Rest $R_1$ und in der Gruppierung A vorhandene weitere funktionelle Gruppen in geschützter Form vorliegen können, die Aminogruppe durch Behandeln mit einem den entsprechenden Acylrest einer Carbonsäure der Formel

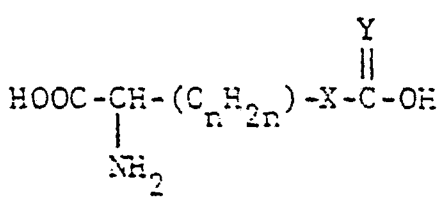

(V)

einführenden Acylierungsmittel, worin die Aminocarbonsäuregruppierung $HOOC-CH(NH_2)-$ in geschützter Form vorliegt, acyliert, oder

c) in einer Verbindung der Formel

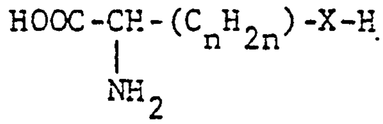

(VI),

worin X Sauerstoff, Schwefel oder die Gruppe -NH- bedeutet, und worin die Aminocarbonsäuregruppierung $HOOC-CH(NH_2)-$ in geschützter Form vorliegt, die Gruppe -X-H mit einem den entsprechenden Acylrest einer Carbonsäure der Formel

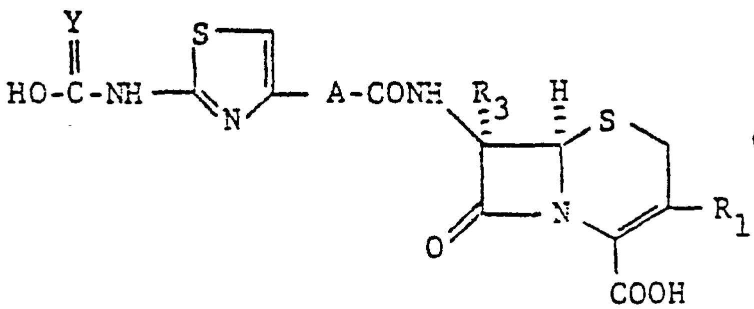

(VII)

einführenden Acylierungsmittel, worin die 4-Carboxylgruppe und gegebenenfalls im Rest $R_1$ und in der Gruppierung A vorhandene funktionelle Gruppen in geschützter Form vorliegen können, acyliert, oder

d) eine Verbindung der Formel

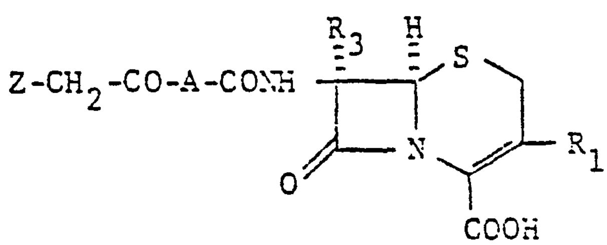

(VIII),

worin Z Halogen bedeutet, und worin funktionelle Gruppen gegebenenfalls geschützt sind, oder ein Salz davon, mit einem Thioharnstoff der Formel

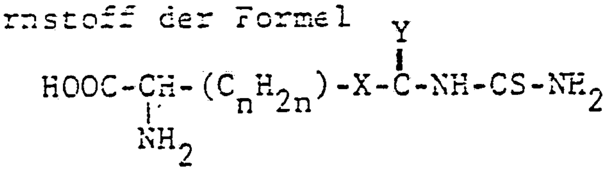
(IX),

worin funktionelle Gruppen geschützt sind, oder einem Salz davon, kondensiert, oder

e) zur Herstellung von Verbindungen der Formel I, worin $R_1$ Wasserstoff ist, in einer Verbindung der Formel

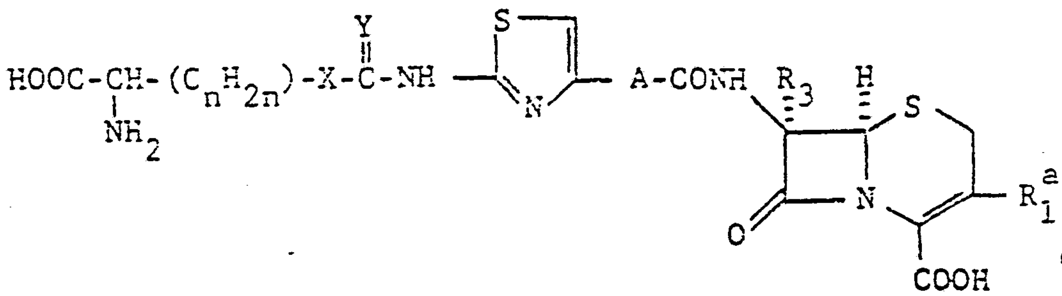
(X),

worin $R_1^a$ eine gegebenenfalls veresterte Hydroxygruppe oder eine sekundäre oder tertiäre Aminogruppe und $R_3$ Wasserstoff ist, und worin funktionelle Gruppen gegebenenfalls geschützt sind, die Gruppe $R_1^a$ durch Wasserstoff ersetzt oder

f) zur Herstellung von Verbindungen der Formel I, worin $R_1$ Wasserstoff ist und worin funktionelle Gruppen geschützt sind, eine Verbindung der Formel

(XI),
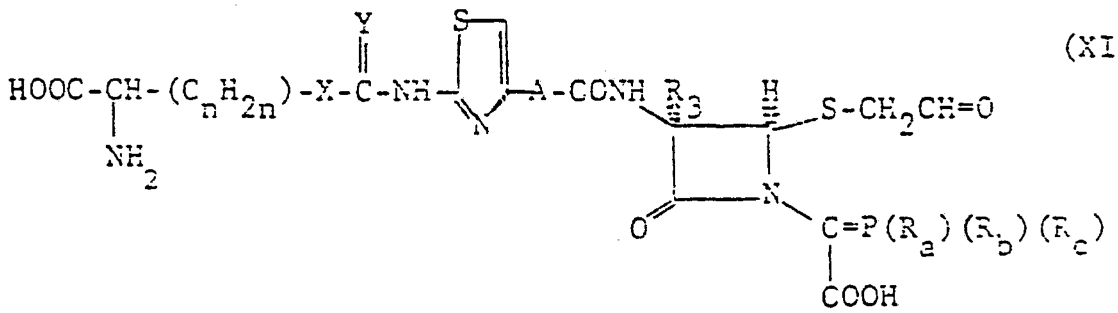

worin $R_3$ Wasserstoff ist, jeder der Reste $R_a$, $R_b$ und $R_c$ einen gegebenenfalls substituierten Kohlenwasserstoffrest darstellt, und worin funktionelle Gruppen geschützt sind, unter Abspaltung von $O=P(R_a)(R_b)(R_c)$ ringschliesst, oder

g) zur Herstellung von Verbindungen der Formel I, worn $R_1$ Niederalkyl, eine veresterte oder verätherte Hydroxygruppe oder Halogen bedeutet, und worin funktionelle Gruppen geschützt sind, eine Verbindung der Formel

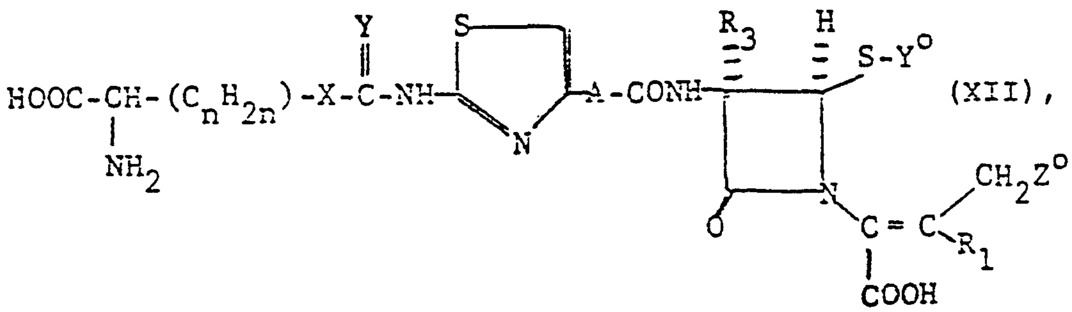

(XII),

worin $R_1$ die obige Bedeutung hat, $R_3$ Wasserstoff ist, $Y^o$ eine Abgangsgruppe darstellt und $Z^o$ Wasserstoff oder Halogen bedeutet, und worin funktionelle Gruppen geschützt sind, unter Abspaltung von $Y^o$ und $Z^o$ ringschliesst oder

h) eine 2-Cephemverbindung der Formel

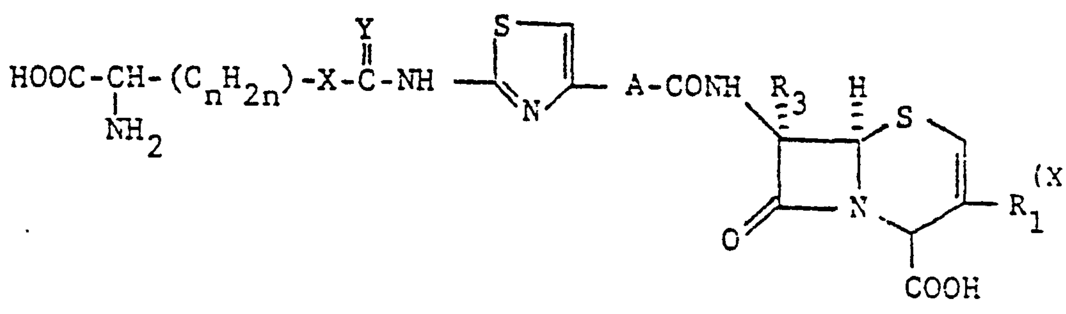

(XIII),

worin funktionelle Gruppen geschützt sind, zur entsprechenden 3-Cephemverbindung der Formel I isomerisiert, und, wenn gewünscht, in einer erhaltenen Verbindung noch nicht geschützte funktionelle Gruppen schützt oder eine

Schutzgruppe gegen eine andere austauscht, und/oder, wenn gewünscht, einen Rest A oder $R_1$ in einen anderen Rest A bzw. $R_1$ überführt, und/oder, wenn gewünscht, eine erhaltene Verbindung, worin $R_3$ Wasserstoff ist, in eine Verbindung überführt, worin $R_3$ Methoxy ist, und/oder, wenn erwünscht, eine erhaltene Verbindung in ein Salz oder ein erhaltenes Salz in eine freie Verbindung oder in ein anderes Salz überführt.

30. Die in den Beispielen genannten Verfahren gemäss Patentanspruch 29.

31. Verbindungen der Formel

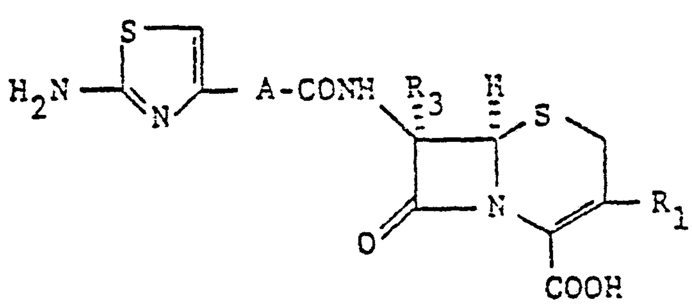

(IV),

worin A Aminomethylen, $R_3$ Wasserstoff oder Methoxy und $R_1$ Wasserstoff, eine der genannten verätherten Hydroxy- oder Mercaptogruppen oder Halogen bedeuten, ihre Salze und geschützten Derivate.

32. 7ß-[(2 R,S)-2-(2-Aminothiazol-4-yl)-2-aminoacetylamino]-3-methoxy-3-cephem-4-carbonsäure.

33. Pharmazeutische Präparate enthaltend eine der Verbindungen der Patentansprüche 30 oder 31.

34. Verfahren zur Herstellung von Verbindungen der Formel

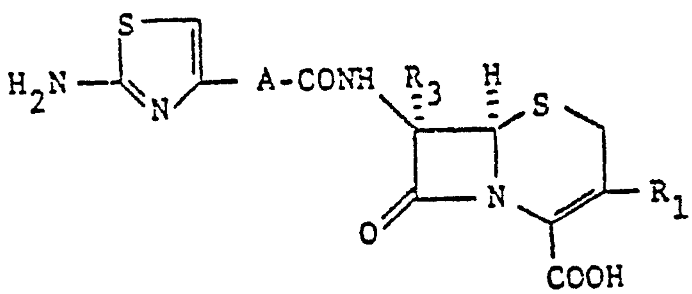

(IV),

worin A Aminomethylen, $R_3$ Wasserstoff oder Methoxy und $R_1$ Wasserstoff, eine der genannten verätherten Hydroxy- oder Mercaptogruppen oder Halogen bedeuten, sowie ihrer Salze und geschützten Derivate, dadurch gekennzeichnet, dass man eine Verbindung der Formel

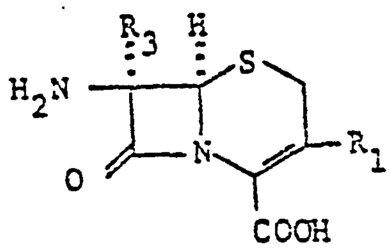

(II),

worin die Aminogruppe gegebenenfalls durch eine die Acylierung erlaubende Gruppe substituiert ist und worin die 4-Carboxylgruppe und gegebenenfalls im Rest $R_1$ vorhandene weitere funktionelle Gruppen in geschützter Form vorliegen können, mit einem den Acylrest einer Säure der Formel

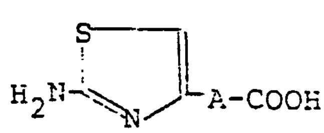

(XIV)

einführenden Acylierungsmittel, worin die Aminogruppe gegebenenfalls geschützt sein kann, und die in der Gruppierung -A- vorhandene Aminogruppe geschützt ist, z.B. mit einem reaktionsfähigen funktionellen Derivat einer solchen Säure

oder einem Salz davon, acyliert, wenn gewünscht in einer erhaltenen Verbindung die Schutzgruppen abspaltet und/oder, wenn gewünscht, eine erhaltene Verbindung, worin $R_3$ Wasserstoff ist, in eine Verbindung überführt, worin $R_3$ Methoxy ist, und/oder wenn notwendig, eine erhaltene 2-Cephemverbindung oder ein erhaltenes Gemisch einer 2-Cephem- und 3-Cephemverbindung, zur 3-Cephemverbindung isomerisiert, und/oder, wenn gewünscht, ein erhaltenes Isomerengemisch in die Isomeren auftrennt, und/oder wenn gewünscht eine erhaltene Verbindung mit salzbildender Gruppe in ein Salz oder ein erhaltenes Salz in die freie Verbindung überführt.

35. Verbindungen der Formel

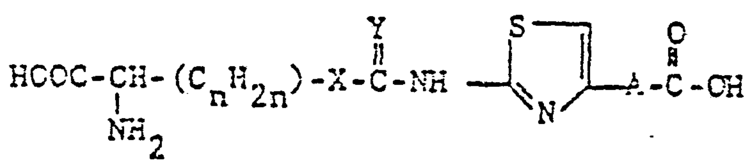

(III)

worin der Index n eine ganze Zahl von 1 bis 4, X Sauerstoff, Schwefel, die Gruppe -NH-, oder die direkte Bindung, Y Sauerstoff oder Schwefel, A Methylen oder durch Amino, Hydroxy, Carboxyl, Sulfo, Oxo, oder die Gruppe =N-O-R°, worin R° Wasserstoff oder gegebenenfalls substituiertes Niederalkyl darstellt, substituiertes Methylen darstellen, und worin die Aminocarbonsäuregruppierung HOOC-CH($NH_2$)- und gegebenenfalls in der Gruppierung A vorhandene weitere funktionelle Gruppen in geschützter Form vorliegen.

36. Verfahren zur Herstellung von Verbindungen der Formel

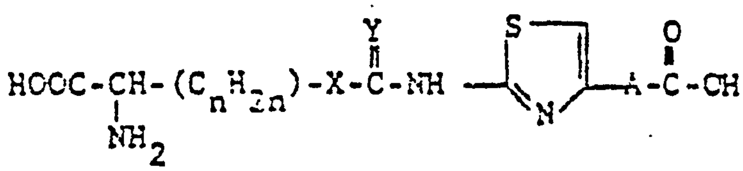 (III)

worin der Index n eine ganze Zahl von 1 bis 4, X Sauerstoff, Schwefel, die Gruppe -NH-, oder die direkte Bindung, Y Sauerstoff oder Schwefel, A Methylen oder durch Amino, Hydroxy, Carboxyl, Sulfo, Oxo, oder die Gruppe =N-O-R°, worin R° Wasserstoff oder gegebenenfalls substituiertes Niederalkyl darstellt, substituiertes Methylen darstellen, und worin die Aminocarbonsäuregruppierung HOOC-CH($NH_2$)- und gegebenenfalls in der Gruppierung A vorhandene weitere funktionelle Gruppen in geschützter Form vorliegen, dadurch gekennzeichnet, dass man eine Verbindung der Formel

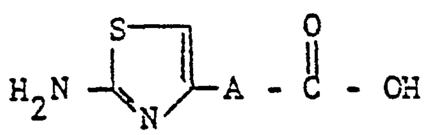 (XIV),

worin die Aminogruppe gegebenenfalls durch eine die Acylierung erlaubende Gruppe substituiert sein kann, und gegebenenfalls in der Gruppierung -A- vorhandene funktionelle Gruppen geschützt sind, unter intermediärem Schutz der Carboxylgruppe, mit einem den entsprechenden Acylrest einer Carbonsäure der Formel

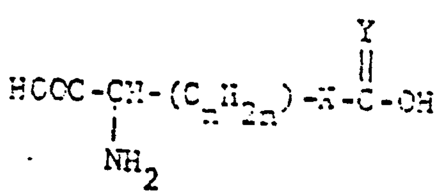 (V)

einführenden Acylierungsmittel, worin die Aminocarbonsäuregruppierung HOOC-CH($NH_2$)- in geschützter Form vorliegt, acyliert, und wenn erwünscht, eine erhaltene Verbindung in eine andere Verbindung der Formel III mit entsprechend geschützten funktionellen Gruppen überführt.

Europäisches Patentamt

**EUROPÄISCHER TEILRECHERCHENBERICHT,** der nach Regel 45 des Europäischen Patent-übereinkommens für das weitere Verfahren als europäischer Recherchenbericht gilt

Nummer der Anmeldung
EP 79 81 0157

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| | DE - A - 2 422 068 (SMITH KLINE)<br>* Seiten 30-36; Ansprüche *<br>-- | 1 | C 07 D 501/20<br>A 61 K 31/545<br>C 07 D 277/48//<br>C 07 C 157/12<br>C 07 D 277/40 |
| P | EP - A - 0 000 500 (CIBA-GEIGY)<br>* Seiten 125-136; Ansprüche *<br>---- | 1-30 | |
| | | | RECHERCHIERTE SACHGEBIETE (Int. Cl.³) |
| | | | C 07 D 501/20<br>501/57<br>A 61 K 31/545 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-11,13,15-27,29
Unvollständig recherchierte Patentansprüche:
Nicht recherchierte Patentansprüche: 14,12,28,30
Grund für die Beschränkung der Recherche:

14: Verfahren zur chirurgischen oder therapeutischen Behandlung des menschlichen oder tierischen Körpers (Siehe Art. 52(4) des Europäischen Patentübereinkommens)

12,28,30:
Vorschriften der Regel 20(6)

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 06-02-1980 | LUYTEN |